# EUROPEAN PATENT SPECIFICATION

(11) **EP 3 282 015 B1**
(45) Date of publication and mention of the grant of the patent: **20.05.2020**
(21) Application number: 17182410.5
(22) Date of filing: 02.12.2011
(51) Int. Cl.: C12N 15/67, C12N 5/16, C12N 9/12, C12N 5/074, C12N 15/63

(54) **METHOD FOR CELLULAR RNA EXPRESSION**
VERFAHREN FÜR ZELLULÄRE RNA-EXPRESSION
PROCÉDÉ D'EXPRESSION D'ARN CELLULAIRE

(30) Priority: 03.12.2010 WO PCT/EP2010/007362
(43) Date of publication of application: 14.02.2018
(62) Divisional of application: 11791447.3
(73) Proprietor: BioNTech RNA Pharmaceuticals GmbH, 55131 Mainz (DE); Translationale Onkologie an der Universitätsmedizin der JGU Mainz gGmbH (TRON), 55131 Mainz (DE)
(72) Inventor: SAHIN, Ugur, 55116 Mainz (DE); BEISSERT, Tim, 64521 Gross-Gerau (DE); POLEGANOV, Marco, 60528 Frankfurt (DE); HERZ, Stephanie, 54317 Kasel (DE)
(74) Representative: Wilk, Thomas

(56) References cited:
- WO-A1-00/14255
- WO-A1-2010/051223
- WO-A1-2012/072096
- WO-A2-02/068635
- HENRY G L ET AL: "Mechanism of interferon action: Translational control and the RNA-dependent protein kinase (PKR): Antagonists of PKR enhance the translational activity of mRNAs that include a 161 nucleotide region from reovirus S1 mRNA", 1 January 1994 (1994-01-01), JOURNAL OF BIOLOGICAL REGULATORS AND HOMEOSTATIC AGENTS, WICHTIG EDITORE, MILAN, IT, PAGE(S) 15 - 25, XP009150095, ISSN: 0393-974X * page 18, right-hand column, paragraph 3 *

## Description

### TECHNICAL FIELD OF THE INVENTION

The present teaching relates to the use of an inhibitor of the activity of RNA-dependent protein kinase (PKR) in an *in vitro* method for providing cells having stem cell characteristics.

### BACKGROUND OF THE INVENTION

The advantages of using RNA as a kind of reversible gene therapy include transient expression and a non-transforming character. RNA does not need to enter the nucleus in order to be expressed and moreover cannot integrate into the host genome, thereby eliminating the risk of oncogenesis. Transfection rates attainable with RNA are relatively high, for many cell types even >90%, and therefore, there is no need for selection of transfected cells. Furthermore, the amounts of protein achieved correspond to those in physiological expression.

RNA has been described for as being useful in de-differentiating somatic cells into stem-like cells without generating embryos or fetuses. De-differentiation of somatic cells to cells having stem cell characteristics, in particular pluripotency, can be effected by introducing RNA encoding factors inducing the de-differentiation of somatic cells into the somatic cells and culturing the somatic cells allowing the cells to de-differentiate. After being de-differentiated, the cells could be induced to re-differentiate into the same or a different somatic cell type such as neuronal, hematopoietic, muscle, epithelial, and other cell types. Thus, such stem-like cells have medical applications for treatment of degenerative diseases by "cell therapy" and may be utilized in novel therapeutic strategies in the treatment of cardiac, neurological, endocrinological, vascular, retinal, dermatological, muscular-skeletal disorders, and other diseases.

Furthermore, the use of RNA provides an attractive alternative to circumvent the potential risks of DNA based vaccines. As with DNA, transfer of RNA into cells can also induce both the cellular and humoral immune responses *in vivo.*

In particular, two different strategies have been pursued for immunotherapy with *in vitro* transcribed RNA (IVT-RNA), which have both been successfully tested in various animal models. Either the RNA is directly injected into the patient by different immunization routes or cells are transfected with IVT-RNA using conventional transfection methods *in vitro* and then the transfected cells are administered to the patient. RNA may, for example, be translated and the expressed protein presented on the MHC molecules on the surface of the cells to elicit an immune response.

It has been attempted to stabilize IVT-RNA by various modifications in order to achieve higher and prolonged expression of transferred IVT-RNA. However, despite the success of RNA transfection-based strategies to express peptides and proteins in cells, there remain issues related to RNA stability, sustained expression of the encoded peptide or protein and cytotoxicity of the RNA. For example, it is known that exogenous single-stranded RNA activates defense mechanisms in mammalian cells. Henry et al, (1994) in JOURNAL OF BIOLOGICAL REGULATORS AND HOMEOSTATIC AGENTS, WICHTIG EDITORE, MILAN, IT, PAGE(S) 15 - 25, discloses methods for expressing RNA in a cell comprising the step of reducing the activity of PKR in a cell by using the inhibitor 2-amino purine (2-AP). The step of reducing the activity of PKR in the cell results in an enhancement of expression of the RNA in the cell. The method is performed in COS cells which are fibroblasts.

### SUMMARY OF THE INVENTION

The present teaching relates to a method for expressing RNA in a cell comprising the step of reducing the activity of RNA-dependent protein kinase (PKR) in the cell.
In one embodiment, the RNA is or has been introduced into the cell such as by electroporation.

In one embodiment, the RNA is *in vitro* transcribed RNA.

In one embodiment, the step of reducing the activity of PKR in the cell results in an enhancement of stability and/or an enhancement of expression of the RNA in the cell, wherein the enhancement of expression of the RNA in the cell preferably comprises an increase in the level of expression and/or an increase in the duration of expression of the RNA in the cell.

In one embodiment, the step of reducing the activity of PKR in the cell comprises treating the cell with at least one PKR inhibitor. In one embodiment, the treatment of the cell with the at least one PKR inhibitor is for a time sufficient to result in an enhancement of stability and/or an enhancement of expression of the RNA in the cell. In one embodiment, the PKR inhibitor inhibits RNA-induced PKR autophosphorylation. In one embodiment, the PKR inhibitor is an ATP-binding site directed inhibitor of PKR. In one embodiment, the PKR inhibitor is an imidazolo-oxindole compound. In one embodiment, the PKR inhibitor is 6,8-dihydro-8-(1H-imidazol-5-ylmethylene)-7H-pyrrolo[2,3-g]benzothiazol-7-one or 2-aminopurine. In one embodiment, the PKR inhibitor is a virally derived inhibitor of PKR, wherein the virally derived inhibitor of PKR is preferably selected from the group consisting of vaccinia virus E3 and/or K3, or their RNA.

In one embodiment, the step of reducing the activity of PKR in the cell comprises silencing expression of the PKR gene.

In one embodiment, the cell is a cell having a barrier function. In one embodiment, the cell is a fibroblast, a keratinocyte, an epithelial cell, or an endothelial cell, wherein the endothelial cell preferably is an endothelial cell of the heart, an endothelial cell of the lung, or an umbilical vein endothelial cell. Preferably, the cell is a human cell.

The present teaching also relates to the use of means which are suitable for reducing the activity of RNA-dependent protein kinase (PKR) in a cell such as means as described herein, in particular at least one PKR inhibitor, such at least one of the PKR inhibitors as described herein, for treating a cell in which RNA is to be expressed.

In one embodiment, the RNA is or has been introduced into the cell such as by electroporation.

In one embodiment, the RNA is *in vitro* transcribed RNA.

In one embodiment, treatment of the cell results in an enhancement of stability and/or an enhancement of expression of the RNA in the cell, wherein the enhancement of expression of the RNA in the cell preferably comprises an increase in the level of expression and/or an increase in the duration of expression of the RNA in the cell.

In one embodiment, the treatment of the cell with the at least one PKR inhibitor is for a time sufficient to result in an enhancement of stability and/or an enhancement of expression of the RNA in the cell. In one embodiment, the PKR inhibitor inhibits RNA-induced PKR autophosphorylation. In one embodiment, the PKR inhibitor is an ATP-binding site directed inhibitor of PKR. In one embodiment, the PKR inhibitor is an imidazolo-oxindole compound. In one embodiment, the PKR inhibitor is 6,8-dihydro-8-(1H-imidazol-5-ylmethylene)-7H-pyrrolo[2,3-g]benzothiazol-7-one or 2-aminopurine. In one embodiment, the PKR inhibitor is a virally derived inhibitor of PKR, wherein the virally derived inhibitor of PKR is preferably selected from the group consisting of vaccinia virus E3 and/or K3, or their RNA.

In one embodiment, the means for reducing the activity of PKR in the cell comprises means for silencing expression of the PKR gene.

In one embodiment, the cell is a cell having a barrier function. In one embodiment, the cell is a fibroblast, a keratinocyte, an epithelial cell, or an endothelial cell, wherein the endothelial cell preferably is an endothelial cell of the heart, an endothelial cell of the lung, or an umbilical vein endothelial cell. Preferably, the cell is a human cell.

The present teaching also relates to a method for providing cells having stem cell characteristics comprising the steps of (i) providing a cell population comprising somatic cells, (ii) reducing the activity of RNA-dependent protein kinase (PKR) in the somatic cells, (iii) introducing RNA capable of expressing one or more factors allowing the reprogramming of the somatic cells to cells having stem cell characteristics into at least a portion of the somatic cells, and (iv) allowing the development of cells having stem cell characteristics.

In one embodiment, the RNA is introduced into the at least a portion of the somatic cells by electroporation.

In one embodiment, the RNA is *in vitro* transcribed RNA.

In one embodiment, the one or more factors comprise OCT4 and SOX2. The one or more factors may further comprise KLF4 and/or c-MYC and/or NANOG and/or LIN28. In one embodiment, the one or more factors comprise OCT4, SOX2, KLF4 and c-MYC and may further comprise LIN28. In one embodiment, the one or more factors comprise OCT4, SOX2, NANOG and LIN28.

In one embodiment, the step of reducing the activity of PKR in the cells results in an enhancement of stability and/or an enhancement of expression of the RNA in the cells, wherein the enhancement of expression of the RNA in the cells preferably comprises an increase in the level of expression and/or an increase in the duration of expression of the RNA in the cells.

In one embodiment, the step of reducing the activity of PKR in the cells comprises treating the cells with at least one PKR inhibitor. In one embodiment, the treatment of the cells with the at least one PKR inhibitor is for a time sufficient to result in an enhancement of stability and/or an enhancement of expression of the RNA in the cells. In one embodiment, the PKR inhibitor inhibits RNA-induced PKR autophosphorylation. In one embodiment, the PKR inhibitor is an ATP-binding site directed inhibitor of PKR. In one embodiment, the PKR inhibitor is an imidazolo-oxindole compound. In one embodiment, the PKR inhibitor is 6,8-dihydro-8-(1H-imidazol-5-ylmethylene)-7H-pyrrolo[2,3-g]benzothiazol-7-one or 2-amino-purine. In one embodiment, the PKR inhibitor is a virally derived inhibitor of PKR, wherein the virally derived inhibitor of PKR is preferably selected from the group consisting of vaccinia virus E3 and/or K3, or their RNA.

In one embodiment, the step of reducing the activity of PKR in the cells comprises silencing expression of the PKR gene.

In one embodiment, the method further comprises the step of culturing the somatic cells in the presence of at least one histone deacetylase inhibitor, wherein the at least one histone deacetylase inhibitor preferably comprises valproic acid, sodium butyrate, trichostatin A and/or scriptaid.

In one embodiment, step (iv) comprises culturing the somatic cells under embryonic stem cell culture conditions.

In one embodiment, the stem cell characteristics comprise an embryonic stem cell morphology.

In one embodiment, the cells having stem cell characteristics have normal karyotypes, express telomerase activity, express cell surface markers that are characteristic for embryonic stem cells and/or express genes that are characteristic for embryonic stem cells.

In one embodiment, the cells having stem cell characteristics exhibit a pluripotent state.

In one embodiment, the cells having stem cell characteristics have the developmental potential to differentiate into advanced derivatives of all three primary germ layers.

In one embodiment, the somatic cells are fibroblasts such as lung fibroblasts, foreskin fibroblasts or dermal fibroblasts. Preferably, the somatic cells are human cells.

The present teaching also relates to a method for providing differentiated cell types comprising the steps of (i) providing cells having stem cell characteristics using the method for providing cells having stem cell characteristics according to the invention, and (ii) culturing the cells having stem cell characteristics under conditions that induce or direct partial or complete differentiation to a differentiated cell type.

As described herein, inhibition of PKR in cells induces the production of interferons. Thus, the teaching also relates to a method of increasing interferon production by a cell comprising the step of reducing the activity of PKR in the cell. According to the present teaching, interferon production by a cell may be increased either *in vitro* or *in vivo.* If interferon production by a cell is increased *in vitro* the cell may be subsequently administered to a subject.

Interferons are known for their antiproliferative and apoptotic effects, their anti-angiogenic effects and their ability to modulate an immune response. Thus, the teaching also relates to a method of treating a subject, preferably a patient such as a cancer patient, comprising the step of reducing the activity of PKR in a cell of said subject such as by administering an inhibitor of expression and/or activity of PKR. Treatment of said subject may aim at modulating, preferably activating, the immune system of said subject. Treatment of said subject may aim, in particular, at achieving or enhancing an anti-cancer effect, in particular an anti-cancer immune response, in said subject. Such treatments may also be achieved by increasing interferon production by a cell *in vitro* and subsequently administering the cell to the subject. In one embodiment, the cell is an autologous cell.

The cell in the above aspects of the invention may be a cell as described herein. The interferon preferably is interferon-a and/or interferon-β, more preferably interferon-a. Activity of PKR may be reduced in the cell as described herein. Furthermore, when reducing the activity of PKR in the above aspects of the invention, RNA may be introduced into the cell, e.g. as described herein, or RNA may not be introduced into the cell. If RNA is introduced into the cell, the RNA may be RNA as described herein.

### BRIEF DESCRIPTION OF THE DRAWINGS

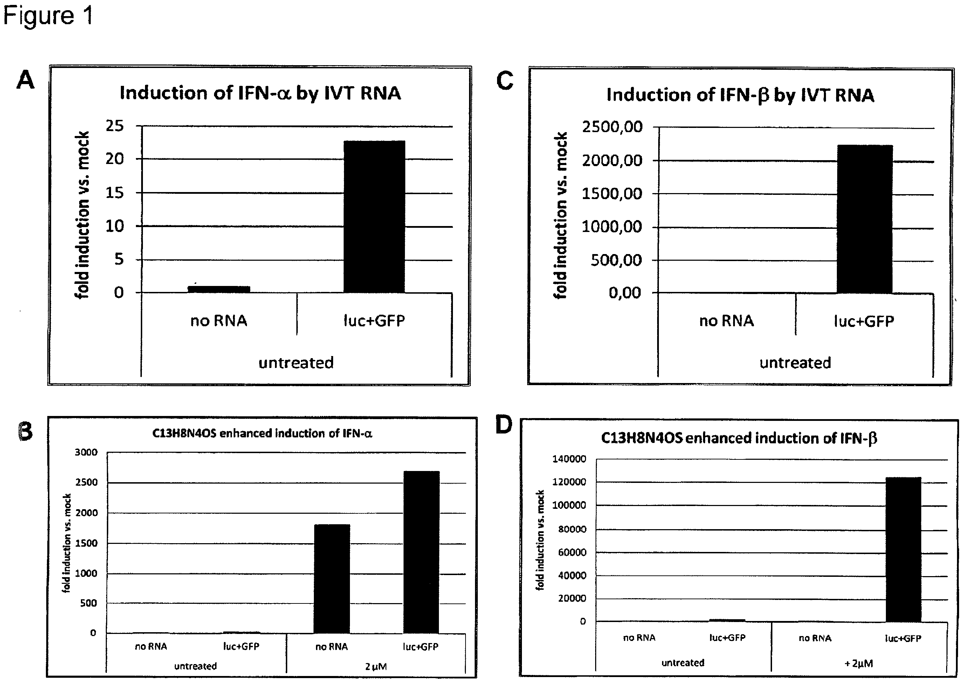

**Figure 1****: Induction of interferons by IVT RNA**
   Primary human foreskin fibroblasts (CCD1079SK) were electroporated with 1 µg IVT RNA encoding for firefly luciferase and 5 µg IVT RNA encoding for destabilized GFP (luc+GFP). The cells were either left untreated or incubated with 2 µM C₁₃H₈N₄OS (PKR-inhibitor). Upregulation of interferon-alpha and interferon-betatranscripts in human fibroblasts induced by RNA electroporation was observed (Fig 1A and 1C). The inhibition of PKR using C₁₃H₈N₄OS resulted in an overwhelming increase of interferon transcripts (Fig 1B and 1D).

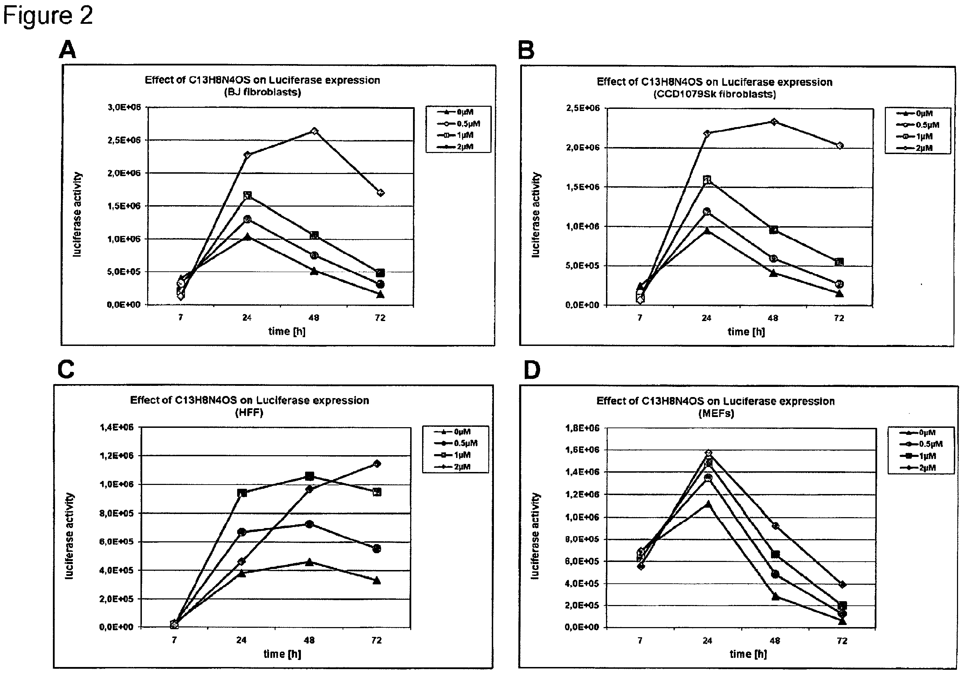

**Figure 2****: Dose dependent increase of luciferase expression by inhibiting PKR**
   **(A-C)** Primary human foreskin fibroblasts from different donors and suppliers (panel A: BJ fibroblasts; panel B CCD1079Sk; panel C: HFF) and (**D**) murine embryonic fibroblasts (MEFs) were electroporated with 0.5 µg IVT RNA encoding for firefly luciferase and 1.25 µg IVT RNA encoding for enhanced GFP (eGFP). The cells were plated in duplicates into 96-well-plates at a density of 30000 cells/well. The cells were either left untreated or incubated with increasing amounts of C₁₃H₈N₄OS (PKR-inhibitor) ranging from 0.5 µM to 2 µM as indicated in the panel legends. Luciferase activity was measured at the indicated time points. Mean values of the duplicates are given. A dose dependent increase of the reporter gene luciferase was observed.

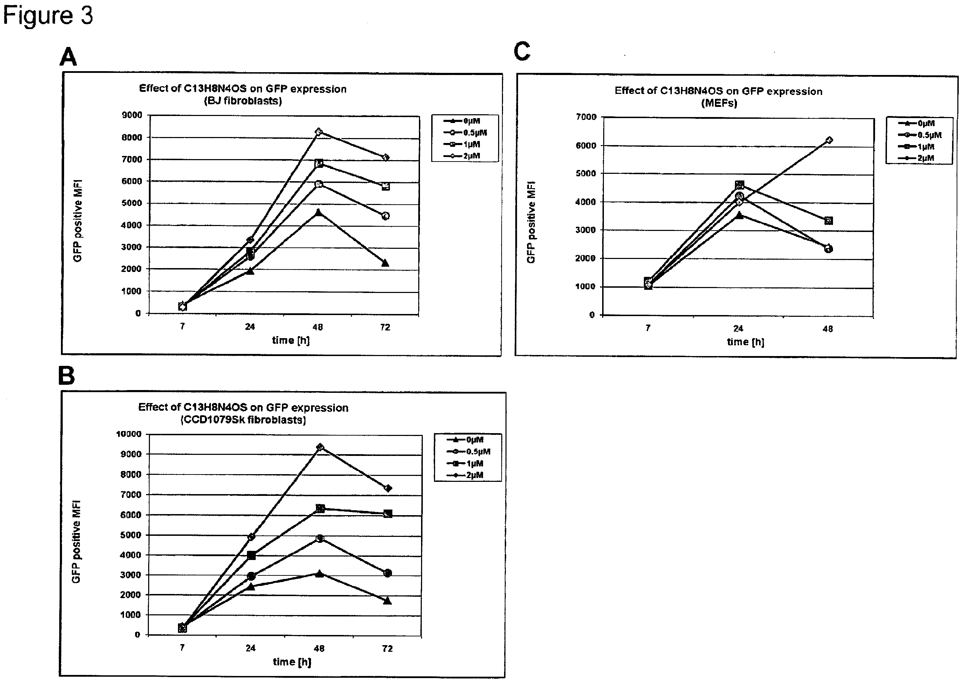

**Figure 3****: Dose dependent increase of GFP expression by inhibiting PKR**
   **(AB)** Primary human foreskin fibroblasts from different donors and suppliers (panel A: BJ fibroblasts; panel B CCD1079Sk) and (C) MEFs were electroporated with 0.5 µg IVT RNA encoding for firefly luciferase and 1.25 µg IVT RNA encoding for enhanced GFP (eGFP). The cells were plated into 96-well-plates at a density of 250000 cells/well. The cells were either left untreated or incubated with increasing amounts of C₁₃H₈N₄OS (PKR-inhibitor) ranging from 0.5 µM to 2 µM as indicated in the panel legends. GFP expression was measured by flow cytometry at the indicated time points. The mean fluorescence intensity (MFI) of the transfected GFP-positive cell fractions is shown. A dose dependent increase of the reporter gene GFP was observed.

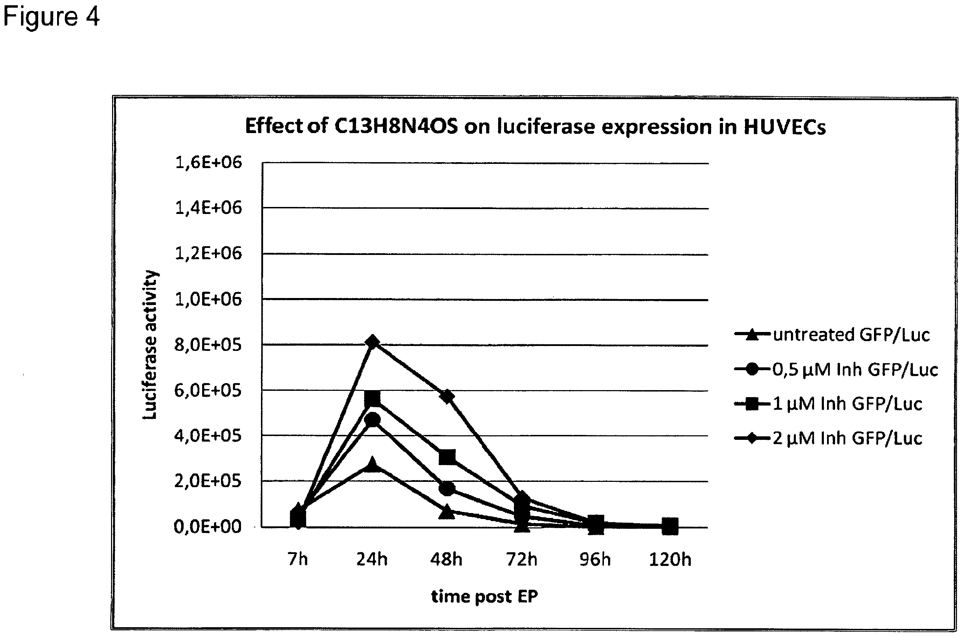

**Figure 4****: Effect of PKR inhibition on IVT RNA-based reporter gene expression in primary human umbilical vein endothelial cells**
   Primary human umbilical vein endothelial cells (HUVECs) were electroporated with 1 µg IVT RNA encoding firefly luciferase and 5 µg IVT RNA encoding for destabilized GFP. The cells were plated in duplicates into 96-well-plates at a density of 10000 cells/well. The cells were either left untreated or incubated with increasing amounts of C₁₃H₈N₄OS (PKR-inhibitor) ranging from 0.5 µM to 2 µM as indicated in the panel legend. Luciferase activity was measured at the indicated time points. Mean values of the duplicates are given. Luciferase expression was enhanced in HUVECs in a dose dependent manner.

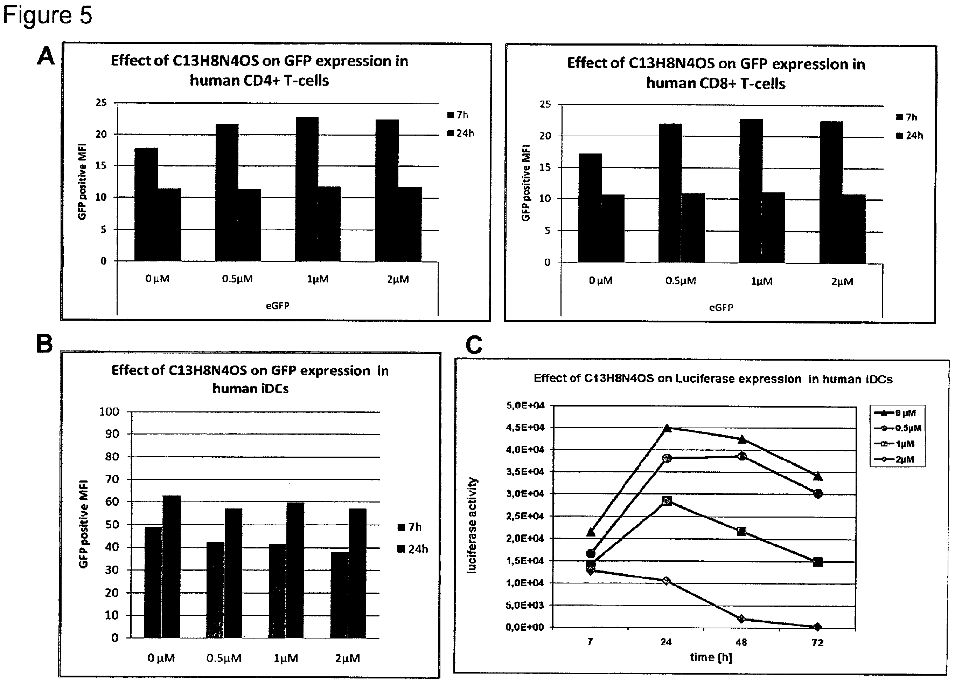

**Figure 5****: Effect of PKR inhibition on IVT RNA-based reporter gene expression in transfected primary CD4+ and CD8+ T-cells and immature dendritic cells**
   Human primary CD4 and CD 8 positive T-cells, as well as immature dendritic cells (iDCs) were isolated and electroporated with 5 µg IVT RNA encoding for destabilized GFP. iDCs were co-electroporated with 1 µg IVT RNA encoding for firefly luciferase. The cells were either left untreated or incubated with increasing amounts of C₁₃H₈N₄OS (PKR-inhibitor) ranging from 0.5 µM to 2 µM as indicated in the panel legends. **(A)** T-cells and **(B)** iDCs were harvested at the indicated time points and GFP expression was assessed by flow cytometry. The mean fluorescence intensity (MFI) of the transfected, GFP-positive cell fractions is shown. (C) For luciferase assays, iDCs were plated in duplicates into 96-well-plates at a density of 10000 cells/well. Luciferase activity was measured at the indicated time points. Mean values of the duplicates are given. Neither in T-cells nor in iDCs PKR inhibition led to an increased GFP expression (Fig. 5A, B). In iDCs, the inhibition of PKR resulted in loss of luciferase expression (Fig 5C)

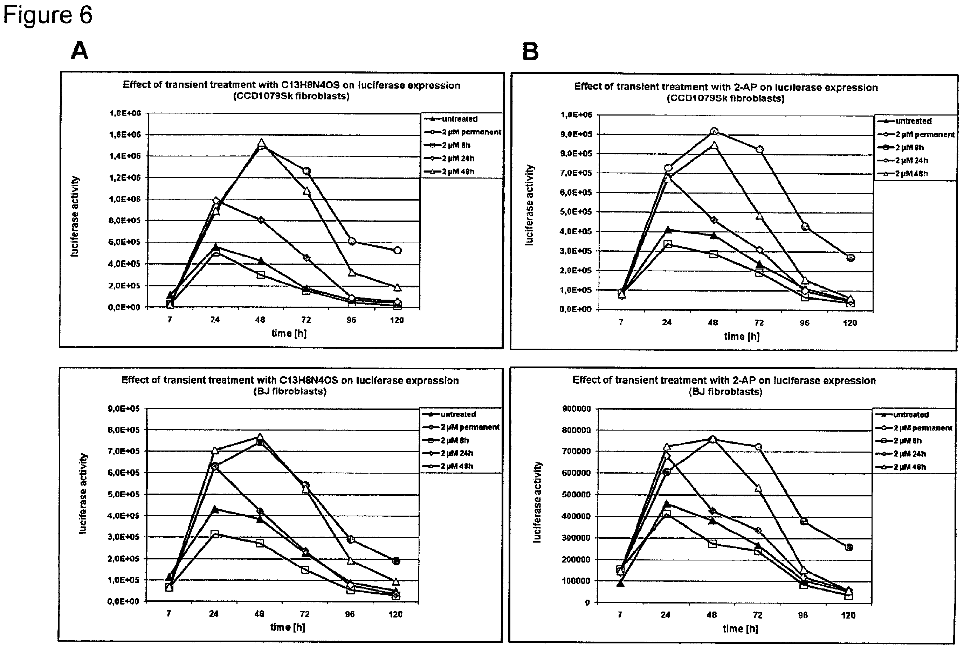

**Figure 6****: Transient inhibition of PKR after electroporation**
   BJ and GCD1079SK fibroblasts were electroporated with 0.5 µg IVT RNA encoding for firefly luciferase and 2.5 µg IVT RNA encoding for eGFP. Electroporations were performed in 2 mm gap cuvettes using optimized parameters for each cell type. 10000 cells/well were plated in duplicates into 96-well-plates. The cells were either left untreated or incubated with (A) 2 µM C₁₃H₈N₄OS or **(B)** 2 µM 2-Aminopurine (2-AP) for 8h, 24h 48h or permanently as indicated in the panel legends. Luciferase activity was measured collectively 72h after electroporation. Mean values of the duplicates are given. These data show that PKR inhibition is reversible. Permanent inhibition results in high luciferase expression levels for 5 days.

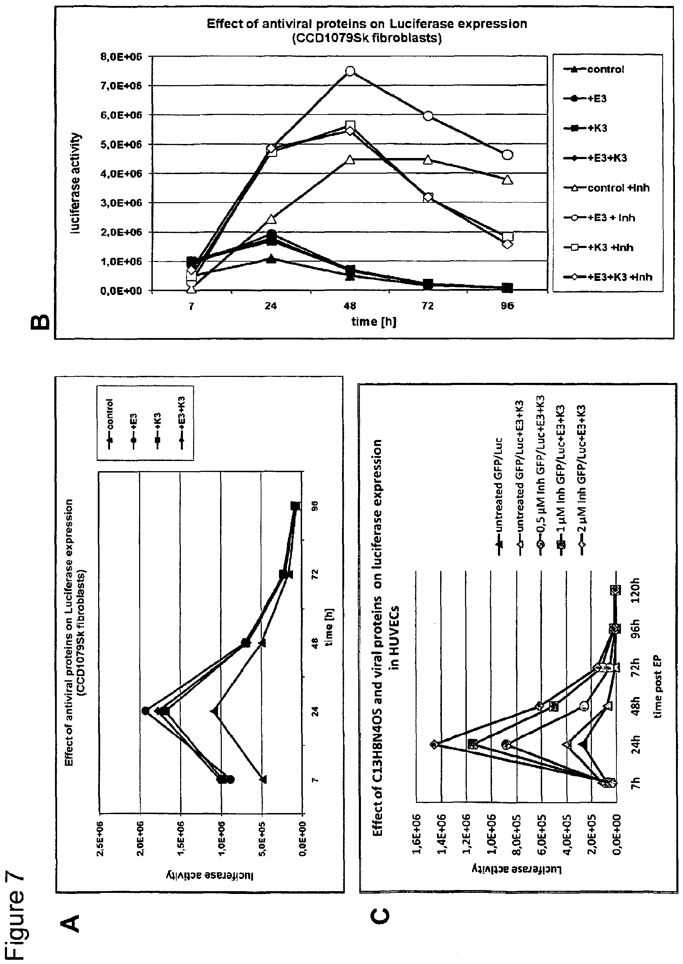

**Figure 7****: Inhibition of PKR using vaccinia virus proteins E3 and K3 cotransfected into fibroblasts and HUVECs**
   CCD1079SK fibroblasts and HUVECs were electroporated with IVT RNA encoding firefly luciferase (1 µg), destabilized GFP (5 µg) and 3 µg of E3 or K3 or both as indicated. Electroporations were performed using optimized parameters for each cell type. 10000 cells/well were plated in duplicates into 96-well-plates. CCD1079Sk were either (A) left untreated (closed symbols) or **(B)** incubated with 2 µM C₁₃H₈N₄OS (open symbols). (C) HUVECs were either left untreated or incubated with increasing concentrations of C₁₃H₈N₄OS as indicated. Luciferase activity was measured at the indicate time points after electroporation. Mean values of the duplicates are given. Both viral proteins led to a 2 fold increase in luciferase expression 24h after electroporation. (Fig. 7A). The additional application of C₁₃H₈N₄OS resulted in an additive increase of luciferase expression and a stabilization (Fig 7B). Similar experiments with HUVEC cells revealed that the combination of E3 and K3 increased luciferase expression, which was additively enhanced by C₁₃H₈N₄OS in a dose dependent fashion (Fig 7C).

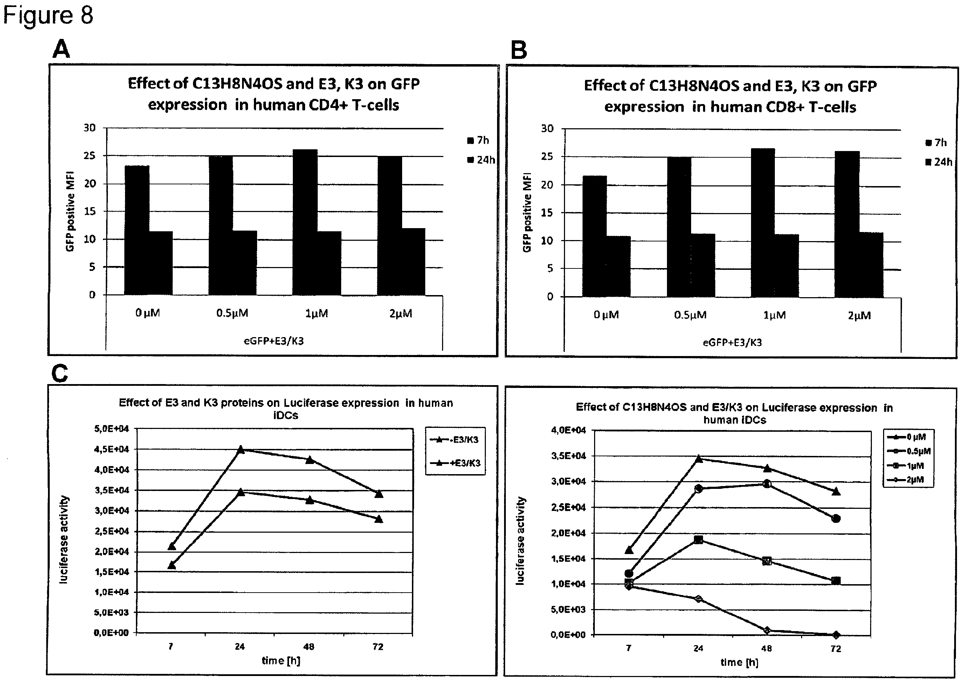

**Figure 8****: Inhibition of PKR using vaccinia virus proteins E3 and K3 cotransfected into human T-cells and iDCs**
   **(A)** Human primary CD4 and **(B)** CD8 positive T-cells were isolated and electroporated with IVT RNA encoding destabilized GFP (10 µg) and 5 µg of both, E3 and K3. Electroporations were performed using 4 mm gap cuvettes and optimized parameters for each cell type. The cells were either left untreated or incubated with increasing concentrations of C₁₃H₈N₄OS. The cells were harvested at the indicated time points and GFP expression was assessed by flow cytometry. The mean fluorescence intensity (MFI) of the transfected, GFP-positive cell fractions is shown. **(C)** Human iDCs were isolated and electroporated with IVT RNA encoding destabilized GFP (10 µg), 1 µg luciferase and 5 µg of both, E3 and K3. Electroporations were performed using 4 mm gap cuvettes and optimized parameters for each cell type. The cells were plated in duplicates into 96-well-plates at a density of 10000 cells/well and either left untreated (left panel) or incubated with increasing concentrations of C₁₃H₈N₄OS (right panel). Luciferase activity was measured at the indicated time points. Mean values of the duplicates are given. As depicted in Fig. 8A ands 8B, viral proteins inhibiting PKR did not increase reporter gene expression in T-cells, neither in presence nor in absence of C₁₃H₈N₄OS. In iDCs, viral proteins - as observed before with the inhibitor - decreased the luciferase expression. Also, E3 and K3 did not revert the negative effect of C₁₃H₈N₄OS on luciferase expression (Fig. 8C).

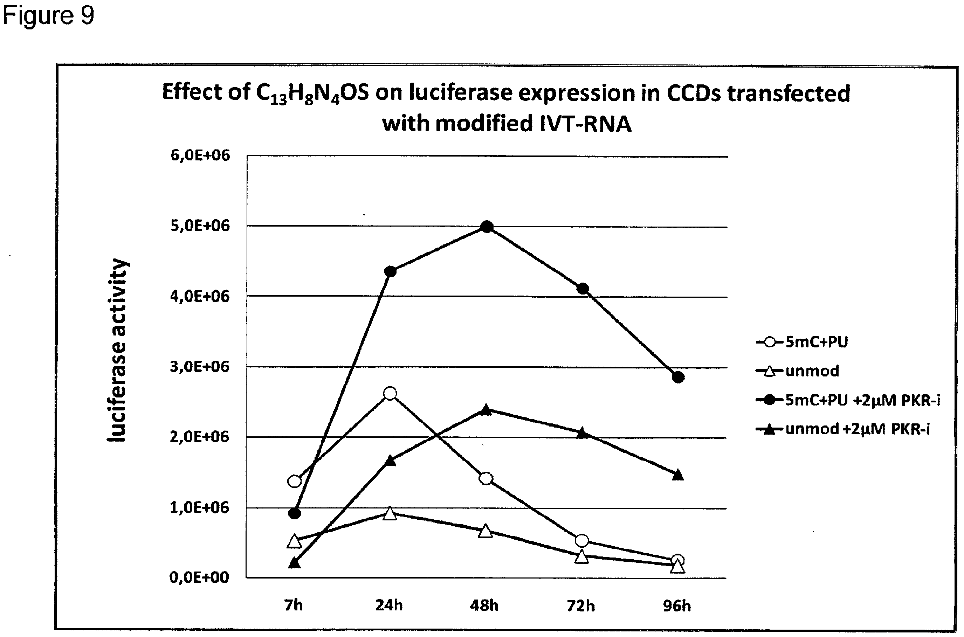

**Figure 9****: Effect of PKR inhibition on reporter gene expression in fibroblasts transfected with RNA composed of modified nucleotides**
   **(A)** CCD1079Sk fibroblasts were co-electroporated with 1 µg IVT-RNA encoding luciferase and 5 µg IVT RNA encoding destabilized GFP, either unmodified (unmod) or modified with 5-methylcytidine (5mC) and pseudouridine (PU) as indicated. Immediately after electroporation, the cells were plated in duplicates into 96-well-plates at a density of 10000 cells/well. Cells remained either untreated or were cultivated in presence of 2 µM PKR-inhibitor. After 7h, 24h, 48h, and 72h luciferase activity was assessed. The incorporation of 5mC and PU into the transcripts increases the luciferase expression by about 2-fold as compared to the unmodified control. The use of 2 µM C₁₃H₈N₄OS further increases and stabilizes the expression of luciferase encoded by modified IVT RNA.

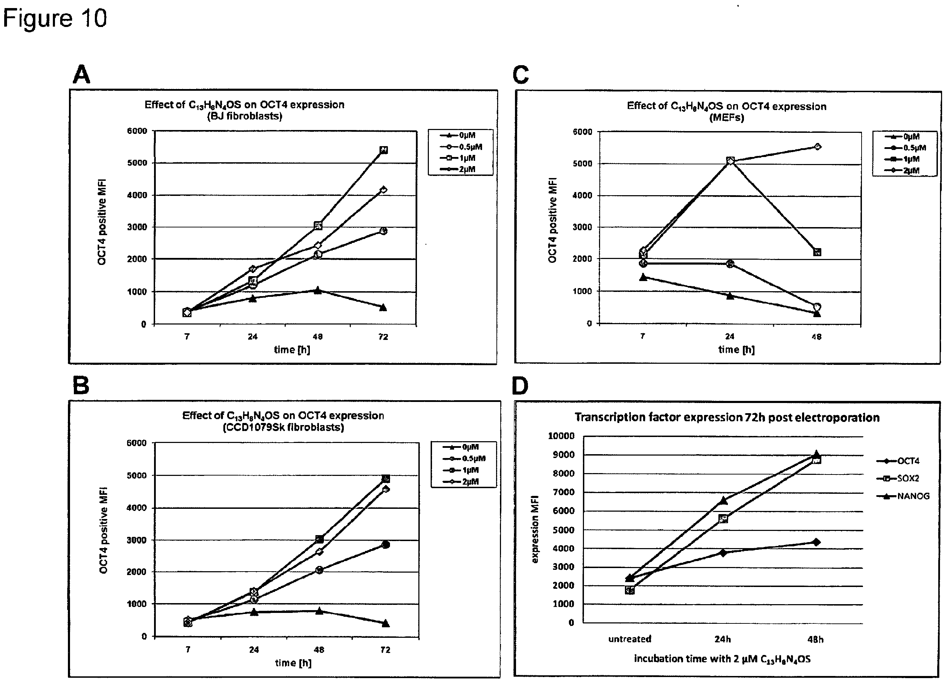

**Figure 10****: Increased and stabilized expression of reprogramming transcription factors by PKR-Inhibition**
   **(AB)** Primary human foreskin fibroblasts from different donors and suppliers (panel A: BJ fibroblasts; panel B: CCD1079Sk) and (C) MEFs were electroporated with 0.5 µg IVT RNA encoding for firefly luciferase and 1.25 µg IVT RNA encoding for eGFP and 2.5 µg of IVT RNA encoding for each of the individual reprogramming factors OCT4, SOX2, KLF4 and c-MYC. Electroporations were performed in 2 mm gap cuvettes using optimized parameters for each cell type. The cells were plated into 6-well-plates at a density of 250000 cells/well. The cells were either left untreated or incubated with increasing amounts of C₁₃H₈N₄OS (PKR-inhibitor) ranging from 0.5 µM to 2 µM as indicated in the panel legend. The OCT4 expression was detected by intracellular staining with a fluorescently labeled antibody and measured by flow cytometry at the indicated time points. The mean fluorescence intensity (MFI) of the transfected OCT4-positive cell fractions is shown. **(D)** CCD1079Sk fibroblasts were electroporated with IVT RNA as in B and treated for 24h or 48h with 2 µM C₁₃H₈N₄OS. 72h post electroporation the cells were harvested and intracellular staining with fluorescent labeled antibodies reactive against the indicated transcription factors were performed. The MFI of the transcription factor-positive cell fractions are shown. The expression of the transcription factors OCT4, Nanog and SOX2 was strongly increased and stabilized in treated cells in a dose and time dependent manner.

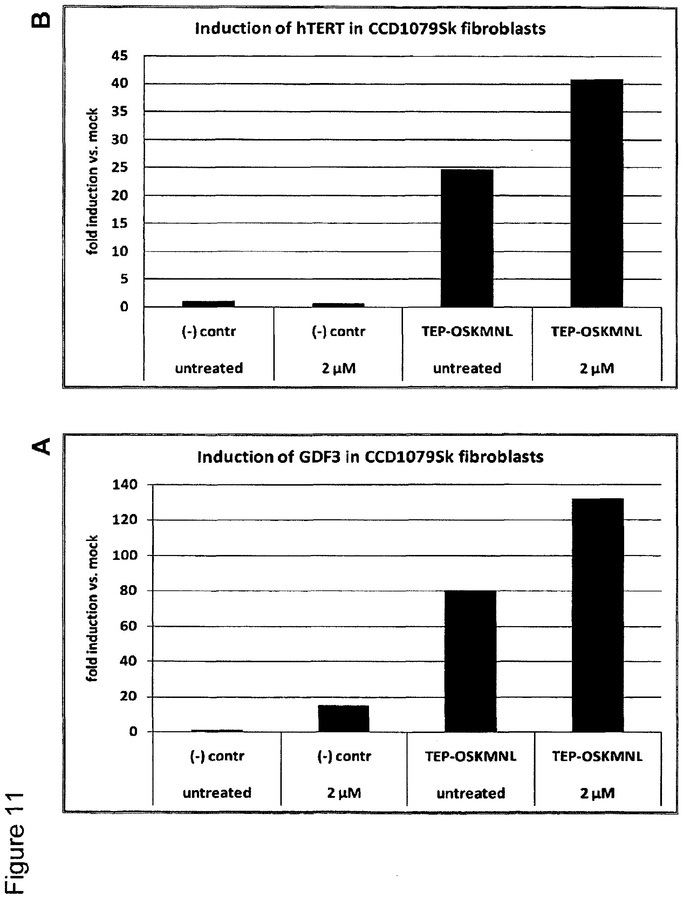

**Figure 11****: Enhanced induction of pluripotency marker genes upon treatment with C₁₃H₈N₄OS**
   CCD1079SK fibroblasts were either mock electroporated or electroporated with IVT RNA encoding for firefly luciferase (1µg), eGFP (1.25µg), SV40 Large T (1.25µg), HPV16-E6 (1.25µg), OCT4, SOX2, KLF4, cMYC, NANOG and LIN28 (2.5µg OCT4 and equimolar amounts for the others). Electroporations were performed in 2 mm gap cuvettes using optimized parameters for CCDs. The cells were plated into 6-well-plates at a density of 250000 cells/well and either left untreated or incubated with 2 µM C₁₃H₈N₄OS as indicated. 72h after electroporation the cells were harvested to extract total RNA and perform qRT-PCR analysis for the pluripotency markers **(A)** GDF3 and **(B)** hTERT. GDF3 and hTERT induction was increased by the treatment 72h after electroporation.

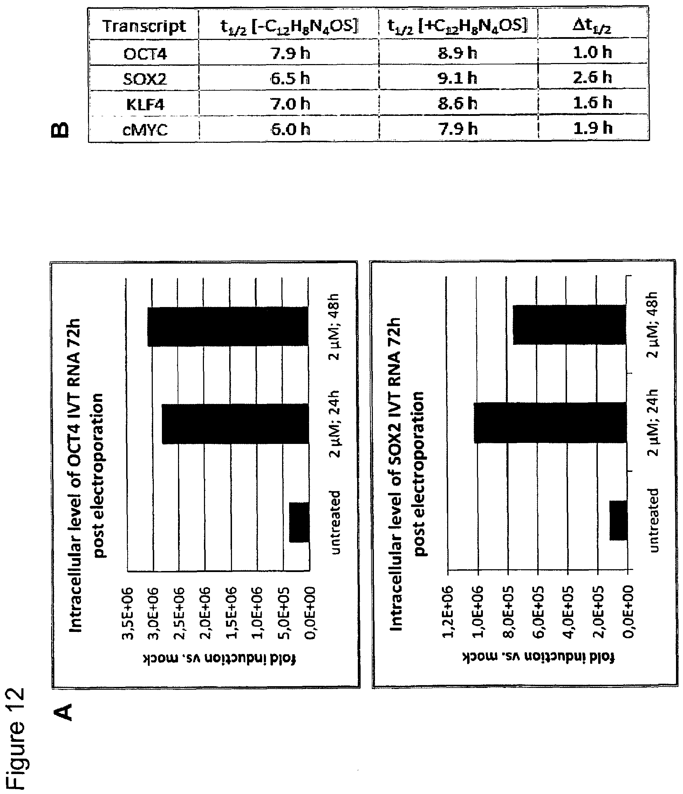

**Figure 12****: Stabilization of IVT-RNA constructs under inhibition of PKR**
   **(A)** CCD1079SK fibroblasts were either mock electroporated or electroporated with IVT RNA encoding for firefly luciferase (1µg), eGFP (1.25µg), SV40 Large T (1.25µg), HPV16-E6 (1.25µg), OCT4, SOX2, KLF4, cMYC, NANOG and LIN28 (2.5µg OCT4 and equimolar amounts for the others). Electroporations were performed in 2 mm gap cuvettes using optimized parameters for CCDs. After electroporation cells were either left untreated or incubated with 2 µM C₁₃H₈N₄OS for 24h or 48h. After 72h cells were harvested to extract total RNA and perform qRT-PCR analysis for the codon optimized IVT-RNA constructs OCT4 and SOX2. **(B)** To determine whether C₁₃H₈N₄OS has an effect on the *in vivo* half-life of IVT-RNA, CCD1079SK fibroblasts were mock electroporated or electroporated with IVT RNA encoding for firefly luciferase (1µg), SV40 Large T (1.25µg), enhanced GFP (1.25µg), E3 (3µg) and K3 (3µg) with addition of IVT-RNA encoding for OCT4, SOX2, KLF4, cMYC (each 2.5µg) or addition of eGFP (10µg) as control. Electroporations were performed in 2 mm gap cuvettes using optimized parameters for CCDs. 250000 cells/well were plated into 6-well-plates and either left untreated or incubated with 2 µM C₁₃H₈N₄OS for 80h. After 8h, 32h, 56h, 80h cells were harvested to extract total RNA and perform qRT-PCR analysis for the codon optimized IVT-RNA constructs OCT4, SOX2, KLF4 and cMYC. Decay of transcripts was plotted against time and difference between half-lifes (t_{1/2}) of the construct in absence or presence of C₁₃H₈N₄OS was calculated (Δt_{1/2}= t_{1/2}(+C₁₃H₈N₄OS) - t_{1/2}(-C₁₃H₈N₄OS)). The transient treatment of cells with 2 µM C₁₃H₈N₄OS increased the abundance of IVT RNA for 72h (A). The half life of the IVT RNA constructs is prolonged for 1-2.5h under PKR-Inhibition (B). Stabilization of reporter expression under PKR-inhibition is therefore to some extent based on stabilization of IVT-RNA constructs.

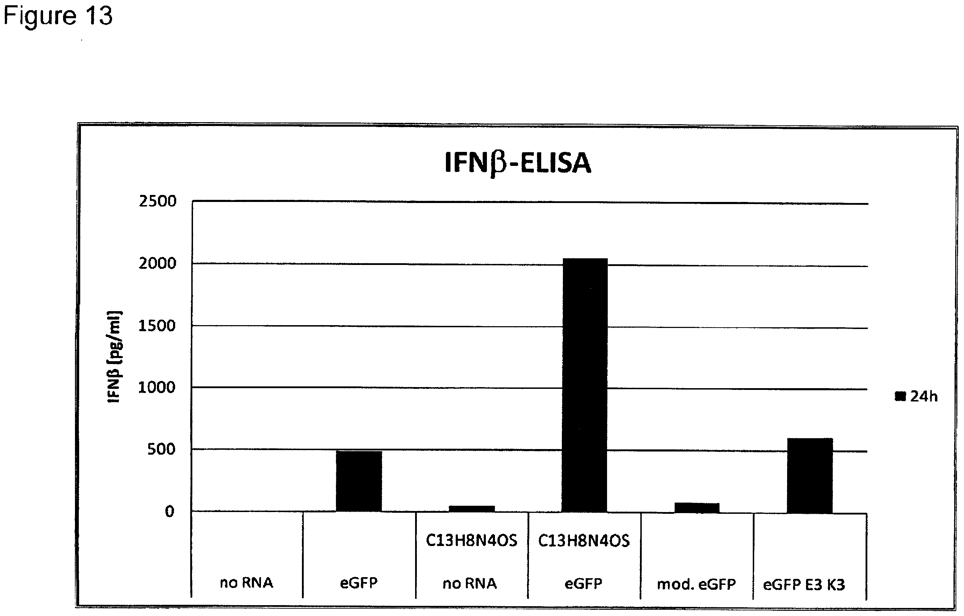

**Figure 13****: Secretion of interferons after electroporation of IVT-RNA and under the effect of C₁₃H₈N₄OS**
   Primary human foreskin fibroblasts (HFF) were electroporated with IVT RNA encoding eGFP (10µg) either unmodified or modified (mod.) with 5-methylcytidine (5mC) and pseudouridine (PU) or unmodified eGFP (10 µg) and with 3 µg of modified (5mC and PU) E3 and K3 as indicated.
   IVT-RNA induces the secretion of IFNβ after electroporation. The secretion is remarkably enhanced when cells are incubated with C₁₃H₈N₄OS. Induction of IFNβ is reduced when IVT-RNA is modified with 5mC and PU.

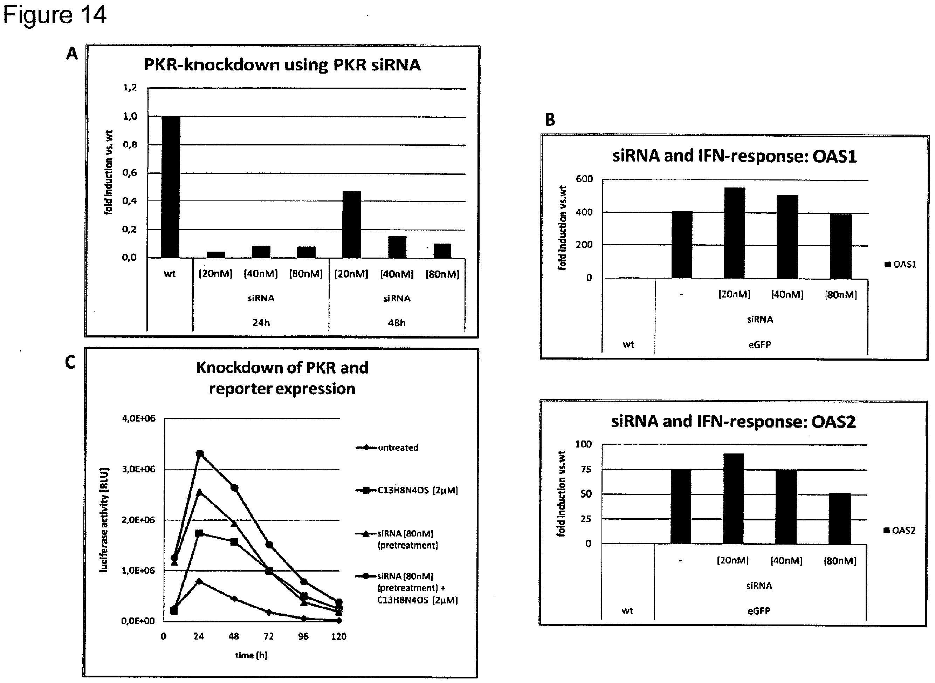

**Figure 14****: PKR-knockdown leads to a stabilization of transcript expression similar to PKR-inhibition**
   (A) Primary human foreskin fibroblasts (CCD1079Sk) were electroporated with an increasing amount of a siRNA-mix targeting PKR (Santa Cruz; sc-36263) ranging from 20nM to 80nM. The expression level of PKR was assessed by qRT-PCR. Depicted are the relative expression levels compared to wildtype cells. PKR is knocked down to 5-10% with all concentrations of siRNA-mix 24h post electroporation. Only 80nM are sufficient to knock down PKR over 48h.
   **(B)** Primary human foreskin fibroblasts (CCD1079Sk) were electroporated with IVT RNA encoding unmodified eGFP (1.5µg) either alone or with an increasing amount of a siRNA-mix targeting PKR ranging from 20nM to 80nM. The expression level of the IFN-response genes OAS1 and OAS2 was assessed by qRT-PCR. Depicted are the relative expression levels compared to wildtype cells. Expression levels of OAS1 and OAS2 are not significantly altered by addition of siRNA-mix indicating that the siRNA is not inducing IFNs after electroporation.
   (C) Primary human foreskin fibroblasts (CCD1079Sk) were electroporated with or without 80nM of siRNA-mix targeting PKR and 48h later with 1 µg IVT RNA encoding for firefly luciferase and for eGFP (2.5µg). Cells were either left untreated or incubated with 2µM of C₁₃H₈N₄OS. Luciferase activity was measured at the indicated time points. Electroporation of cells preincubated with siRNA targeting PKR and therefore being in a state of lacking PKR leads to a quite similar kinetic and increase of reporter gene expression as incubation with 2µM of the PKR-inhibitor C₁₃H₈N₄OS with a slightly lower stabilizing effect. Combination of preincubation with siRNA-mix targeting PKR and the use of the inhibitor leads to an even higher expression level of the reporter transcript.

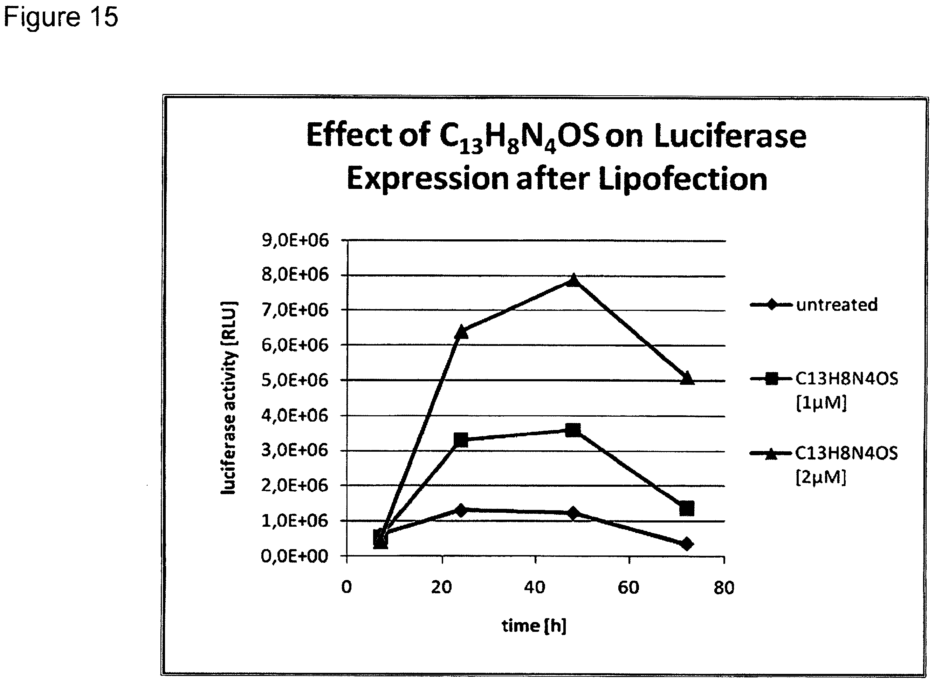

**Figure 15****: Effect of C₁₃H₈N₄OS on translation of IVT RNA using lipofection**
   1.2 µg 5'-triphosphorylated and non-modified IVT RNA (0.8µg encoding luciferase, 0.4µg GFP) was packaged with 6µl RNAiMAX transfection reagent (Invitrogen) and transfected into human foreskin fibroblasts and the medium supplemented with increasing concentrations of C₁₃H₈N₄OS. At the indicated time points the cells were lysed and luciferase activity of the lysates was measured. C₁₃H₈N₄OS had a stabilizing effect on translation of luciferase. This effect was dose-dependent.

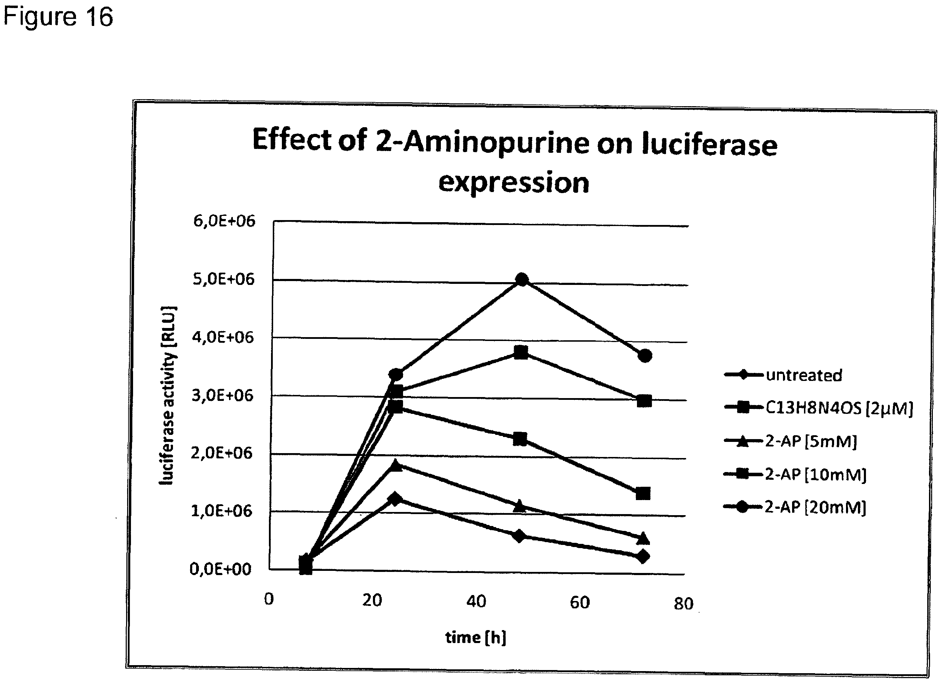

**Figure 16****: Effect of 2-aminopurine on luciferase expression**
   Human foreskin fibroblasts were electroporated with 2µg IVT RNA coding for firefly luciferase and 5 µg IVT RNA coding for GFP and incubated with the concentrations of 2-AP indicated in the panel legend. 10 mM to 20 mM 2-AP lead to a similar increase of translation as 2 µM C₁₃H₈N₄OS.

### DETAILED DESCRIPTION OF THE INVENTION

Although the present invention is described in detail below, it is to be understood that the teaching as such is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present teaching will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present teaching unless the context indicates otherwise. For example, if in a preferred embodiment RNA comprises a poly(A)-tail consisting of 120 nucleotides and in another preferred embodiment the RNA molecule comprises a 5'-cap analog, then in a preferred embodiment, the RNA comprises the poly(A)-tail consisting of 120 nucleotides and the 5'-cap analog.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Terms such as "reducing" or "inhibiting" relate to the ability to cause an overall decrease, preferably of 5% or greater, 10% or greater, 20% or greater, more preferably of 50% or greater, and most preferably of 75% or greater, in the level. The term "inhibit" or similar phrases includes a complete or essentially complete inhibition, i.e. a reduction to zero or essentially to zero.

Terms such as "increasing", "enhancing", or "prolonging" preferably relate to an increase, enhancement, or prolongation by about at least 10%, preferably at least 20%, preferably at least 30%, preferably at least 40%, preferably at least 50%, preferably at least 80%, preferably at least 100%, preferably at least 200% and in particular at least 300%. These terms may also relate to an increase, enhancement, or prolongation from zero or a non-measurable or non-detectable level to a level of more than zero or a level which is measurable or detectable.

The term "recombinant" in the context of the present invention means "made through genetic engineering". Preferably, a "recombinant entity" such as a recombinant protein in the context of the present invention is not occurring naturally, and preferably is a result of a combination of entities such as amino acid or nucleic acid sequences which are not combined in nature. For example, a recombinant protein in the context of the present invention may contain several amino acid sequences derived from different proteins fused together, e.g., by peptide bonds.

The term "naturally occurring" as used herein refers to the fact that an object can be found in nature. For example, a protein or nucleic acid that is present in an organism (including viruses) and can be isolated from a source in nature and which has not been intentionally modified by man in the laboratory is naturally occurring.

A nucleic acid is according to the teaching preferably deoxyribonucleic acid (DNA) or ribonucleic acid (RNA), more preferably RNA, most preferably *in vitro* transcribed RNA (IVT RNA). Nucleic acids include according to the invention genomic DNA, cDNA, mRNA, recombinantly produced and chemically synthesized molecules. According to the teaching, a nucleic acid may be present as a single-stranded or double-stranded and linear or covalently circularly closed molecule. A nucleic acid can, according to the teaching, be isolated. The term "isolated nucleic acid" means, according to the invention, that the nucleic acid (i) was amplified *in vitro,* for example via polymerase chain reaction (PCR), (ii) was produced recombinantly by cloning, (iii) was purified, for example, by cleavage and separation by gel electrophoresis, or (iv) was synthesized, for example, by chemical synthesis. A nucleic can be employed for introduction into, i.e. transfection of, cells, in particular, in the form of RNA which can be prepared by *in vitro* transcription from a DNA template. The RNA can moreover be modified before application by stabilizing sequences, capping, and polyadenylation.

As a nucleic acid, in particular RNA, for expression of more than one peptide or protein, either of a nucleic acid type in which the different peptides or proteins are present in different nucleic acid molecules or a nucleic acid type in which the peptides or proteins are present in the same nucleic acid molecule can be used.

In the context of the present teaching, the term "RNA" relates to a molecule which comprises at least one ribonucleotide residue and preferably being entirely or substantially composed of ribonucleotide residues. "Ribonucleotide" relates to a nucleotide with a hydroxyl group at the 2'-position of a β-D-ribofuranosyl group. The term "RNA" comprises double-stranded RNA, single-stranded RNA, isolated RNA such as partially or completely purified RNA, essentially pure RNA, synthetic RNA, and recombinantly generated RNA such as modified RNA which differs from naturally occurring RNA by addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

According to the present teaching, the term "RNA" includes and preferably relates to "mRNA". The term "mRNA" means "messenger-RNA" and relates to a "transcript" which is generated by using a DNA template and encodes a peptide or protein. Typically, an mRNA comprises a 5'-UTR, a protein coding region, and a 3'-UTR. mRNA only possesses limited half-life in cells and *in vitro.* In the context of the present invention, mRNA may be generated by *in vitro* transcription from a DNA template. The *in vitro* transcription methodology is known to the skilled person. For example, there is a variety of *in vitro* transcription kits commercially available.

According to the teaching, the stability and translation efficiency of RNA may be modified as required. For example, RNA may be stabilized and its translation increased by one or more modifications having a stabilizing effects and/or increasing translation efficiency of RNA. Such modifications are described, for example, in PCT/EP2006/009448 (WO2007/036366). In order to increase expression of the RNA used according to the present teaching, it may be modified within the coding region, i.e. the sequence encoding the expressed peptide or protein, preferably without altering the sequence of the expressed peptide or protein, so as to increase the GC-content to increase mRNA stability and to perform a codon optimization and, thus, enhance translation in cells.

The term "modification" in the context of the RNA used in the present teaching includes any modification of an RNA which is not naturally present in said RNA.

In one embodiment, the RNA used according to the invention does not have uncapped 5'-triphosphates. Removal of such uncapped 5'-triphosphates can be achieved by treating RNA with a phosphatase.

The RNA may have modified ribonucleotides in order to increase its stability and/or decrease cytotoxicity. For example, in one embodiment, in the RNA used according to the invention 5-methylcytidine is substituted partially or completely, preferably completely, for cytidine. Alternatively or additionally, in one embodiment, in the RNA used according to the invention pseudouridine is substituted partially or completely, preferably completely, for uridine.

In one embodiment, the term "modification" relates to providing an RNA with a 5'-cap or 5'-cap analog. The term "5'-cap" refers to a cap structure found on the 5'-end of an mRNA molecule and generally consists of a guanosine nucleotide connected to the mRNA via an unusual 5' to 5' triphosphate linkage. In one embodiment, this guanosine is methylated at the 7-position. The term "conventional 5'-cap" refers to a naturally occurring RNA 5'-cap, preferably to the 7-methylguanosine cap (m⁷G). In the context of the present invention, the term "5'-cap" includes a 5'-cap analog that resembles the RNA cap structure and is modified to possess the ability to stabilize RNA and/or enhance translation of RNA if attached thereto, preferably *in vivo* and/or in a cell.

Preferably, the 5' end of the RNA includes a Cap structure having the following general formula: 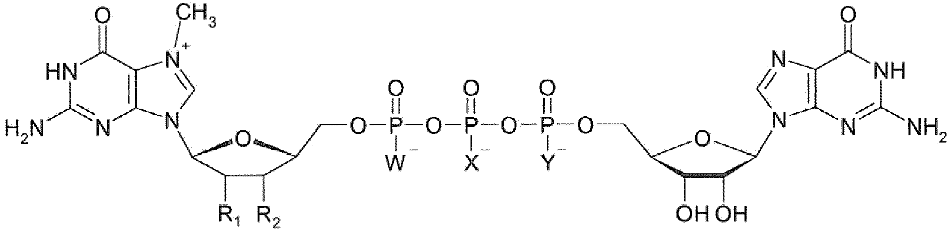 wherein R₁ and R₂ are independently hydroxy or methoxy and W⁻, X⁻ and Y⁻ are independently oxygen, sulfur, selenium, or BH₃. In a preferred embodiment, R₁ and R₂ are hydroxy and W⁻, X⁻ and Y⁻ are oxygen. In a further preferred embodiment, one of R₁ and R₂, preferably R₁ is hydroxy and the other is methoxy and W⁻, X⁻ and Y⁻ are oxygen. In a further preferred embodiment, R₁ and R₂ are hydroxy and one of W⁻, X⁻ and Y⁻, preferably X⁻ is sulfur, selenium, or BH₃, preferably sulfur, while the other are oxygen. In a further preferred embodiment, one of R₁ and R₂, preferably R₂ is hydroxy and the other is methoxy and one of W⁻, X⁻ and Y⁻, preferably X⁻ is sulfur, selenium, or BH₃, preferably sulfur while the other are oxygen.

In the above formula, the nucleotide on the right hand side is connected to the RNA chain through its 3' group.

Those Cap structures wherein at least one of W⁻, X⁻ and Y⁻ is sulfur, i.e. which have a phosphorothioate moiety, exist in different diastereoisomeric forms all of which are encompassed herein. Furthermore, the present invention encompasses all tautomers and stereoisomers of the above formula.

For example, the Cap structure having the above structure wherein R₁ is methoxy, R₂ is hydroxy, X⁻ is sulfur and W⁻ and Y⁻ are oxygen exists in two diastereoisomeric forms (Rp and Sp). These can be resolved by reverse phase HPLC and are named D1 and D2 according to their elution order from the reverse phase HPLC column. According to the invention, the D1 isomer of m₂^{7,2'-*O*}Gpp_{S}pG is particularly preferred.

Providing an RNA with a 5'-cap or 5'-cap analog may be achieved by *in vitro* transcription of a DNA template in presence of said 5'-cap or 5'-cap analog, wherein said 5'-cap is co-transcriptionally incorporated into the generated RNA strand, or the RNA may be generated, for example, by *in vitro* transcription, and the 5'-cap may be attached to the RNA post-transcriptionally using capping enzymes, for example, capping enzymes of vaccinia virus.

The RNA may comprise further modifications. For example, a further RNA modification may be an extension or truncation of the naturally occurring poly(A) tail or an alteration of the 5'- or 3'-untranslated regions (UTR) such as introduction of a UTR which is not related to the coding region of said RNA, for example, the exchange of the existing 3'-UTR with or the insertion of one or more, preferably two copies of a 3'-UTR derived from a globin gene, such as alpha2-globin, alphal-globin, beta-globin, preferably beta-globin, more preferably human beta-globin.

RNA having an unmasked poly-A sequence is translated more efficiently than RNA having a masked poly-A sequence. The term "poly(A) tail" or "poly-A sequence" relates to a sequence of adenyl (A) residues which typically is located on the 3'-end of a RNA molecule and "unmasked poly-A sequence" means that the poly-A sequence at the 3' end of an RNA molecule ends with an A of the poly-A sequence and is not followed by nucleotides other than A located at the 3' end, i.e. downstream, of the poly-A sequence. Furthermore, a long poly-A sequence of about 120 base pairs results in an optimal transcript stability and translation efficiency of RNA.

Therefore, in order to increase stability and/or expression of the RNA used according to the present teaching, it may be modified so as to be present in conjunction with a poly-A sequence, preferably having a length of 10 to 500, more preferably 30 to 300, even more preferably 65 to 200 and especially 100 to 150 adenosine residues. In an especially preferred embodiment the poly-A sequence has a length of approximately 120 adenosine residues. To further increase stability and/or expression of the RNA used according to the teaching, the poly-A sequence can be unmasked.

In addition, incorporation of a 3'-non translated region (UTR) into the 3'-non translated region of an RNA molecule can result in an enhancement in translation efficiency. A synergistic effect may be achieved by incorporating two or more of such 3'-non translated regions. The 3'-non translated regions may be autologous or heterologous to the RNA into which they are introduced. In one particular embodiment the 3'-non translated region is derived from the human β-globin gene.

A combination of the above described modifications, i.e. incorporation of a poly-A sequence, unmasking of a poly-A sequence and incorporation of one or more 3'-non translated regions, has a synergistic influence on the stability of RNA and increase in translation efficiency.

The term "stability" of RNA relates to the "half-life" of RNA. "Half-life" relates to the period of time which is needed to eliminate half of the activity, amount, or number of molecules. In the context of the present invention, the half-life of an RNA is indicative for the stability of said RNA. The half-life of RNA may influence the "duration of expression" of the RNA. It can be expected that RNA having a long half-life will be expressed for an extended time period.

Of course, if according to the present invention it is desired to decrease stability and/or translation efficiency of RNA, it is possible to modify RNA so as to interfere with the function of elements as described above increasing the stability and/or translation efficiency of RNA.

The term "expression" is used in its most general meaning and comprises the production of RNA and/or peptides or proteins, e.g. by transcription and/or translation. With respect to RNA, the term "expression" or "translation" relates in particular to the production of peptides or proteins. It also comprises partial expression of nucleic acids. Moreover, expression can be transient or stable.

According to the teaching, terms such as "RNA expression", "expressing RNA", or "expression of RNA" relate to the production of peptide or protein encoded by the RNA. Preferably, such terms relate to the translation of RNA so as to express, i.e. produce peptide or protein encoded by the RNA.

In the context of the present teaching, the term "transcription" relates to a process, wherein the genetic code in a DNA sequence is transcribed into RNA. Subsequently, the RNA may be translated into protein. According to the present invention, the term "transcription" comprises *"in vitro* transcription", wherein the term *"in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are according to the present invention encompassed by the term "vector". According to the present teaching, the RNA used in the present teaching preferably is *in vitro* transcribed RNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription according to the invention is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The cDNA containing vector template may comprise vectors carrying different cDNA inserts which following transcription results in a population of different RNA molecules optionally capable of expressing different peptides or proteins or may comprise vectors carrying only one species of cDNA insert which following transcription only results in a population of one RNA species capable of expressing only one peptide or protein. Thus, it is possible to produce RNA capable of expressing a single peptide or protein only or to produce compositions of different RNAs such as RNA libraries and whole-cell RNA capable of expressing more than one peptide or protein, e.g. a composition of peptides or proteins. The present teaching envisions the introduction of all such RNA into cells.

The term "translation" as used herein relates to the process in the ribosomes of a cell by which a strand of messenger RNA directs the assembly of a sequence of amino acids to make a peptide or protein.

Expression control sequences or regulatory sequences, which according to the teaching may be linked functionally with a nucleic acid, can be homologous or heterologous with respect to the nucleic acid. A coding sequence and a regulatory sequence are linked together "functionally" if they are bound together covalently, so that the transcription or translation of the coding sequence is under the control or under the influence of the regulatory sequence. If the coding sequence is to be translated into a functional protein, with functional linkage of a regulatory sequence with the coding sequence, induction of the regulatory sequence leads to a transcription of the coding sequence, without causing a reading frame shift in the coding sequence or inability of the coding sequence to be translated into the desired protein or peptide.

The term "expression control sequence" or "regulatory sequence" comprises, according to the teaching, promoters, ribosome-binding sequences and other control elements, which control the transcription of a nucleic acid or the translation of the derived RNA. In certain embodiments of the invention, the regulatory sequences can be controlled. The precise structure of regulatory sequences can vary depending on the species or depending on the cell type, but generally comprises 5'-untranscribed and 5'- and 3'-untranslated sequences, which are involved in the initiation of transcription or translation, such as TATA-box, capping-sequence, CAAT-sequence and the like. In particular, 5'-untranscribed regulatory sequences comprise a promoter region that includes a promoter sequence for transcriptional control of the functionally bound gene. Regulatory sequences can also comprise enhancer sequences or upstream activator sequences.

Terms such as "enhancement of expression", "enhanced expression" or "increased expression" mean in the context of the present teaching that the amount of peptide or protein expressed by a given number of RNA molecules is higher than the amount of peptide or protein expressed by the same number of RNA molecules, wherein expression of the RNA molecules is performed under the same conditions except the condition which results in the enhanced or increased expression of the RNA, such as a reduction of the activity of RNA-dependent protein kinase (PKR) in a cell versus non-reduction of the activity of RNA-dependent protein kinase (PKR) in a cell. In this context, "same conditions" refer to a situation wherein the same RNA sequences encoding the same peptide or protein are introduced by the same means into the same cells, the cells are cultured under the same conditions (except the condition which results in the enhanced or increased expression) and the amount of peptide or protein is measured by the same means. The amount of peptide or protein may be given in moles, or by weight, e. g. in grams, or by mass or by polypeptide activity, e.g. if the peptide or protein is an enzyme it may be given as catalytic activity or if the peptide or protein is an antibody or antigen or a receptor it may be given as binding affinity. In one embodiment, terms such as "enhancement of expression", "enhanced expression" or "increased expression" mean in the context of the present invention that the amount of peptide or protein expressed by a given number of RNA molecules and within a given period of time is higher than the amount of peptide or protein expressed by the same number of RNA molecules and within the same period of time. For example, the maximum value of peptide or protein expressed by a given number of RNA molecules at a particular time point may be higher than the maximum value of peptide or protein expressed by the same number of RNA molecules. In other embodiments, the maximum value of peptide or protein expressed by a given number of RNA molecules does not need to be higher than the maximum value of peptide or protein expressed by the same number of RNA molecules. However, the average amount of peptide or protein expressed by the given number of RNA molecules within a given period of time may be higher than the average amount of peptide or protein expressed by the same number of RNA molecules. The latter cases are referred to herein as "higher level of expression" or "increased level of expression" and relate to higher maximum values of expression and/or higher average values of expression. Alternatively or additionally, terms such as "enhancement of expression", "enhanced expression" or "increased expression" mean in the context of the present teaching also that the time in which peptide or protein is expressed by RNA molecules may be longer than the time in which the peptide or protein is expressed by the same RNA molecules. Thus, in one embodiment, terms such as "enhancement of expression", "enhanced expression" or "increased expression" mean in the context of the present teaching also that the amount of peptide or protein expressed by a given number of RNA molecules is higher than the amount of peptide or protein expressed by the same number of RNA molecules since the period of time in which the RNA is stably present and expressed is longer than the period of time in which the same number of RNA molecules is stably present and expressed. These cases are referred to herein also as "increased duration of expression". Preferably, such longer time periods refer to expression for at least 48 h, preferably for at least 72 h, more preferably for at least 96 h, in particular for at least 120 h or even longer following introduction of RNA or following the first introduction (e.g. in case of repeated transfections) of RNA into a cell.

The level of expression and/or duration of expression of RNA may be determined by measuring the amount, such as the total amount expressed and/or the amount expressed in a given time period, and/or the time of expression of the peptide or protein encoded by the RNA, for example, by using an ELISA procedure, an immunohistochemistry procedure, a quantitative image analysis procedure, a Western Blot, mass spectrometry, a quantitative immunohistochemistry procedure, or an enzymatic assay.

In particular embodiments, the RNA according to the teaching comprises a population of different RNA molecules, e.g. a mixture of different RNA molecules optionally encoding different peptides and/or protein, whole-cell RNA, an RNA library, or a portion of thereof, e.g. a library of RNA molecules expressed in a particular cell type, such as undifferentiated cells, in particular stem cells such as embryonic stem cells, or a fraction of the library of RNA molecules such as RNA with enriched expression in undifferentiated cells, in particular stem cells such as embryonic stem cells relative to differentiated cells. Thus, according to the teaching, the term "RNA" may include a mixture of RNA molecules, whole-cell RNA or a fraction thereof, which may be obtained by a process comprising the isolation of RNA from cells and/or by recombinant means, in particular by *in vitro* transcription.

Preferably, according to the teaching, the RNA to be expressed in a cell is introduced into said cell, either *in vitro* or *in vivo,* preferably *in vitro.* RNA may be introduced into a cell either prior to, after and/or simultaneously with reducing the activity of RNA-dependent protein kinase (PKR) in the cell. Preferably, the activity of RNA-dependent protein kinase (PKR) in the cell is reduced as long as expression of the RNA in the cell is desired. In one embodiment of the methods according to the invention, the RNA that is to be introduced into a cell is obtained by *in vitro* transcription of an appropriate DNA template.

The RNA used according to the present teaching may have a known composition (in this embodiment it is preferably known which peptides or proteins are being expressed by the RNA) or the composition of the RNA may be partially or entirely unknown. Alternatively, the RNA may have a known function or the function of the RNA may be partially or entirely unknown.

According to the teaching, the terms "RNA capable of expressing" and "RNA encoding" are used interchangeably herein and with respect to a particular peptide or protein mean that the RNA, if present in the appropriate environment, preferably within a cell, can be expressed to produce said peptide or protein. Preferably, RNA according to the teaching is able to interact with the cellular translation machinery to provide the peptide or protein it is capable of expressing.

According to the teaching, RNA may be introduced into cells either *in vitro* or *in vivo,* preferably *in vitro.* The cells into which the RNA has been introduced *in vitro* may, preferably following expression of the RNA *in vitro* by the methods of the teaching, be administered to a patient.

Terms such as "transferring", "introducing" or "transfecting" are used interchangeably herein and relate to the introduction of nucleic acids, in particular exogenous or heterologous nucleic acids, in particular RNA into a cell. According to the present teaching, the cell can be an isolated cell or it can form part of an organ, a tissue and/or an organism. Any technique which is suitable to introduce RNA into cells may be used. Preferably, the RNA is introduced into cells by standard techniques. Such techniques comprise transfection of nucleic acid calcium phosphate precipitates, transfection of nucleic acids which are associated with DEAE, the transfection or infection with viruses which carry the nucleic acids of interest, electroporation, lipofection, and microinjection. According to the present teaching, the administration of a nucleic acid is either achieved as naked nucleic acid or in combination with an administration reagent. Preferably, administration of nucleic acids is in the form of naked nucleic acids. Preferably, the RNA is administered in combination with stabilizing substances such as RNase inhibitors. The present teaching also envisions the repeated introduction of nucleic acids into cells to allow sustained expression for extended time periods.

Cells can be transfected, for example, using commercially available liposome-based transfection kits such as Lipofectamine™ (Invitrogen) and can be transfected with any carriers with which RNA can be associated, e.g. by forming complexes with the RNA or forming vesicles in which the RNA is enclosed or encapsulated, resulting in increased stability of the RNA compared to naked RNA. Carriers useful according to the teaching include, for example, lipid-containing carriers such as cationic lipids, liposomes, in particular cationic liposomes, and micelles. Cationic lipids may form complexes with negatively charged nucleic acids. Any cationic lipid may be used according to the teaching.

Preferably, the introduction of RNA which encodes a peptide or protein into a cell results in expression of said peptide or protein in the cell. In particular embodiments, the targeting of the nucleic acids to particular cells is preferred. In such embodiments, a carrier which is applied for the administration of the nucleic acid to a cell (for example, a retrovirus or a liposome), exhibits a targeting molecule. For example, a molecule such as an antibody which is specific for a surface membrane protein on the target cell or a ligand for a receptor on the target cell may be incorporated into the nucleic acid carrier or may be bound thereto. In case the nucleic acid is administered by liposomes, proteins which bind to a surface membrane protein which is associated with endocytosis may be incorporated into the liposome formulation in order to enable targeting and/or uptake. Such proteins encompass capsid proteins of fragments thereof which are specific for a particular cell type, antibodies against proteins which are internalized, proteins which target an intracellular location etc.

Electroporation or electropermeabilization relates to a significant increase in the electrical conductivity and permeability of the cell plasma membrane caused by an externally applied electrical field. It is usually used in molecular biology as a way of introducing some substance into a cell. Electroporation is usually done with electroporators, appliances which create an electro-magnetic field in the cell solution. The cell suspension is pipetted into a glass or plastic cuvette which has two aluminium electrodes on its sides. For electroporation, a cell suspension of around 50 microliters is typically used. Prior to electroporation it is mixed with the nucleic acid to be transfected. The mixture is pipetted into the cuvette, the voltage and capacitance is set and the cuvette inserted into the electroporator. Preferably, liquid medium is added immediately after electroporation (in the cuvette or in an Eppendorf tube), and the tube is incubated at the cells' optimal temperature for an hour or more to allow recovery of the cells and optionally expression of antibiotic resistance.

According to the teaching it is preferred that a nucleic acid such as RNA encoding a peptide or protein once taken up by or introduced into a cell which cell may be present *in vitro* or in a subject results in expression of said peptide or protein. The cell may express the encoded peptide or protein intracellularly, may secrete the encoded peptide or protein, or may express it on the surface.

If according to the teaching RNA capable of expressing certain factors for reprogramming of somatic cells is introduced into somatic cells, it is preferred that this introduction of RNA results in expression of said factors for a time period to complete the reprogramming process and in the development of cells having stem cell characteristics. Preferably, introduction of RNA capable of expression certain factors as disclosed herein into somatic cells results in expression of said factors for an extended period of time, preferably for at least 10 days, preferably for at least 11 days and more preferably for at least 12 days. To achieve such long term expression, RNA is preferably periodically introduced into the cells more than one time, preferably using electroporation. Preferably, RNA is introduced into the cells at least twice, more preferably at least 3 times, more preferably at least 4 times, even more preferably at least 5 times up to preferably 6 times, more preferably up to 7 times or even up to 8, 9 or 10 times, preferably over a time period of at least 10 days, preferably for at least 11 days and more preferably for at least 12 days to ensure expression of one or more factors for an extended period of time. Preferably, the time periods elapsing between the repeated introductions of the RNA are from 24 hours to 120 hours, preferably 48 hours to 96 hours. In one embodiment, time periods elapsing between the repeated introductions of the RNA are not longer than 72 hours, preferably not longer than 48 hours or 36 hours. In one embodiment, prior to the next electroporation, cells are allowed to recover from the previous electroporation. In this embodiment, the time periods elapsing between the repeated introductions of the RNA are at least 72 hours, preferably at least 96 hours, more preferably at least 120 hours. In any case, the conditions should be selected so that the factors are expressed in the cells in amounts and for periods of time which support the reprogramming process.

Preferably at least 1 µg, preferably at least 1.25 µg, more preferably at least 1.5 µg and preferably up to 20 µg, more preferably up to 15 µg, more preferably up to 10 µg, more preferably up to 5 µg, preferably 1 to 10 µg, even more preferably 1 to 5 µg, or 1 to 2.5 µg of RNA for each peptide, protein or factor is used per electroporation.

Preferably, if a loss of viability of cells occurs by repeated electroporations, previously not electroporated cells are added as carrier cells. Preferably, previously not electroporated cells are added prior to, during or after one or more of the 4^{th} and subsequent, preferably, the 5^{th} and subsequent electroporations such as prior to, during or after the 4^{th} and 6^{th} electroporation. Preferably, previously not electroporated cells are added prior to, during or after the 4^{th} or 5^{th} and each subsequent electroporation. Preferably, the previously not electroporated cells are the same cells as those into which RNA is introduced.

The RNA-binding protein RNA-dependent protein kinase (Protein kinase RNA-activated; PKR) binds RNAs in a length-dependent fashion. PKR is an interferon-induced serine/threonine protein kinase initially identified in viral response by virtue of its binding to and activation by the extensive secondary structure formed by viral RNA sequences. Human PKR is 68 kDa with an about 20 kDa N-terminal dsRNA-binding domain and a C-terminal protein kinase domain. *In vitro* PKR is activated by binding to RNA molecules with extensive duplex secondary structure. *In vivo* the enzyme is believed to be activated by viral double-stranded RNA (dsRNA) or viral replicative intermediates comprising dsRNA. Binding to double-stranded RNA to PKR causes a conformational change in the enzyme that alters the ATP-binding site in the kinase domain and leads to autophosphorylation at multiple serine and threonine residues throughout the PKR sequence. RNA-stimulated autophosphorylation increases cellular sensitivity to apoptotic and pro-inflammatory stimuli through a number of putative pathways, including phosphorylation of its known substrate eukaryotic initiation factor 2 (eIF2a).

The term "PKR" preferably relates to human PKR, and, in particular, to a protein comprising the amino acid sequence according to SEQ ID NO: 13 of the sequence listing or a variant of said amino acid sequence. In one embodiment, the term "PKR" relates to a protein comprising an amino acid sequence encoded by the nucleic acid sequence according to SEQ ID NO: 32. The term "PKR" includes any variants, in particular mutants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. A species homolog is a nucleic acid or amino acid sequence with a different species of origin from that of a given nucleic acid or amino acid sequence. One skilled in the art would understand that the cDNA sequence of PKR as described above would be equivalent to PKR mRNA, and can be used for the generation of inhibitory nucleic acids against PKR.

Protein kinase activity including protein kinase autophosphorylation can be measured by a variety of techniques known to the skilled person. One method involves separation of unreacted ATP from the phosphorylated kinase substrate by e.g. precipitating phosphoprotein onto cellulose strips by trichloroacetic acid followed by washing, or adsorption of phosphoprotein onto phosphocellulose strips. For example, dephosphoPKR can be activated by incubation with poly[I:C] and autophosphorylation can be allowed to proceed in the presence of [γ-32P]ATP. The ability of compounds to block this RNA-induced PKR autophosphorylation can be tested. Another method involves detection and quantification of phospho-PKR in relation to the total amount of PKR in the same lysate of cells by Western blotting with antibodies specific for phospho-PKR or full length PKR. Another method involves detection and quantification of the phosphorylated substrate of PKR, e.g. phospho-eIF2α in relation to the total amount of eIF2α in the same lysate of cells by Western blotting with antibodies specific for phospho-eIF2α or full length eIF2α.

Viral defense mechanisms against PKR function e.g. through decoy dsRNA (adenovirus VAI RNA; Epstein-Barr virus EBER; HIV TAR), PKR degradation (poliovirus 2A^{pro}), hiding viral dsRNA (vaccinia virus E3L; reovirus sigma3; influenza virus NS1), blocking dimerization (influenza virus p58^{IPK}; Hepatitis C virus NS5A), pseudosubstrates (vaccinia virus K3L; HIV Tat) or dephosphorylation of substrate (herpes simplex virus ICP34.5).

A decoy RNA is pseudosubstrate RNA that has similar structure to the RNA substrate of an enzyme, in order to make the enzyme bind to the pseudosubstrate rather than to the real substrate, thus blocking the activity of the enzyme.

According to the present teaching, the term "reducing the activity of RNA-dependent protein kinase (PKR)" relates to measures that result in a lower degree of homodimerization of PKR, in a lower degree of autophosphorylation of PKR and/or in a lower degree of phosphorylation of targets which are kinase substrates of PKR such as eIF2a compared to the normal situation, in particular the normal situation in a cell, wherein the activity of PKR is not reduced/has not been reduced by man. Preferably, said term includes all measures that result in a lower degree of autophosphorylation of PKR and/or in a lower degree of phosphorylation of targets which are kinase substrates of PKR.

According to the teaching, it is envisioned to reduce the activity of PKR in a cell *in vitro* or *in vivo*, preferably *in vitro.* Thus, according to the present invention, the cell can be an isolated cell or it can form part of an organ, a tissue and/or an organism.

According to the teaching, all measures and means that result in a reduction of the activity of PKR are suitable for reducing the activity of PKR. Reducing the activity of PKR in a cell preferably results in an enhancement of stability and/or an enhancement of expression of the RNA in the cell compared to the normal situation, in particular the normal situation in a cell, wherein the activity of PKR is not reduced/has not been reduced by man. Enhancement of expression of RNA in a cell preferably comprises an increase in the level of expression and/or an increase in the duration of expression of the RNA in the cell compared to the normal situation, in particular the normal situation in a cell, wherein the activity of PKR is not reduced/has not been reduced by man.

In one embodiment, reducing the activity of RNA-dependent protein kinase (PKR) in a cell comprises treating the cell with an inhibitor of expression and/or activity of PKR. According to the teaching, the phrase "inhibit expression and/or activity" includes a complete or essentially complete inhibition of expression and/or activity and a reduction in expression and/or activity. Preferably, the treatment of the cell with a PKR inhibitor is for a time sufficient to result in an enhancement of stability and/or an enhancement of expression of the RNA in the cell.

Preferably, said inhibition of expression of PKR may take place by inhibiting the production of or reducing the level of transcript, i.e. mRNA, coding for PKR, e.g. by inhibiting transcription or inducing degradation of transcript, and/or by inhibiting the production of PKR, e.g. by inhibiting translation of transcript coding for PKR. In one embodiment, said PKR inhibitor is specific for a nucleic acid encoding PKR. In a particular embodiment, the inhibitor of expression of PKR is an inhibitory nucleic acid (e.g., antisense molecule, ribozyme, iRNA, siRNA or a DNA encoding the same) selectively hybridizing to and being specific for PKR, thereby inhibiting (e.g., reducing) transcription and/or translation thereof.

Inhibitory nucleic acids of this invention include oligonucleotides having sequences in the antisense orientation relative to the target nucleic acids. Suitable inhibitory oligonucleotides typically vary in length from five to several hundred nucleotides, more typically about 20-70 nucleotides in length or shorter, even more typically about 10-30 nucleotides in length. These inhibitory oligonucleotides may be applied, either *in vitro* or *in vivo,* as free (naked) nucleic acids or in protected forms, e.g., encapsulated in liposomes. The use of liposomal or other protected forms may be advantageous as it may enhance *in vivo* stability and thus facilitate delivery to target sites.

Also, the target nucleic acid may be used to design ribozymes that target the cleavage of the corresponding mRNAs in cells. Similarly, these ribozymes may be administered in free (naked) form or by the use of delivery systems that enhance stability and/or targeting, e.g., liposomes.

Also, the target nucleic acid may be used to design siRNAs that can inhibit (e.g., reduce) expression of the nucleic acid. The siRNAs may be administered in free (naked) form or by the use of delivery systems that enhance stability and/or targeting, e.g., liposomes. They may also be administered in the form of their precursors or encoding DNAs.

siRNA preferably comprises a sense RNA strand and an antisense RNA strand, wherein the sense and antisense RNA strands form an RNA duplex, and wherein the sense RNA strand comprises a nucleotide sequence substantially identical to a target sequence of about 19 to about 25 contiguous nucleotides in a target nucleic acid, preferably mRNA coding for PKR.

In one embodiment, said PKR inhibitor is directed at the PKR protein and preferably is specific for PKR. PKR can be inhibited in various ways, e.g. through inhibiting PKR autophosphorylation and/or dimerization, providing a PKR pseudo-activator, or providing a PKR pseudo-substrate. The PKR inhibitor may be an agent which is involved in a viral defense mechanism as discussed above. For example, vaccinia virus E3L encodes a dsRNA binding protein that inhibits PKR in virus-infected cells, presumably by sequestering dsRNA activators. K3, also encoded by vaccinia virus, functions as a pseudosubstrate inhibitor by binding to PKR. Thus, providing vaccinia virus E3L may result in inhibition of PKR. Providing adenovirus VAI RNA, HIV Tat or Epstein-Barr virus EBER1 RNA may result in PKR pseudo-activation. Thus, for example, all viral factors, i.e. virally derived inhibitors, blocking PKR activity such as those described herein may be used for reducing the activity of PKR. Such factors may be provided to a cell either in the form of nucleic acid such as RNA or peptide/protein, as appropriate.

In one embodiment, the PKR inhibitor is a chemical inhibitor. Preferably, the PKR inhibitor is an inhibitor of RNA-induced PKR autophosphorylation. Preferably, the PKR inhibitor is an ATP-binding site directed inhibitor of PKR.

In one embodiment, the PKR inhibitor is 6,8-dihydro-8-(1H-imidazol-5-ylmethylene)-7H-pyrrolo[2,3-g]benzothiazol-7-one. In one embodiment, the PKR inhibitor has the following formula:

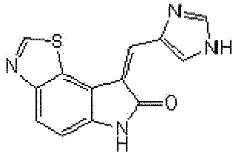

In one embodiment, the PKR inhibitor is 2-aminopurine. In one embodiment, the PKR inhibitor has the following formula:

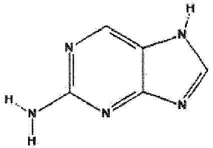

Preferably, an inhibitor as disclosed above is used in a concentration of at least 0.5 µM or higher, at least 1 µM or higher or at least 2 µM or higher and preferably in a concentration up to 5 µM, up to 4 µM, up to 3 µM or up to 2 µM.

In a further embodiment, the inhibitor of activity of PKR is an antibody that specifically binds to PKR. Binding of the antibody to PKR can interfere with the function of PKR, e.g. by inhibiting binding activity or catalytic activity.

In one embodiment, it is envisioned to reduce the activity of PKR in a cell by treating the cell with one or more virally derived inhibitors such as vaccinia virus E3 and/or K3 as well as treating the cell with one or more chemical PKR inhibitors such as 6,8-dihydro-8-(1H-imidazol-5-ylmethylene)-7H-pyrrolo[2,3-g]benzothiazol-7-one and/or 2-aminopurine.

In one embodiment, cells are treated to reduce the activity of PKR prior to, simultaneously with and/or following introduction of RNA or the first introduction (e.g. in case of repeated transfections) of RNA. In one embodiment, cells are treated to reduce the activity of PKR following, preferably immediately following introduction of RNA or the first introduction (e.g. in case of repeated transfections) of RNA.

In one embodiment, cells are treated to reduce the activity of PKR for at least 24 h, at least 48 h, at least 72 h, at least 96 h, at least 120 h or even longer. Most preferably, cells are treated to reduce the activity of PKR for the entire period of time in which expression of RNA is desired, such as permanently, optionally with repeated transfection of RNA.

"Antisense molecules" or "antisense nucleic acids" may be used for regulating, in particular reducing, expression of a nucleic acid. The term "antisense molecule" or "antisense nucleic acid" refers according to the invention to an oligonucleotide which is an oligoribonucleotide, oligodeoxyribonucleotide, modified oligoribonucleotide or modified oligodeoxyribonucleotide and which hybridizes under physiological conditions to DNA comprising a particular gene or to mRNA of said gene, thereby inhibiting transcription of said gene and/or translation of said mRNA. According to the invention, an "antisense molecule" also comprises a construct which contains a nucleic acid or a part thereof in reverse orientation with respect to its natural promoter. An antisense transcript of a nucleic acid or of a part thereof may form a duplex with naturally occurring mRNA and thus prevent accumulation of or translation of the mRNA. Another possibility is the use of ribozymes for inactivating a nucleic acid.

In preferred embodiments, the antisense oligonucleotide hybridizes with an N-terminal or 5' upstream site such as a translation initiation site, transcription initiation site or promoter site. In further embodiments, the antisense oligonucleotide hybridizes with a 3'-untranslated region or mRNA splicing site.

By "small interfering RNA" or "siRNA" as used herein is meant an RNA molecule, preferably greater than 10 nucleotides in length, more preferably greater than 15 nucleotides in length, and most preferably 18, 19, 20, 21, 22, 23, 24, 25, 26, 27, 28, 29, or 30 nucleotides in length that is used to identify a target gene or mRNA to be degraded. A range of 19-25 nucleotides is the most preferred size for siRNAs.

One or both strands of the siRNA can also comprise a 3'-overhang. As used herein, a "3'-overhang" refers to at least one unpaired nucleotide extending from the 3'-end of an RNA strand. Thus in one embodiment, the siRNA comprises at least one 3'-overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length. In the embodiment in which both strands of the siRNA molecule comprise a 3'-overhang, the length of the overhangs can be the same or different for each strand. In a most preferred embodiment, the 3'-overhang is present on both strands of the siRNA, and is 2 nucleotides in length. For example, each strand of the siRNA of the invention can comprise 3'-overhangs of dideoxythymidylic acid ("TT") or diuridylic acid ("uu").

In order to enhance the stability of the siRNA, the 3'-overhangs can be also stabilized against degradation. In one embodiment, the overhangs are stabilized by including purine nucleotides, such as adenosine or guanosine nucleotides. Alternatively, substitution of pyrimidine nucleotides by modified analogues, e.g., substitution of uridine nucleotides in the 3'-overhangs with 2'-deoxythymidine, is tolerated and does not affect the efficiency of RNAi degradation. In particular, the absence of a 2'-hydroxyl in the 2'-deoxythymidine significantly enhances the nuclease resistance of the 3'-overhang in tissue culture medium.

The sense and antisense strands of the siRNA can comprise two complementary, single-stranded RNA molecules or can comprise a single molecule in which two complementary portions are base-paired and are covalently linked by a single-stranded "hairpin" area. That is, the sense region and antisense region can be covalently connected via a linker molecule. The linker molecule can be a polynucleotide or non-nucleotide linker. Without wishing to be bound by any theory, it is believed that the hairpin area of the latter type of siRNA molecule is cleaved intracellularly by the "Dicer" protein (or its equivalent) to form a siRNA of two individual base-paired RNA molecules.

As used herein, "target mRNA" refers to an RNA molecule that is a target for downregulation.

siRNA can be expressed from pol III expression vectors without a change in targeting site, as expression of RNAs from pol III promoters is only believed to be efficient when the first transcribed nucleotide is a purine.

siRNA according to the invention can be targeted to any stretch of approximately 19-25 contiguous nucleotides in any of the target mRNA sequences (the "target sequence"). Techniques for selecting target sequences for siRNA are given, for example, in Tuschl T. et al., "The siRNA User Guide", revised Oct. 11, 2002, the entire disclosure of which is herein incorporated by reference. "The siRNA User Guide" is available on the world wide web at a website maintained by Dr. Thomas Tuschl, Laboratory of RNA Molecular Biology, Rockefeller University, New York, USA, and can be found by accessing the website of the Rockefeller University and searching with the keyword "siRNA". Thus, the sense strand of the present siRNA comprises a nucleotide sequence substantially identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (i.e., in the 3'-direction) from the start codon. The target sequence can, however, be located in the 5'- or 3'-untranslated regions, or in the region nearby the start codon.

siRNA can be obtained using a number of techniques known to those of skill in the art. For example, siRNA can be chemically synthesized or recombinantly produced using methods known in the art, such as the Drosophila *in vitro* system described in U.S. published application 2002/0086356 of Tuschl et al., the entire disclosure of which is herein incorporated by reference.

Preferably, siRNA is chemically synthesized using appropriately protected ribonucleoside phosphoramidites and a conventional DNA/RNA synthesizer. siRNA can be synthesized as two separate, complementary RNA molecules, or as a single RNA molecule with two complementary regions.

Alternatively, siRNA can also be expressed from recombinant circular or linear DNA plasmids using any suitable promoter. Suitable promoters for expressing siRNA of the invention from a plasmid include, for example, the U6 or H1 RNA pol III promoter sequences and the cytomegalovirus promoter.

Selection of other suitable promoters is within the skill in the art. The recombinant plasmids can also comprise inducible or regulatable promoters for expression of the siRNA in a particular tissue or in a particular intracellular environment.

The siRNA expressed from recombinant plasmids can either be isolated from cultured cell expression systems by standard techniques, or can be expressed intracellularly. The use of recombinant plasmids to deliver siRNA to cells *in vivo* is within the skill in the art. siRNA can be expressed from a recombinant plasmid either as two separate, complementary RNA molecules, or as a single RNA molecule with two complementary regions.

Selection of plasmids suitable for expressing siRNA, methods for inserting nucleic acid sequences for expressing the siRNA into the plasmid, and methods of delivering the recombinant plasmid to the cells of interest are within the skill in the art.

The term "cell" or "host cell" preferably relates to an intact cell, i.e. a cell with an intact membrane that has not released its normal intracellular components such as enzymes, organelles, or genetic material. An intact cell preferably is a viable cell, i.e. a living cell capable of carrying out its normal metabolic functions. Preferably said term encompasses any cell which can be transformed or transfected with an exogenous nucleic acid. The term "cell" includes according to the invention prokaryotic cells (e.g., E. coli) or eukaryotic cells. Mammalian cells are particularly preferred, such as cells from humans, mice, hamsters, pigs, goats, and primates. The cell is preferably a cell in which a reduction of the activity of PKR results in an enhancement of stability and/or an enhancement of expression of RNA in the cell. In one embodiment, the cell is a somatic cell as described herein. In one embodiment, the cell is a cell having a barrier function. Preferably, the cell is a fibroblast such as a fibroblast described herein, a keratinocyte, an epithelial cell, or an endothelial cell such as an endothelial cell of the heart, an endothelial cell of the lung, or an umbilical vein endothelial cell. Preferably, the cell is a human cell.

A fibroblast is a type of cell that synthesizes the extracellular matrix and collagen and plays a critical role in wound healing. The main function of fibroblasts is to maintain the structural integrity of connective tissues by continuously secreting precursors of the extracellular matrix. Fibroblasts are the most common cells of connective tissue in animals. Fibroblasts are morphologically heterogeneous with diverse appearances depending on their location and activity.

Keratinocytes are the predominant cell type in the epidermis, the outermost layer of the human skin. The primary function of keratinocytes is the formation of the keratin layer that protects the skin and the underlying tissue from environmental damage such as heat, UV radiation and water loss.

Epithelium is a tissue composed of cells that line the cavities and surfaces of structures throughout the body. Many glands are also formed from epithelial tissue. It lies on top of connective tissue, and the two layers are separated by a basement membrane. In humans, epithelium is classified as a primary body tissue, the other ones being connective tissue, muscle tissue and nervous tissue. Functions of epithelial cells include secretion, selective absorption, protection, transcellular transport and detection of sensation.

The endothelium is the thin layer of cells that lines the interior surface of blood vessels, forming an interface between circulating blood in the lumen and the rest of the vessel wall. These cells are called endothelial cells. Endothelial cells line the entire circulatory system, from the heart to the smallest capillary. Endothelial tissue is a specialized type of epithelium tissue.

According to the invention, RNA encodes/is capable of expressing a peptide or protein, such as a reprogramming factor also referred to as "factor" herein, the expression of which in a cell is desired.

According to the present teaching, the term "peptide" comprises oligo- and polypeptides and refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" refers to large peptides, preferably to peptides with more than 100 amino acid residues, but in general the terms "peptides" and "proteins" are synonyms and are used interchangeably herein.

The present teaching also includes "variants" of the peptides, proteins, or amino acid sequences described herein.

For the purposes of the present teaching, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids or nucleotides that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the invention at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

The invention includes derivatives of the peptides or proteins described herein which are comprised by the terms "peptide" and "protein". According to the invention, "derivatives" of proteins and peptides are modified forms of proteins and peptides. Such modifications include any chemical modification and comprise single or multiple substitutions, deletions and/or additions of any molecules associated with the protein or peptide, such as carbohydrates, lipids and/or proteins or peptides. In one embodiment, "derivatives" of proteins or peptides include those modified analogs resulting from glycosylation, acetylation, phosphorylation, amidation, palmitoylation, myristoylation, isoprenylation, lipidation, alkylation, derivatization, introduction of protective/blocking groups, proteolytic cleavage or binding to an antibody or to another cellular ligand. The term "derivative" also extends to all functional chemical equivalents of said proteins and peptides. Preferably, a modified peptide has increased stability and/or increased immunogenicity.

According to the teaching, a variant of a peptide or protein preferably has a functional property of the peptide or protein from which it has been derived. Such functional properties are described herein for OCT4, SOX2, NANOG, LIN28, KLF4 and c-MYC, respectively. Preferably, a variant of a peptide or protein has the same property in reprogramming an animal differentiated cell as the peptide or protein from which it has been derived. Preferably, the variant induces or enhances reprogramming of an animal differentiated cell.

In one embodiment, the peptide or protein encoded by the RNA is a factor allowing the reprogramming of somatic cells to cells having stem cell characteristics. In one embodiment, the peptide or protein comprises one or more antigens and/or one or more antigen peptides. Preferably, said RNA is capable of expressing said peptide or protein, in particular if introduced into a cell.

A "stem cell" is a cell with the ability to self-renew, to remain undifferentiated, and to become differentiated. A stem cell can divide without limit, for at least the lifetime of the animal in which it naturally resides. A stem cell is not terminally differentiated; it is not at the end stage of a differentiation pathway. When a stem cell divides, each daughter cell can either remain a stem cell or embark on a course that leads toward terminal differentiation.

Totipotent stem cells are cells having totipotential differentiation properties and being capable of developing into a complete organism. This property is possessed by cells up to the 8-cell stage after fertilization of the oocyte by the sperm. When these cells are isolated and transplanted into the uterus, they can develop into a complete organism.

Pluripotent stem cells are cells capable of developing into various cells and tissues derived from the ectodermal, mesodermal and endodermal layers. Pluripotent stem cells which are derived from the inner cell mass located inside of blastocysts, generated 4-5 days after fertilization are called "embryonic stem cells" and can differentiate into various other tissue cells but cannot form new living organisms.

Multipotent stem cells are stem cells differentiating normally into only cell types specific to their tissue and organ of origin. Multipotent stem cells are involved not only in the growth and development of various tissues and organs during the fetal, neonatal and adult periods but also in the maintenance of adult tissue homeostasis and the function of inducing regeneration upon tissue damage. Tissue-specific multipotent cells are collectively called "adult stem cells".

An "embryonic stem cell" is a stem cell that is present in or isolated from an embryo. It can be pluripotent, having the capacity to differentiate into each and every cell present in the organism, or multipotent, with the ability to differentiate into more than one cell type.

As used herein, "embryo" refers to an animal in the early stages of it development. These stages are characterized by implantation and gastrulation, where the three germ layers are defined and established and by differentiation of the germs layers into the respective organs and organ systems. The three germ layers are the endoderm, ectoderm and mesoderm.

A "blastocyst" is an embryo at an early stage of development in which the fertilized ovum has undergone cleavage, and a spherical layer of cells surrounding a fluid-filled cavity is forming, or has formed. This spherical layer of cells is the trophectoderm. Inside the trophectoderm is a cluster of cells termed the inner cell mass (ICM). The trophectoderm is the precursor of the placenta, and the ICM is the precursor of the embryo.

An adult stem cell, also called a somatic stem cell, is a stem cell found in an adult. An adult stem cell is found in a differentiated tissue, can renew itself, and can differentiate, with some limitations, to yield specialized cell types of its tissue of origin. Examples include mesenchymal stem cells, hematopoietic stem cells, and neural stem cells.

A "differentiated cell" is a mature cell that has undergone progressive developmental changes to a more specialized form or function. Cell differentiation is the process a cell undergoes as it matures to an overtly specialized cell type. Differentiated cells have distinct characteristics, perform specific functions, and are less likely to divide than their less differentiated counterparts.

An "undifferentiated" cell, for example, an immature, embryonic, or primitive cell, typically has a nonspecific appearance, may perform multiple, non-specific activities, and may perform poorly, if at all, in functions typically performed by differentiated cells.

"Somatic cell" refers to any and all differentiated cells and does not include stem cells, germ cells, or gametes. Preferably, "somatic cell" as used herein refers to a terminally differentiated cell.

As used herein, "committed" refers to cells which are considered to be permanently committed to a specific function. Committed cells are also referred to as "terminally differentiated cells".

As used herein, "differentiation" refers to the adaptation of cells for a particular form or function. In cells, differentiation leads to a more committed cell.

As used herein, "de-differentiation" refers to loss of specialization in form or function. In cells, de-differentiation leads to a less committed cell.

As used herein "reprogramming" refers to the resetting of the genetic program of a cell. A reprogrammed cell preferably exhibits pluripotency.

The terms "de-differentiated" and "reprogrammed" or similar terms are used interchangeably herein to denote somatic cell-derived cells having stem cell characteristics. However, said terms are not intended to limit the subject-matter disclosed herein by mechanistic or functional considerations.

The term "RNA inducing the development of stem cell characteristics" or "RNA capable of expressing one or more factors allowing the reprogramming of the somatic cells to cells having stem cell characteristics" refers to RNA which when introduced into a somatic cell induces the cell to de-differentiate.

As used herein, "germ cell" refers to a reproductive cell such as a spermatocyte or an oocyte, or a cell that will develop into a reproductive cell.

As used herein, "pluripotent" refers to cells that can give rise to any cell type except the cells of the placenta or other supporting cells of the uterus.

Terms such as "cell having stem cell characteristics", "cell having stem cell properties" or "stem like cell" are used herein to designate cells which, although they are derived from differentiated somatic non-stem cells, exhibit one or more features typical for stem cells, in particular embryonic stem cells. Such features include an embryonic stem cell morphology such as compact colonies, high nucleus to cytoplasm ratio and prominent nucleoli, normal karyotypes, expression of telomerase activity, expression of cell surface markers that are characteristic for embryonic stem cells, and/or expression of genes that are characteristic for embryonic stem cells. The cell surface markers that are characteristic for embryonic stem cells are, for example, selected from the group consisting of stage-specific embryonic antigen-3 (SSEA-3), SSEA-4, tumor-related antigen-1-60 (TRA-1-60), TRA-1-81, and TRA-2-49/6E. The genes that are characteristic for embryonic stem cells are selected, for example, from the group consisting of endogenous OCT4, endogenous NANOG, growth and differentiation factor 3 (GDF3), reduced expression 1 (REX1), fibroblast growth factor 4 (FGF4), embryonic cell-specific gene 1 (ESG1), developmental pluripotency-associated 2 (DPPA2), DPPA4, and telomerase reverse transcriptase (TERT). In one embodiment, the one or more features typical for stem cells include pluripotency.

In one embodiment of the method of the teaching, the stem cell characteristics comprise an embryonic stem cell morphology, wherein said embryonic stem cell morphology preferably comprises morphological criteria selected from the group consisting of compact colonies, high nucleus to cytoplasm ratio and prominent nucleoli. In certain embodiments, the cells having stem cell characteristics have normal karyotypes, express telomerase activity, express cell surface markers that are characteristic for embryonic stem cells and/or express genes that are characteristic for embryonic stem cells. The cell surface markers that are characteristic for embryonic stem cells may be selected from the group consisting of stage-specific embryonic antigen-3 (SSEA-3), SSEA-4, tumor-related antigen-1-60 (TRA-1-60), TRA-1-81, and TRA-2-49/6E and the genes that are characteristic for embryonic stem cells may be selected from the group consisting of endogenous OCT4, endogenous NANOG, growth and differentiation factor 3 (GDF3), reduced expression 1 (REX1), fibroblast growth factor 4 (FGF4), embryonic cell-specific gene 1 (ESG1), developmental pluripotency-associated 2 (DPPA2), DPPA4, and telomerase reverse transcriptase (TERT).

Preferably, the cells having stem cell characteristics are de-differentiated and/or reprogrammed somatic cells. Preferably, the cells having stem cell characteristics exhibit the essential characteristics of embryonic stem cells such as a pluripotent state. Preferably, the cells having stem cell characteristics have the developmental potential to differentiate into advanced derivatives of all three primary germ layers. In one embodiment, the primary germ layer is endoderm and the advanced derivative is gut-like epithelial tissue. In a further embodiment, the primary germ layer is mesoderm and the advanced derivative is striated muscle and/or cartilage. In an even further embodiment, the primary germ layer is ectoderm and the advanced derivative is neural tissue and/or epidermal tissue. In one preferred embodiment, the cells having stem cell characteristics have the developmental potential to differentiate into neuronal cells and/or cardiac cells.

In one embodiment, the somatic cells are embryonic stem cell derived somatic cells with a mesenchymal phenotype. In a preferred embodiment, the somatic cells are fibroblasts such as fetal fibroblasts or postnatal fibroblasts or keratinocytes, preferably hair follicle derived keratinocytes. In further embodiments, the fibroblasts are lung fibroblasts, foreskin fibroblasts or dermal fibroblasts. In particular embodiments, the fibroblasts are fibroblasts as deposited at the American Type Culture Collection (ATCC) under catalogue no. CCL-186, as deposited at the American Type Culture Collection (ATCC) under catalogue no. CRL-2097 or as deposited at the American Type Culture Collection (ATCC) under catalogue no. CRL-2522, or as distributed by System Biosciences under the catalogue no. PC501A-HFF. In one embodiment, the fibroblasts are adult human dermal fibroblasts. Preferably, the somatic cells are human cells. According to the present teaching, the somatic cells may be genetically modified.

The term "factor" according to the invention when used in conjunction with the expression thereof by RNA includes proteins and peptides as well as derivatives and variants thereof. For example, the term "factor" comprises OCT4, SOX2, NANOG, LIN28, KLF4 and c-MYC.

The factors can be of any animal species; e.g., mammals and rodents. Examples of mammals include but are not limited to human and non-human primates. Primates include but are not limited to humans, chimpanzees, baboons, cynomolgus monkeys, and any other New or Old World monkeys. Rodents include but are not limited to mouse, rat, guinea pig, hamster and gerbil.

According to the present teaching, one or more factors capable of allowing the reprogramming of somatic cells to cells having stem cell characteristics comprise an assembly of factors selected from the group consisting of (i) OCT4 and SOX2, (ii) OCT4, SOX2, and one or both of NANOG and LIN28, (iii) OCT4, SOX2 and one or both of KLF4 and c-MYC. In one embodiment, said one or more factors capable of being expressed by the RNA comprise OCT4, SOX2, NANOG and LIN28 or OCT4, SOX2, KLF4 and c-MYC. Preferably, the RNA is introduced into said animal differentiated somatic cell by electroporation or microinjection. Preferably, the method of the teaching further comprises allowing the development of cells having stem cell characteristics, e.g. by culturing the somatic cell under embryonic stem cell culture conditions, preferably conditions suitable for maintaining pluripotent stem cells in an undifferentiated state.

OCT4 is a transcription factor of the eukaryotic POU transcription factors and an indicator of pluripotency of embryonic stem cells. It is a maternally expressed Octomer binding protein. It has been observed to be present in oocytes, the inner cell mass of blastocytes and also in the primordial germ cell. The gene *POU5F1* encodes the OCT4 protein. Synonyms to the gene name include *OCT3, OCT4, OTF3* and *MGC22487.* The presence of OCT4 at specific concentrations is necessary for embryonic stem cells to remain undifferentiated.

Preferably, "OCT4 protein" or simply "OCT4" relates to human OCT4 and preferably comprises an amino acid sequence encoded by the nucleic acid according to SEQ ID NO: 1, preferably the amino acid sequence according to SEQ ID NO: 2. One skilled in the art would understand that the cDNA sequence of OCT4 as described above would be equivalent to OCT4 mRNA, and can be used for the generation of RNA capable of expressing OCT4.

Sox2 is a member of the Sox (SRY-related HMG box) gene family that encode transcription factors with a single HMG DNA-binding domain. SOX2 has been found to control neural progenitor cells by inhibiting their ability to differentiate. The repression of the factor results in delamination from the ventricular zone, which is followed by an exit from the cell cycle. These cells also begin to lose their progenitor character through the loss of progenitor and early neuronal differentiation markers.

Preferably, "SOX2 protein" or simply "SOX2" relates to human SOX2 and preferably comprises an amino acid sequence encoded by the nucleic acid according to SEQ ID NO: 3, preferably the amino acid sequence according to SEQ ID NO: 4. One skilled in the art would understand that the cDNA sequence of SOX2 as described above would be equivalent to SOX2 mRNA, and can be used for the generation of RNA capable of expressing SOX2.

NANOG is a NK-2 type homeodomain gene, and has been proposed to play a key role in maintaining stem cell pluripotency presumably by regulating the expression of genes critical to embryonic stem cell renewal and differentiation. NANOG behaves as a transcription activator with two unusually strong activation domains embedded in its C terminus. Reduction of NANOG expression induces differentiation of embryonic stem cells.

Preferably, "NANOG protein" or simply "NANOG" relates to human NANOG and preferably comprises an amino acid sequence encoded by the nucleic acid according to SEQ ID NO: 5, preferably the amino acid sequence according to SEQ ID NO: 6. One skilled in the art would understand that the cDNA sequence of NANOG as described above would be equivalent to NANOG mRNA, and can be used for the generation of RNA capable of expressing NANOG.

LIN28 is a conserved cytoplasmic protein with an unusual pairing of RNA-binding motifs: a cold shock domain and a pair of retroviral-type CCHC zinc fingers. In mammals, it is abundant in diverse types of undifferentiated cells. In pluripotent mammalian cells, LIN28 is observed in RNase-sensitive complexes with Poly(A)-Binding Protein, and in polysomal fractions of sucrose gradients, suggesting it is associated with translating mRNAs.

Preferably, "LIN28 protein" or simply "LIN28" relates to human LIN28 and preferably comprises an amino acid sequence encoded by the nucleic acid according to SEQ ID NO: 7, preferably the amino acid sequence according to SEQ ID NO: 8. One skilled in the art would understand that the cDNA sequence of LIN28 as described above would be equivalent to LIN28 mRNA, and can be used for the generation of RNA capable of expressing LIN28.

Krueppel-like factor (KLF4) is a zinc-finger transcription factor, which is strongly expressed in postmitotic epithelial cells of different tissues, e.g. the colon, the stomach and the skin. KLF4 is essential for the terminal differentiation of these cells and involved in the cell cycle regulation.

Preferably, "KLF4 protein" or simply "KLF4" relates to human KLF4 and preferably comprises an amino acid sequence encoded by the nucleic acid according to SEQ ID NO: 9, preferably the amino acid sequence according to SEQ ID NO: 10. One skilled in the art would understand that the cDNA sequence of KLF4 as described above would be equivalent to KLF4 mRNA, and can be used for the generation of RNA capable of expressing KLF4.

MYC (cMYC) is a protooncogene, which is overexpressed in a wide range of human cancers. When it is specifically-mutated, or overexpressed, it increases cell proliferation and functions as an oncogene. MYC gene encodes for a transcription factor that regulates expression of 15% of all genes through binding on Enhancer Box sequences (E-boxes) and recruiting histone acetyltransferases (HATs). MYC belongs to MYC family of transcription factors, which also includes N-MYC and L-MYC genes. MYC-family transcription factors contain the bHLH/LZ (basic Helix-Loop-Helix Leucine Zipper) domain

Preferably, "cMYC protein" or simply "cMYC" relates to human cMYC and preferably comprises an amino acid sequence encoded by the nucleic acid according to SEQ ID NO: 11, preferably the amino acid sequence according to SEQ ID NO: 12. One skilled in the art would understand that the cDNA sequence of cMYC as described above would be equivalent to cMYC mRNA, and can be used for the generation of RNA capable of expressing cMYC.

A reference herein to specific factors such as OCT4, SOX2, NANOG, LIN28, KLF4 or c-MYC or to specific sequences thereof is to be understood so as to also include all variants of these specific factors or the specific sequences thereof as described herein. In particular, it is to be understood so as to also include all splice variants, posttranslationally modified variants, conformations, isoforms and species homologs of these specific factors/sequences which are naturally expressed by cells.

Preferably, the step of allowing the development of cells having stem cell characteristics used in the methods for providing cells having stem cell characteristics described herein comprises culturing the somatic cells under embryonic stem cell culture conditions, preferably conditions suitable for maintaining pluripotent stem cells in an undifferentiated state.

Preferably, to allow the development of cells having stem cell characteristics, cells are cultivated in the presence of one or more DNA methyltransferase inhibitors and/or one or more histone deacetylase inhibitors. Preferred compounds are selected from the group consisting of 5'-azacytidine (5'-azaC), suberoylanilide hydroxamic acid (SAHA), dexamethasone, trichostatin A (TSA), sodium butyrate (NaBu), Scriptaid and valproic acid (VPA). Preferably, cells are cultivated in the presence of valproic acid (VPA), preferably in a concentration of between 0.5 and 10 mM, more preferably between 1 and 5 mM, most preferably in a concentration of about 2 mM.

The methods of the present invention can be used to effect de-differentiation of any type of somatic cell. Cells that may be used include cells that can be de-differentiated or reprogrammed by the methods of the present teaching, in particular cells that are fully or partially differentiated, more preferably terminally differentiated. Preferably, the somatic cell is a diploid cell derived from pre-embryonic, embryonic, fetal, and post-natal multi-cellular organisms. Examples of cells that may be used include but are not limited to fibroblasts, such as fetal and neonatal fibroblasts or adult fibroblasts, keratinocytes, in particular primary keratinocytes, more preferably keratinocytes derived from hair, adipose cells, epithelial cells, epidermal cells, chondrocytes, cumulus cells, neural cells, glial cells, astrocytes, cardiac cells, esophageal cells, muscle cells, melanocytes, hematopoietic cells, osteocytes, macrophages, monocytes, and mononuclear cells.

The cells with which the methods of the teaching can be used can be of any animal species; e.g., mammals and rodents. Examples of mammalian cells that can be de-differentiated and re-differentiated by the present invention include but are not limited to human and non-human primate cells. Primate cells with which the invention may be performed include but are not limited to cells of humans, chimpanzees, baboons, cynomolgus monkeys, and any other New or Old World monkeys. Rodent cells with which the invention may be performed include but are not limited to mouse, rat, guinea pig, hamster and gerbil cells.

De-differentiated cells prepared according to the present teaching are expected to display many of the same requirements as pluripotent stem cells and can be expanded and maintained under conditions used for embryonic stem cells, e.g. ES cell medium or any medium that supports growth of the embryonic cells. Embryonic stem cells retain their pluripotency *in vitro* when maintained on inactivated fetal fibroblasts such as irradiated mouse embryonic fibroblasts or human fibroblasts (e.g., human foreskin fibroblasts, human skin fibroblasts, human endometrial fibroblasts, human oviductal fibroblasts) in culture. In one embodiment, the human feeder cells may be autologous feeder cells derived from the same culture of reprogrammed cells by direct differentiation.

Furthermore, human embryonic stem cells can successfully be propagated on Matrigel in a medium conditioned by mouse fetal fibroblasts. Human stem cells can be grown in culture for extended period of time and remain undifferentiated under specific culture conditions.

In certain embodiments, the cell culture conditions may include contacting the cells with factors that can inhibit differentiation or otherwise potentiate de-differentiation of cells, e.g., prevent the differentiation of cells into non-ES cells, trophectoderm or other cell types.

De-differentiated cells prepared according to the present invention can be evaluated by methods including monitoring changes in the cells' phenotype and characterizing their gene and protein expression. Gene expression can be determined by RT-PCR, and translation products can be determined by immunocytochemistry and Western blotting. In particular, de-differentiated cells can be characterized to determine the pattern of gene expression and whether the reprogrammed cells display a pattern of gene expression similar to the expression pattern expected of undifferentiated, pluripotent control cells such as embryonic stem cells using techniques well known in the art including transcriptomics.

The expression of the following genes of de-differentiated cells can be assessed in this respect: OCT4, NANOG, growth and differentiation factor 3 (GDF3), reduced expression 1 (REX1), fibroblast growth factor 4 (FGF4), embryonic cell-specific gene 1 (ESG1), developmental pluripotency-associated 2 (DPPA2), DPPA4, telomerase reverse transcriptase (TERT), embryonic antigen-3 (SSEA-3), SSEA-4, tumor-related antigen-1-60 (TRA-1-60), TRA-1-81, and TRA-2-49/6E.

The undifferentiated or embryonic stem cells to which the reprogrammed cells may be compared may be from the same species as the differentiated somatic cells. Alternatively, the undifferentiated or embryonic stem cells to which the reprogrammed cells may be compared may be from a different species as the differentiated somatic cells.

In some embodiments, a similarity in gene expression pattern exists between a reprogrammed cell and an undifferentiated cell, e.g., embryonic stem cell, if certain genes specifically expressed in an undifferentiated cell are also expressed in the reprogrammed cell. For example, certain genes, e.g., telomerase, that are typically undetectable in differentiated somatic cells may be used to monitor the extent of reprogramming. Likewise, for certain genes, the absence of expression may be used to assess the extent of reprogramming.

Self-renewing capacity, marked by induction of telomerase activity, is another characteristic of stem cells that can be monitored in de-differentiated cells.

Karyotypic analysis may be performed by means of chromosome spreads from mitotic cells, spectral karyotyping, assays of telomere length, total genomic hybridization, or other techniques well known in the art.

Using the present teaching, RNA encoding appropriate factors is incorporated into one or more somatic cells, e.g. by electroporation. After incorporation, cells are preferably cultured using conditions that support maintenance of de-differentiated cells (i.e. stem cell culture conditions). The de-differentiated cells can then be expanded and induced to re-differentiate into different type of somatic cells that are needed for cell therapy. De-differentiated cells obtained according to the present teaching can be induced to differentiate into one or more desired somatic cell types *in vitro* or *in vivo.*

Preferably, the de-differentiated cells obtained according to the present invention may give rise to cells from any of three embryonic germ layers, i.e., endoderm, mesoderm, and ectoderm. For example, the de-differentiated cells may differentiate into skeletal muscle, skeleton, dermis of skin, connective tissue, urogenital system, heart, blood (lymph cells), and spleen (mesoderm); stomach, colon, liver, pancreas, urinary bladder; lining of urethra, epithelial parts of trachea, lungs, pharynx, thyroid, parathyroid, intestine (endoderm); or central nervous system, retina and lens, cranial and sensory, ganglia and nerves, pigment cells, head connective tissue, epidermis, hair, mammary glands (ectoderm). The de-differentiated cells obtained according to the present teaching can be re-differentiated *in vitro* or *in vivo* using techniques known in the art.

In one embodiment, the reprogrammed cells resulting from the methods described herein are used to produce differentiated progeny. Thus, in one aspect, the present teaching provides a method for producing differentiated cells, comprising: (i) obtaining reprogrammed cells using the methods of this teaching; and (ii) inducing differentiation of the reprogrammed cells to produce differentiated cells. Step (ii) can be performed *in vivo* or *in vitro.* Furthermore, differentiation can be induced through the presence of appropriate differentiation factors which can either be added or are present *in situ,* e.g. in a body, organ or tissue into which the reprogrammed cells have been introduced. The differentiated cells can be used to derive cells, tissues and/or organs which are advantageously used in the area of cell, tissue, and/or organ transplantation. If desired, genetic modifications can be introduced, for example, into somatic cells prior to reprogramming. The differentiated cells of the present teaching preferably do not possess the pluripotency of an embryonic stem cell, or an embryonic germ cell, and are, in essence, tissue-specific partially or fully differentiated cells.

One advantage of the methods of the present invention is that the reprogrammed cells obtained by the present teaching can be differentiated without prior selection or purification or establishment of a cell line. Accordingly in certain embodiments, a heterogeneous population of cells comprising reprogrammed cells are differentiated into a desired cell type. In one embodiment, a mixture of cells obtained from the methods of the present invention is exposed to one or more differentiation factors and cultured *in vitro.*

Methods of differentiating reprogrammed cells obtained by the methods disclosed herein may comprise a step of permeabilization of the reprogrammed cell. For example, cells generated by the reprogramming techniques described herein, or alternatively a heterogeneous mixture of cells comprising reprogrammed cells, may be permeabilized before exposure to one or more differentiation factors or cell extract or other preparation comprising differentiation factors.

For example, differentiated cells may be obtained by culturing undifferentiated reprogrammed cells in the presence of at least one differentiation factor and selecting differentiated cells from the culture. Selection of differentiated cells may be based on phenotype, such as the expression of certain cell markers present on differentiated cells, or by functional assays (e.g., the ability to perform one or more functions of a particular differentiated cell type).

In another embodiment, the cells reprogrammed according to the present invention are genetically modified through the addition, deletion, or modification of their DNA sequence(s).

The reprogrammed or de-differentiated cells prepared according to the present teaching or cells derived from the reprogrammed or de-differentiated cells are useful in research and in therapy. Reprogrammed pluripotent cells may be differentiated into any of the cells in the body including, without limitation, skin, cartilage, bone skeletal muscle, cardiac muscle, renal, hepatic, blood and blood forming, vascular precursor and vascular endothelial, pancreatic beta, neurons, glia, retinal, neuronal, intestinal, lung, and liver cells.

The reprogrammed cells are useful for regenerative/reparative therapy and may be transplanted into a patient in need thereof. In one embodiment, the cells are autologous with the patient.

The reprogrammed cells provided in accordance with the present invention may be used, for example, in therapeutic strategies in the treatment of cardiac, neurological, endocrinological, vascular, retinal, dermatological, muscular-skeletal disorders, and other diseases.

For example, and not intended as a limitation, the reprogrammed cells of the present teaching can be used to replenish cells in animals whose natural cells have been depleted due to age or ablation therapy such as cancer radiotherapy and chemotherapy. In another non-limiting example, the reprogrammed cells of the present invention are useful in organ regeneration and tissue repair. In one embodiment of the present teaching, reprogrammed cells can be used to reinvigorate damaged muscle tissue including dystrophic muscles and muscles damaged by ischemic events such as myocardial infarcts. In another embodiment, the reprogrammed cells disclosed herein can be used to ameliorate scarring in animals, including humans, following a traumatic injury or surgery. In this embodiment, the reprogrammed cells of the present invention are administered systemically, such as intravenously, and migrate to the site of the freshly traumatized tissue recruited by circulating cytokines secreted by the damaged cells. In another embodiment, the reprogrammed cells can be administered locally to a treatment site in need or repair or regeneration.

In further embodiments, the RNA used in accordance with the present teaching encodes a peptide or protein, which is of therapeutic value. Cells containing the RNA can, for example, be manipulated *in vitro* to express the RNA and thus, the peptide or protein, using the methods described herein. The cells expressing the peptide or protein can subsequently be introduced into a patient.

In a particularly preferred embodiment, the RNA used in the present teaching encodes a peptide or protein comprising an immunogen, antigen or antigen peptide. In one embodiment, the peptide or protein is processed after expression to provide said immunogen, antigen or antigen peptide. In another embodiment, the peptide or protein itself is the immunogen, antigen or antigen peptide. Cells expressing such peptide or protein comprising an immunogen, antigen or antigen peptide can be used, for example, in immunotherapy to elicit an immune response against the immunogen, antigen or antigen peptide in a patient.

An "antigen" according to the invention covers any substance that will elicit an immune response. In particular, an "antigen" relates to any substance that reacts specifically with antibodies or T-lymphocytes (T-cells). According to the present teaching, the term "antigen" comprises any molecule which comprises at least one epitope. Preferably, an antigen in the context of the present teaching is a molecule which, optionally after processing, induces an immune reaction, which is preferably specific for the antigen. According to the present teaching, any suitable antigen may be used, which is a candidate for an immune reaction, wherein the immune reaction may be both a humoral as well as a cellular immune reaction. In the context of the embodiments of the present teaching, the antigen is preferably presented by a cell, preferably by an antigen presenting cell, in the context of MHC molecules, which results in an immune reaction against the antigen. An antigen is preferably a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present teaching, an antigen may correspond to a naturally occurring product, for example, a viral protein, or a part thereof.

In a preferred embodiment, the antigen is a tumor antigen, i.e., a part of a tumor cell, which may be derived from the cytoplasm, the cell surface or the cell nucleus, in particular those which primarily occur intracellularly or as surface antigens of tumor cells. For example, tumor antigens include the carcinoembryonal antigen, α1-fetoprotein, isoferritin, and fetal sulphoglycoprotein, α2-H-ferroprotein and γ-fetoprotein, as well as various virus tumor antigens. According to the present teaching, a tumor antigen preferably comprises any antigen which is characteristic for tumors or cancers as well as for tumor or cancer cells with respect to type and/or expression level. In another embodiment, the antigen is a virus antigen such as viral ribonucleoprotein or coat protein. In particular, the antigen should be presented by MHC molecules which results in modulation, in particular activation of cells of the immune system, preferably CD4⁺ and CD8⁺ lymphocytes, in particular via the modulation of the activity of a T-cell receptor.

In preferred embodiments, the antigen is a tumor antigen and the present teaching involves the stimulation of an anti-tumor CTL response against tumor cells expressing such tumor antigen and preferably presenting such tumor antigen with class I MHC.

The term "immunogenicity" relates to the relative effectivity of an antigen to induce an immune reaction.

The term "pathogen" relates to pathogenic microorganisms and comprises viruses, bacteria, fungi, unicellular organisms, and parasites. Examples for pathogenic viruses are human immunodeficiency virus (HIV), cytomegalovirus (CMV), herpes virus (HSV), hepatitis A-virus (HAV), HBV, HCV, papilloma virus, and human T-lymphotrophic virus (HTLV). Unicellular organisms comprise plasmodia trypanosomes, amoeba, etc.

Examples for antigens that may be used in accordance with the methods of the present teaching are p53, ART-4, BAGE, ss-catenin/m, Bcr-abL CAMEL, CAP-1, CASP-8, CDC27/m, CDK4/m, CEA, CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (orhTRT), LAGE, LDLR/FUT, MAGE-A, preferably MAGE-A1, MAGE-A2, MAGE-A3, MAGE-A4, MAGE-A5, MAGE-A6, MAGE-A7, MAGE-A8, MAGE-A9, MAGE-A10, MAGE-A11, or MAGE-A12, MAGE-B, MAGE-C, MART-1/Melan-A, MC1R, Myosin/m, MUC1, MUM-1, - 2, -3, NA88-A, NF1, NY-ESO-1, NY-BR-1, p190 minor BCR-abL, Plac-1, Pm1/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1, TRP-2, TRP-2/INT2, TPTE and WT, preferably WT-1.

"A portion or fragment of an antigen" or "an antigen peptide" according to the teaching preferably is an incomplete representation of an antigen and is capable of eliciting an immune response against the antigen.

In this context, the teaching also makes use of peptides comprising amino acid sequences derived from antigens, also termed "antigen peptides" herein. By "antigen peptide", or "antigen peptide derived from an antigen" is meant an oligopeptide or polypeptide comprising an amino acid sequence substantially corresponding to the amino acid sequence of a fragment or peptide of an antigen. An antigen peptide may be of any length.

Preferably, the antigen peptides are capable of stimulating an immune response, preferably a cellular response against the antigen or cells characterized by expression of the antigen and preferably by presentation of the antigen. Preferably, an antigen peptide is capable of stimulating a cellular response against a cell characterized by presentation of an antigen with class I MHC and preferably is capable of stimulating an antigen-responsive CTL. Preferably, the antigen peptides according to the invention are MHC class I and/or class II presented peptides or can be processed to produce MHC class I and/or class II presented peptides. Preferably, the antigen peptides comprise an amino acid sequence substantially corresponding to the amino acid sequence of a fragment of an antigen. Preferably, said fragment of an antigen is an MHC class I and/or class II presented peptide. Preferably, an antigen peptide according to the invention comprises an amino acid sequence substantially corresponding to the amino acid sequence of such fragment and is processed to produce such fragment, i.e., an MHC class I and/or class II presented peptide derived from an antigen.

If an antigen peptide is to be presented directly, i.e., without processing, in particular without cleavage, it has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length. Preferably the sequence of an antigen peptide which is to be presented directly is derived from the amino acid sequence of an antigen, i.e., its sequence substantially corresponds and is preferably completely identical to a fragment of an antigen.

If an antigen peptide is to be presented following processing, in particular following cleavage, the peptide produced by processing has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length. Preferably, the sequence of the peptide which is to be presented following processing is derived from the amino acid sequence of an antigen, i.e., its sequence substantially corresponds and is preferably completely identical to a fragment of an antigen. Thus, an antigen peptide according to the invention in one embodiment comprises a sequence of 7-20 amino acids in length, more preferably 7-12 amino acids in length, more preferably 8-11 amino acids in length, in particular 9 or 10 amino acids in length which substantially corresponds and is preferably completely identical to a fragment of an antigen and following processing of the antigen peptide makes up the presented peptide. However, the antigen peptide may also comprise a sequence which substantially corresponds and preferably is completely identical to a fragment of an antigen which is even longer than the above stated sequence. In one embodiment, an antigen peptide may comprise the entire sequence of an antigen.

Peptides having amino acid sequences substantially corresponding to a sequence of a peptide which is presented by the class I MHC may differ at one or more residues that are not essential for TCR recognition of the peptide as presented by the class I MHC, or for peptide binding to MHC. Such substantially corresponding peptides are also capable of stimulating an antigen-responsive CTL. Peptides having amino acid sequences differing from a presented peptide at residues that do not affect TCR recognition but improve the stability of binding to MHC may improve the immunogenicity of the antigen peptide, and may be referred to herein as "optimized peptide". Using existing knowledge about which of these residues may be more likely to affect binding either to the MHC or to the TCR, a rational approach to the design of substantially corresponding peptides may be employed. Resulting peptides that are functional are contemplated as antigen peptides.

"Antigen processing" refers to the degradation of an antigen into fragments (e.g., the degradation of a protein into peptides) and the association of one or more of these fragments (e.g., via binding) with MHC molecules for presentation by "antigen presenting cells" to specific T-cells.

"Antigen presenting cells" (APC) are cells which present peptide fragments of protein antigens in association with MHC molecules on their cell surface. Some APCs may activate antigen-specific T-cells.

The term "immunotherapy" relates to a treatment involving activation of a specific immune reaction.

The term *"in vivo"* relates to the situation in a subject.

The terms "subject" and "individual" are used interchangeably and relate to mammals. For example, mammals in the context of the present invention are humans, non-human primates, domesticated animals such as dogs, cats, sheep, cattle, goats, pigs, horses etc., laboratory animals such as mice, rats, rabbits, guinea pigs, etc. as well as animals in captivity such as animals of zoos. The term "animal" as used herein also includes humans. The term "subject" may also include a patient, i.e., an animal, preferably a human having a disease.

If according to the teaching administration to a subject is desired the composition for administration is generally administered in pharmaceutically compatible amounts and in pharmaceutically compatible preparations. The term "pharmaceutically compatible" refers to a nontoxic material, which does not interact with the action of the active component of the pharmaceutical composition. Preparations of this kind may usually contain salts, buffer substances, preservatives, excipients and carriers and are administered in a manner known to the skilled person.

The present teaching is described in detail by the figures and examples below, which are used only for illustration purposes.

### Example 1:

### Cell culture

Primary human newborn foreskin fibroblasts (CCD-1079Sk, CCDs), BJ human neonatal foreskin fibroblasts were obtained from ATCC (Manassas, VA, USA) and cultivated in MEM (Invitrogen, Karlsruhe, Germany) supplemented with 10% heat-inactivated fetal bovine serum gold (PAA Labratories, Pasching, Austria), 1x non-essential amino acids (Invitrogen), 1mM sodium pyruvate (Invitrogen), 2mM glutamine (Invitrogen), 50U/ml penicillin (Invitrogen) and 50µg/ml streptomycin (Invitrogen). Another charge of human neonatal foreskin fibroblasts (HFF) were obtained from SBI (Mountain View, CA, USA) and cultivated similar to CCDs and BJs. Murine embryonic fibroblasts (MEFs) were isolated from 14 days old C57BL/6 mice embryos and cultivated in DMEM (Invitrogen) supplemented with 15% heat-inactivated fetal bovine serum, 1x non-essential amino acids, 1mM sodium pyruvate, 2mM glutamine, 50U/ml penicillin and 50µg/ml streptomycin. Human epidermal keratinocytes were obtained from PromoCell (Heidelberg, Germany) and cultured in keratinocyte basal medium 2 with additives included by the supplier (PromoCell, Heidelberg, Germany). Human umbilical vein endothelial cells (HUVEC) from Lonza (Walkersville, MD, USA) were cultivated using the Clonetics ® Endothelial Cell System (Lonza).

Peripheral blood mononuclear cells (PBMCs) were isolated by Ficoll Hypaque (Amersham Biosciences, Glattbrugg, Switzerland) density gradient centrifugation from buffy coats obtained from healthy blood bank donors. Monocytes were enriched from PBMCs with anti-CD14 microbeads (Miltenyi Biotec, Bergisch-Gladbach, Germany) according to the manufacturer's instructions. To obtain immature dendritic cells (iDCs), monocytes were differentiated for 5 days in RPMI 1640 medium (Invitrogen) with 2mM glutamine, 100U/ml penicillin, 100µg/ml streptomycin, 1mM sodium pyruvate, 1x non-essential amino acids, 10% heat inactivated human AB-serum supplemented with 1000U/ml GM-CSF (Essex, Luzern, Switzerland) and 1000 U/ml IL-4 (Strathmann Biotech, Hamburg, Germany).

### Generation of in vitro transcribed RNA

Plasmid constructs used as templates for *in vitro* transcription of RNAs encoding luciferase, eGFP, destabilized eGFP (d2eGFP), OCT4, SOX2, KLF4, cMYC, LIN28, NANOG, SV40 largeT Antigen, E6, p53-DD, E3 and K3 were based on pST1-2hBgUTR-A120 (Holtkamp 2006). The nucleotide sequence of the genes was codon optimized to increase the GC-content and to enhance translation in target cells. For generation of *in vitro* transcribed RNAs, plasmids were linearized downstream of the poly(A) tail using a class II restriction endonuclease. Linearized plasmids were purified by phenol-chloroform extraction and ethanol precipitation, quantified spectrophotometrically, and subjected to *in vitro* transcription with T7 RNA polymerase using the MEGAscript T7 Kit (Ambion, Austin, TX, USA). For incorporation of the cap analog β-S-ARCA (D1), 6mM of the analog was added to the reaction whereas the GTP concentration was lowered to 1.5mM. Reactions were incubated 3-6h at 37°C, DNase (Ambion) treated and purified using the the MEGAclear Kit (Ambion) according to the manufacturer.

### Transfer of IVT-RNA into cells

Transfer of IVT-RNA into cells was performed by electroporation (EP). To this end, 1-10x10⁷ cells per EP were harvested, washed sequentially with PBS supplemented with 2mM EDTA and X-VIVO 15 medium (Lonza), resuspended in 125µl X-VIVO 15 medium and transferred to a 2-mm gap sterile electroporation cuvette (Bio-RAD, Hercules, CA, USA). The appropriate amount of *in vitro* transcribed RNA was added and cells were electroporated using a BTX ECM® 830 Electroporation System (BTX, Holliston, MA, USA) and previously optimized electroporation parameters for CCDs (110V, 3x12ms pulse, 400ms interval), BJs (110V, 3x12ms pulse, 400ms interval), MEFs (125V, 5x6ms pulse, 400ms interval), HFFs (125V, 1x24ms pulse), Keratinocytes (125V, 4x6ms pulse, 200ms interval) and HUVECs (125V, 1x20ms pulse). Human PBMCs and iDC were electroporated in a 4-mm gap sterile electroporation cuvette (Bio-RAD) using a Gene-Pulser-II apparatus (Bio-Rad, Munich, Germany) with voltage and capacitance settings of 450V/250µF (PBMC) 300V/150µF (iDC). Cells were diluted in culture media immediately after electroporation.

### Reporter gene assays

For luciferase assays, cells electroporated with *in vitro* transcribed RNA coding for luciferase were plated in 96-well white flat-bottom microplates (Nunc, Langenselbold, Germany) and incubated at 37°C. Lysis of cells was performed using the 'Bright-Glo Luciferase Assay System' (Promega, Madison, WI, USA). Bioluminescence flux was measured with the Infinite M200 microplate luminescence reader (Tecan, Crailsheim, Germany) with 1s integration time. Data of Luciferase activity presented herein is in counts per second.

For quantification of d2eGFP and eGFP protein, cells were washed in PBS, fixed using PBS supplemented with 2% formaldehyde and analyzed by flow cytometry on a FACS Canto II flow cytometer (BD Biosciences, Heidelberg, Germany). Gating was performed on living cells and mean fluorescence intensity (MFI) of GFP expressing cell populations was quantified using FlowJo software (Tree Star, Ashland, OR, USA). Human T-cell subpopulations of electroporated PBMCs were stained with CD4 or CD8 reactive fluorescent antibodies prior to flow cytometry. Gating was performed on living CD4+ and CD8+ T-cells and GFP expression and MFI of both T-cell subpopulations was determined by flow cytometry.

### Quantification of transcript levels by real-time reverse transcriptase-PCR

Total cellular RNA was extracted from cells with RNeasy Mini Kit or RNeasy Micro Kit (Qiagen, Hilden, Germany), reverse transcribed with oligo-dT18 using Superscript II (Invitrogen, Carlsbad, CA, USA), and subjected to real-time quantitative analysis on ABI PRISM 7700 Sequence Detection System instrument and software (Applied Biosystems, Foster City, CA, USA) using the QuantiTect SYBR Green PCR Kit (Qiagen). Reactions were performed in triplicates with primer amplifying a specific region of the codon optimized OCT4
(OCT4-s: 5'-ACCTGGAAAACCTGTTCCTGC-3' (SEQ ID NO: 14); OCT4-as: 5'-AGCTCGGATCCTCATCAGTTG-3' (SEQ ID NO: 15)),
SOX2 (SOX2-s: 5'-AACCAGCGGATGGACAGCTAC-3' (SEQ ID NO: 16); SOX2-as: 5'-GCTTTTCACCACGCTGCCCAT-3' (SEQ ID NO: 17)),
KLF4 (KLF4-s: 5'-AAGACCTACACCAAGAGCAGC-3' (SEQ ID NO: 18); KLF4-as: 5'-AGGTGGTCAGATCTGCTGAAG-3' (SEQ ID NO: 19)) and
cMYC (cMYC-s: 5'-CCCCTGAACGACAGCTCTAGC-3' (SEQ ID NO: 20); cMYC-as: 5'-TTCTCCACGGACACCACGTCG-3' (SEQ ID NO: 21)) or
   the endogenous transcripts of
IFNα (IFNα-s: 5-AAATACAGCCCTTGTGCCTGG-3' (SEQ ID NO: 22); IFNα-as: 5'-GGTGAGCTGGCATACGAATCA-3' (SEQ ID NO: 23)),
IFNβ (IFNβ-s: 5'-AAGGCCAAGGAGTACAGTC-3' (SEQ ID NO: 24); IFNβ-as: 5'-ATCTTCAGTTTCGGAGGTAA-3' (SEQ ID NO: 25)),
GDF3 (GDF3-s: 5'-TCCCAGACCAAGGTTTCTTTC-3' (SEQ ID NO: 26); GDF3-as: 5'-TTACCTGGCTTAGGGGTGGTC-3' (SEQ ID NO: 27)) and
TERT (TERT-s: 5'-CCTGCTCAAGCTGACTCGACACCGTG-3' (SEQ ID NO: 28); TERT-as: 5'-GGAAAAGCTGGCCCTGGGGTGGAGC-3' (SEQ ID NO: 29)) (each 333nM)
with initial denaturation/activation for 15min at 95°C, and 40 cycles of 30s at 95°C, 30s at primer specific annealing temperature (OCT4, cMYC, IFNα, IFNβ, GDF3, TERT: 60°C; SOX2: 64°C; KLF4: 58°C) and 30s at 72°C. Expression of transcripts was quantified using ΔΔCt calculation relative to HPRT-encoding RNA as internal standard (HPRT-s, 5'-TGACACTGGCAAAACAATGCA-3' (SEQ ID NO: 30); HPRT-as: 5'-GGTCCTTTTCACCAGCAAGCT-3' (SEQ ID NO: 31); PCR conditions as above with 62°C as annealing temperature) to normalize for variances in the quality of RNA and the amount of input complementary DNA.

### Intracellular FACS staining for OCT4, SOX2 and NANOG

Intracellular FACS staining of OCT4, SOX2 and NANOG was done using the human pluripotent stem cell transcription factor analysis kit following the manufacturer's instructions (BD Bioscience). Briefly, 3-5x10⁵ cells were harvested, washed twice with PBS and fixed for 20min in 250µl BD Cytofix Buffer (BD Biosciences) at room temperature. After the incubation cells were washed twice with PBS and permeabilized by washing twice with 1xPerm/Wash Buffer (BD Bioscience) followed by 10 minutes incubation in 50µl 1x Perm/Wash at room temperature. Staining with isotype control and specific antibodies was performed for 30min at room temperature in the dark followed by washing two times with 1xPerm/Wash. Cells were resuspended in PBS supplemented with 2% formaldehyde and analyzed by flow cytometry as described above.

### Example 2:

Primary human foreskin fibroblasts (CCD1079SK) were electroporated with 1 µg IVT RNA encoding for firefly luciferase and 5 µg IVT RNA encoding for destabilized GFP (luc+GFP). Electroporations were performed in 2 mm gap cuvettes using optimized parameters. The cells were plated in 6-well-plates at a density of 300000 cells/well and either left untreated or incubated with 2 µM C₁₃H₈N₄OS (PKR-inhibitor). 24h later cells were harvested for total RNA extraction and reverse transcription. The induction of type-I interferons was assessed by qRT-PCR using specific primer for IFNα or IFNβ in the presence or absence of PKR-inhibitor as indicated in Fig. 1. Panels A and C show the same samples as B and D with reduced values of the y-axis.

Upregulation of interferon transcripts in human fibroblasts induced by RNA electroporation was observed (Fig 1A and 1C). Surprisingly, the inhibition of PKR using C₁₃H₈N₄OS resulted in an overwhelming increase of interferon transcripts (Fig 1B and 1D): IFN-α was induced even in the absence of RNA, IFN-β was induced by C₁₃H₈N₄OS only in the presence of RNA. These data show that the inhibition of PKR using a small molecule inhibitor does not abrogate the interferon response pathway.

### Example 3:

Human and mouse primary fibroblasts were electroporated with IVT RNA encoding the reporter genes firefly luciferase and GFP. Electroporations were performed in 2 mm gap cuvettes using optimized parameters for each cell type. Immediately after electroporation the cells were plated in absence or presence of increasing concentrations of the C₁₃H₈N₄OS PKR-inhibitor.

As depicted in Fig. 2 and 3 a dose dependent increase of the reporter genes luciferase and GFP in human and mouse fibroblasts was observed. In the presence of the highest concentration used (2 µM), a high level of stabilization of luciferase and GFP expression was observed.

### Example 4:

Primary human umbilical vein endothelial cells (HUVECs) were electroporated with IVT RNA and the effect of PKR inhibition on reporter gene expression was analysed in similar experimental settings as described above. Electroporations were performed in 2 mm gap cuvettes using optimized parameters for HUVECs. Luciferase expression was enhanced in HUVECs in a dose dependent manner. However, the expression is not as long lasting as observed in fibroblasts (Fig 4).

Next similar experiments were performed with human CD4 and CD8-positive T-cells as well as immature dendritic cells (iDCs). Electroporations were performed using optimized parameters for each cell type. In contrast to our previous observations in fibroblasts and endothelial cells, neither in T-cells nor in iDCs PKR inhibition led to an increased GFP expression (Fig. 5A, B). In iDCs, the inhibition of PKR resulted in loss of luciferase expression (Fig 5C).

### Example 5:

In order to investigate whether the observed stabilization of reporter gene expression is due to an irreversible inhibition of PKR by high concentrations of C₁₃H₈N₄OS we decided to perform transient treatments of electroporated cells. Furthermore a second PKR inhibitor, 2-aminopurine (2-AP) was included into this experiment. Both inhibitors were used at 2 µM final concentrations. As depicted in Fig. 6, increased protein translation from IVT RNA is dependent on the incubation time with both inhibitors. A 7h incubation period with either PKR inhibitor did not results in a significant increase of luciferase expression and a 24h treatment was rapidly reversed after removal of the inhibitors. Even a 48h treatment was less efficient than a permanent treatment. These data show that PKR inhibition is reversible. Permanent inhibition results in high expression levels for 5 days.

### Example 6:

Viral protein which are known to inhibit PKR, namely the vaccinia virus proteins E3 and K3, were investigated. Both proteins were encoded by IVT RNA and co-transferred by electroporation into fibroblasts, together with IVT RNA encoding reporter genes. In addition, we incubated a part of the cells with 2 µM C₁₃H₈N₄OS to investigate additive effects. Both viral proteins led to a 2 fold increase in luciferase expression 24h after electroporation. There was no additive effect of the two proteins. Also, the expression was not stabilized as observed with the small molecule inhibitors, but rapidly declined to the same level as the controls without viral proteins (Fig. 7A). The additional application of C₁₃H₈N₄OS resulted in an additive increase of luciferase expression and a stabilization compared to untreated controls as seen before with the small molecule inhibition of PKR (Fig 7B). Similar experiments with HUVEC cells revealed that the combination of E3 and K3 increased luciferase expression, which was additively enhanced by C₁₃H₈N₄OS in a dose dependent fashion (Fig 7C).

In a similar set of experiments we investigated whether viral proteins could rescue the non existing effects of C₁₃H₈N₄OS in T-cells or revert the inhibitory effects in dendritic cells. As depicted in Fig. 8A ands 8B, viral proteins inhibiting PKR did not increase reporter gene expression in T-cells, neither in presence nor in absence of C₁₃H₈N₄OS. In iDCs, viral proteins - as observed before with the inhibitor - decreased the luciferase expression. Also, E3 and K3 did not revert the negative effect of C₁₃H₈N₄OS on luciferase expression (Fig. 8C).

### Example 7:

Similar experiments as described above were performed using IVT RNA composed of unmodified nucleotides as before, and IVT RNA that was composed of 5mC and PU instead of cytidine and uridine. As depicted in Fig. 9, luciferase expression in cells electroporated with unmodified IVT-RNA is greatly enhanced and stabilized by 2 µM C₁₃H₈N₄OS and recapitulates our previous findings. The incorporation of 5mC and PU into the transcripts increases the luciferase expression by about 2-fold as compared to the unmodified control. The use of 2 µM C₁₃H₈N₄OS further increases and stabilizes the expression of luciferase encoded by modified IVT RNA.

### Example 8:

We investigated the stability of reprogramming transcription factor expression upon IVT RNA transfer. In similar experiments described above, we treated electroporated cells either permanently with increasing concentrations of C₁₃H₈N₄OS, or transiently with 2 µM C₁₃H₈N₄OS. As demonstrated in Fig. 10, the expression of the transcription factors OCT4, Nanog and SOX2 was strongly increased and stabilized in treated cells in a dose and time dependent manner.

A direct consequence of reprogramming transcription factor expression is the induction of pluripotency marker genes at the transcriptional level. In order to investigate whether stabilized and increased transcription factor expression by PKR inhibition facilitates marker induction, we electroporated CCD1079Sk fibroblasts with IVT RNA encoding largeT, HPV16-E6, p53DD, OCT4, SOX2, KLF4, CMYC, Nanog and LIN28 (TEP-OSKMLN). As depicted in Fig. 11, GDF3 and hTERT induction was increased by the treatment 72h after electroporation.

### Example 9:

In order to investigate whether the observed stabilized protein expression is related to an increased half life of the transfected IVT RNA, we assessed the pace of intracellular IVT RNA degradation. To this aim, we investigated the intracellular level of transferred IVT RNA 72h after electroporation in transiently treated and untreated cells. As shown in Fig. 12A, the transient treatment of cells with 2 µM C₁₃H₈N₄OS increased the abundance of IVT RNA for 72h. In accordance to this data, the half life of the IVT RNA constructs OCT4, SOX2, KLF4 and cMYC is prolonged for 1-2.5h under PKR-Inhibition as seen in Fig. 12B. Stabilization of reporter expression under PKR-inhibition is therefore to some extent based on stabilization of IVT-RNA constructs.

### Example 10:

Primary human foreskin fibroblasts (HFF) were electroporated with IVT RNA encoding eGFP (10µg) either unmodified or modified (mod.) with 5-methylcytidine (5mC) and pseudouridine (PU) or unmodified eGFP (10 µg) and with 3 µg of modified (5mC and PU) E3 and K3 as indicated in Fig. 13. Electroporations were performed in 2 mm gap cuvettes using optimized parameters for HFFs. 300000 cells/well were plated into 6-well-plates and cells were either left untreated or incubated with 2 µM C₁₃H₈N₄OS. After 24h supernatants were harvested and measured for IFN-secretion using the human IFNα and β Kit from PBL Interferon Source.

IVT-RNA induces the secretion of IFNβ after electroporation. The secretion is remarkably enhanced when cells are incubated with C₁₃H₈N₄OS. These results are in concordance with the results from the qRT-PCR. As expected, induction of IFNβ is reduced when IVT-RNA is modified with 5mC and PU. Addition of E3 and K3-IVT-RNA has no effect. Secretion of IFNα was not observed in these experiments (data not shown).

### Example 11:

Primary human foreskin fibroblasts (CCD1079Sk) were electroporated with an increasing amount of a siRNA-mix targeting PKR (Santa Cruz; sc-36263) ranging from 20nM to 80nM. Electroporations were performed in 2 mm gap cuvettes using optimized parameters for CDDs. Cells were plated into 6wells and 24h or 48h later harvested for total RNA extraction and reverse transcription. The expression level of PKR was assessed by qRT-PCR using specific primer. Depicted are the relative expression levels compared to wildtype cells. It was found that PKR is knocked down to 5-10% with all concentrations of siRNA-mix 24h post electroporation. Only 80nM are sufficient to knock down PKR over 48h; cf. Fig. 14(A).

Primary human foreskin fibroblasts (CCD1079Sk) were electroporated with IVT RNA encoding unmodified eGFP (1.5µg) either alone or with an increasing amount of a siRNA-mix targeting PKR ranging from 20nM to 80nM. Electroporations were performed in 2 mm gap cuvettes using optimized parameters for CCDs. Cells were plated into 6wells and 24h later harvested for total RNA extraction and reverse transcription. The expression level of the IFN-response genes OAS1 and OAS2 was assessed by qRT-PCR using specific primer. Depicted in Fig. 14(B) are the relative expression levels compared to wildtype cells. It was found that expression levels of OAS1 and OAS2 are not significantly altered by addition of siRNA-mix indicating that the siRNA is not inducing IFNs after electroporation. (On the contrary it seems that there is a tendency to reduce IFN response by application of high doses of PKR siRNA-mix.)

Primary human foreskin fibroblasts (CCD1079Sk) were electroporated with or without 80nM of siRNA-mix targeting PKR. 48h later cells were harvested and electroporated a second time with 1 µg IVT RNA encoding for firefly luciferase and for eGFP (2.5µg). All electroporations were performed in 2 mm gap cuvettes using optimized parameters for CCDs. Cells were plated in duplicates into 96-well-plates at a density of 10000 cells/well and either left untreated or incubated with 2µM of C₁₃H₈N₄OS. Luciferase activity was measured at the time points indicated in Fig. 14(C). Mean values of the duplicates are given. It was found that electroporation of cells preincubated with siRNA targeting PKR and therefore being in a state of lacking PKR leads to a quite similar kinetic and increase of reporter gene expression as incubation with 2µM of the PKR-inhibitor C₁₃H₈N₄OS with a slightly lower stabilizing effect. Combination of preincubation with siRNA-mix targeting PKR and the use of the inhibitor leads to an even higher expression level of the reporter transcript but is not that much stabilized as in the presence of the inhibitor alone.

### Example 12:

To study whether C₁₃H₈N₄OS could also enhance translation upon liposomal IVT RNA transfer, 1.2µg 5'-triphosphorylated and non-modified IVT RNA (0.8µg encoding luciferase, 0.4µg GFP) was packaged with 6µl RNAiMAX transfection reagent (Invitrogen) and transfected into human foreskin fibroblasts. After 3h incubation with the transfection mixture, the medium was renewed and supplemented with increasing concentrations of C₁₃H₈N₄OS. At the time points indicated in Fig. 15 the cells were lysed using luciferase stabilizing passive lysis buffer (Promega) according to the manufacturer's instructions. By the end of the time course, luciferase activity of all lysates was measured. C₁₃H₈N₄OS had a stabilizing effect on translation of luciferase. This effect was dose-dependent. One of three independently performed experiments is shown. Thus, the effect of C₁₃H₈N₄OS on translation of IVT RNA is not restricted to the delivery via electroporation. C₁₃H₈N₄OS is also enhancing translation of luciferase when IVT RNA is brought into the cells by liposomes. The effect of C₁₃H₈N₄OS is therefore independently from the route of delivery.

### Example 13:

Human foreskin fibroblasts were electroporated with 2µg IVT RNA coding for firefly luciferase and 5 µg IVT RNA coding for GFP. After electroporation the cells were plated in the presence of increasing concentrations of 2-AP as indicated in the panel legend in Fig. 16. 2 µM C₁₃H₈N₄OS served as positive control. Luciferase expression was followed for 72h. GFP expression proved that all electroporations were successful (data not shown). 10 mM to 20 mM 2-AP lead to a similar increase of translation as 2 µM C₁₃H₈N₄OS.

## Claims

1. Use of an inhibitor of the activity of RNA-dependent protein kinase (PKR) in an *in vitro* method for providing cells having stem cell characteristics, wherein said method comprises the steps of (i) reducing the PKR activity in a cell population comprising somatic cells, (ii) introducing RNA capable of expressing one or more factors allowing the reprogramming of the somatic cells to cells having stem cell characteristics into at least a portion of the somatic cells, and (iii) allowing the development of cells having stem cell characteristics.

2. The use of claim 1, wherein the PKR inhibitor inhibits RNA-induced PKR autophosphorylation.

3. The use of claim 1 or 2, wherein the PKR inhibitor is an ATP-binding site directed inhibitor of PKR.

4. The use of any one of claims 1 to 3, wherein the PKR inhibitor is an imidazolo-oxindole compound.

5. The use of any one of claims 1 to 4, wherein the PKR inhibitor is 6,8-dihydro-8-(1H-imidazol-5-ylmethylene)-7H-pyrrolo[2,3-g]benzothiazol-7-one.

6. The use of any one of claims 1 to 3, wherein the PKR inhibitor is 2-aminopurine.

7. The use of any one of claims 1 to 3, wherein the PKR inhibitor is a virally derived inhibitor of PKR.

8. The use of claim 7, wherein the virally derived inhibitor of PKR is selected from the group consisting of vaccinia virus E3 and/or K3, or their RNA.

## Patentansprüche

1. Verwendung eines Inhibitors der Aktivität von RNA-abhängiger Proteinkinase (PKR) in einem *in vitro*-Verfahren zur Bereitstellung von Zellen mit Stammzelleigenschaften, wobei das Verfahren die Schritte (i) Reduzieren der PKR-Aktivität in einer Zellpopulation, die somatische Zellen umfasst, (ii) Einbringen von RNA, die in der Lage ist, einen oder mehrere Faktoren zu exprimieren, der/die die Reprogrammierung der somatischen Zellen zu Zellen mit Stammzelleigenschaften ermöglicht/ermöglichen, in mindestens einen Teil der somatischen Zellen und (iii) Ermöglichen der Entwicklung von Zellen mit Stammzelleigenschaften umfasst.

2. Verwendung nach Anspruch 1, wobei der PKR-Inhibitor RNA-induzierte PKR-Autophosphorylierung hemmt.

3. Verwendung nach Anspruch 1 oder 2, wobei der PKR-Inhibitor ein ATP-Bindungsstelle-gerichteter Inhibitor von PKR ist.

4. Verwendung nach einem der Ansprüche 1 bis 3, wobei der PKR-Inhibitor eine Imidazolo-Oxindol-Verbindung ist.

5. Verwendung nach einem der Ansprüche 1 bis 4, wobei der PKR-Inhibitor 6,8-Dihydro-8-(1H-imidazol-5-ylmethylen)-7H-pyrrolo[2,3-g]benzothiazol-7-on ist.

6. Verwendung nach einem der Ansprüche 1 bis 3, wobei der PKR-Inhibitor 2-Aminopurin ist.

7. Verwendung nach einem der Ansprüche 1 bis 3, wobei der PKR-Inhibitor ein viral abgeleiteter Inhibitor von PKR ist.

8. Verwendung nach Anspruch 7, wobei der viral abgeleitete Inhibitor von PKR ausgewählt ist aus der Gruppe, bestehend aus Vaccinia-Virus E3 und/oder K3 oder deren RNA.

## Revendications

1. Utilisation d'un inhibiteur de l'activité de la protéine kinase (PKR) dépendante de l'ARN dans un procédé *in vitro* pour fournir des cellules ayant des caractéristiques des cellules souches, dans laquelle ledit procédé comprend
les étapes de (i) réduction de l'activité de la PKR dans une population de cellules comprenant des cellules somatiques, (ii) l'introduction d'ARN capable d'exprimer un ou plusieurs facteurs permettant la reprogrammation des cellule somatiques en cellules ayant des caractéristiques des cellules souches dans au moins une partie des cellules somatiques, et (iii) la permission du développement de cellules ayant des caractéristiques des cellules souches.

2. Utilisation de la revendication 1, dans laquelle l'inhibiteur de la PKR inhibe l'autophosphorylation de la PKR induite par ARN.

3. Utilisation selon la revendication 1 ou 2, dans laquelle l'inhibiteur de la PKR est un inhibiteur de la PKR dirigé contre le site de liaison à l'ATP.

4. Utilisation selon l'une quelconque des revendications 1 à 3, dans laquelle l'inhibiteur de la PKR est un composé d'imidazolo-oxindole.

5. Utilisation selon l'une quelconque des revendications 1 à 4, dans laquelle l'inhibiteur de la PKR est la 6,8-dihydro-8-(1H-imidazol-5-ylméthylène)-7H-pyrrolo[2.3-g]benzothiazol-7-one.

6. Utilisation selon l'une quelconque des revendications 1 à 3, dans laquelle l'inhibiteur de la PKR est la 2-aminopurine.

7. Utilisation selon l'une quelconque des revendications 1 à 3, dans laquelle l'inhibiteur de la PKR est un inhibiteur de la PKR viralement dérivé.

8. Utilisation selon la revendication 7, dans laquelle l'inhibiteur de la PKR viralement dérivé est choisi dans le groupe constitué par le virus de la vaccine E3 et/ou K3, ou leur ARN.


### SEQUENCE LISTING

<110> BioNTech RNA Pharmaceuticals GmbH, et al.
<120> METHOD FOR CELLULAR RNA EXPRESSION
<130> 674-60 PCTEPT1
<150> PCT/EP2010/007362
   <151> 2010-12-03
<160> 32
<170> PatentIn version 3.5
<210> 1
   <211> 1411
   <212> DNA
   <213> Homo sapiens
<400> 1
   ccttcgcaag ccctcatttc accaggcccc cggcttgggg cgccttcctt ccccatggcg        60
   ggacacctgg cttcggattt cgccttctcg ccccctccag gtggtggagg tgatgggcca       120
   ggggggccgg agccgggctg ggttgatcct cggacctggc taagcttcca aggccctcct       180
   ggagggccag gaatcgggcc gggggttggg ccaggctctg aggtgtgggg gattccccca       240
   tgccccccgc cgtatgagtt ctgtgggggg atggcgtact gtgggcccca ggttggagtg       300
   gggctagtgc cccaaggcgg cttggagacc tctcagcctg agggcgaagc aggagtcggg       360
   gtggagagca actccgatgg ggcctccccg gagccctgca ccgtcacccc tggtgccgtg       420
   aagctggaga aggagaagct ggagcaaaac ccggaggagt cccaggacat caaagctctg       480
   cagaaagaac tcgagcaatt tgccaagctc ctgaagcaga agaggatcac cctgggatat       540
   acacaggccg atgtggggct caccctgggg gttctatttg ggaaggtatt cagccaaacg       600
   accatctgcc gctttgaggc tctgcagctt agcttcaaga acatgtgtaa gctgcggccc       660
   ttgctgcaga agtgggtgga ggaagctgac aacaatgaaa atcttcagga gatatgcaaa       720
   gcagaaaccc tcgtgcaggc ccgaaagaga aagcgaacca gtatcgagaa ccgagtgaga       780
   ggcaacctgg agaatttgtt cctgcagtgc ccgaaaccca cactgcagca gatcagccac       840
   atcgcccagc agcttgggct cgagaaggat gtggtccgag tgtggttctg taaccggcgc       900
   cagaagggca agcgatcaag cagcgactat gcacaacgag aggattttga ggctgctggg       960
   tctcctttct cagggggacc agtgtccttt cctctggccc cagggcccca ttttggtacc     1020
   ccaggctatg ggagccctca cttcactgca ctgtactcct cggtcccttt ccctgagggg      1080
   gaagcctttc cccctgtctc cgtcaccact ctgggctctc ccatgcattc aaactgaggt      1140
   gcctgccctt ctaggaatgg gggacagggg gaggggagga gctagggaaa gaaaacctgg      1200
   agtttgtgcc agggtttttg ggattaagtt cttcattcac taaggaagga attgggaaca      1260
   caaagggtgg gggcagggga gtttggggca actggttgga gggaaggtga agttcaatga      1320
   tgctcttgat tttaatccca catcatgtat cacttttttc ttaaataaag aagcctggga       1380
   cacagtagat agacacactt aaaaaaaaaa a                                     1411
<210> 2
   <211> 360
   <212> PRT
   <213> Homo sapiens
<400> 2
   Met Ala Gly His Leu Ala Ser Asp Phe Ala Phe Ser Pro Pro Pro Gly
   1               5                   10                  15
   Gly Gly Gly Asp Gly Pro Gly Gly Pro Glu Pro Gly Trp Val Asp Pro
               20                  25                  30
   Arg Thr Trp Leu Ser Phe Gln Gly Pro Pro Gly Gly Pro Gly Ile Gly
           35                  40                  45
   Pro Gly Val Gly Pro Gly Ser Glu Val Trp Gly Ile Pro Pro Cys Pro
       50                  55                  60
   Pro Pro Tyr Glu Phe Cys Gly Gly Met Ala Tyr Cys Gly Pro Gln Val
   65                  70                  75                  80
   Gly Val Gly Leu Val Pro Gln Gly Gly Leu Glu Thr Ser Gln Pro Glu
                   85                  90                  95
   Gly Glu Ala Gly Val Gly Val Glu Ser Asn Ser Asp Gly Ala Ser Pro
               100                 105                 110
   Glu Pro Cys Thr Val Thr Pro Gly Ala Val Lys Leu Glu Lys Glu Lys
           115                 120                 125
   Leu Glu Gln Asn Pro Glu Glu Ser Gln Asp Ile Lys Ala Leu Gln Lys
       130                 135                 140
   Glu Leu Glu Gln Phe Ala Lys Leu Leu Lys Gln Lys Arg Ile Thr Leu
   145                 150                 155                 160
   Gly Tyr Thr Gln Ala Asp Val Gly Leu Thr Leu Gly Val Leu Phe Gly
                   165                 170                 175
   Lys Val Phe Ser Gln Thr Thr Ile Cys Arg Phe Glu Ala Leu Gln Leu
               180                 185                 190
   Ser Phe Lys Asn Met Cys Lys Leu Arg Pro Leu Leu Gln Lys Trp Val
               195                 200                 205
   Glu Glu Ala Asp Asn Asn Glu Asn Leu Gln Glu Ile Cys Lys Ala Glu
       210                 215                 220
   Thr Leu Val Gln Ala Arg Lys Arg Lys Arg Thr Ser Ile Glu Asn Arg
   225                 230                 235                 240
   Val Arg Gly Asn Leu Glu Asn Leu Phe Leu Gln Cys Pro Lys Pro Thr
                   245                 250                 255
   Leu Gln Gln Ile Ser His Ile Ala Gln Gln Leu Gly Leu Glu Lys Asp
               260                 265                 270
   Val Val Arg Val Trp Phe Cys Asn Arg Arg Gln Lys Gly Lys Arg Ser
           275                 280                 285
   Ser Ser Asp Tyr Ala Gln Arg Glu Asp Phe Glu Ala Ala Gly Ser Pro
       290                 295                 300
   Phe Ser Gly Gly Pro Val Ser Phe Pro Leu Ala Pro Gly Pro His Phe
   305                 310                 315                 320
   Gly Thr Pro Gly Tyr Gly Ser Pro His Phe Thr Ala Leu Tyr Ser Ser
                   325                 330                 335
   Val Pro Phe Pro Glu Gly Glu Ala Phe Pro Pro Val Ser Val Thr Thr
               340                 345                 350
   Leu Gly Ser Pro Met His Ser Asn
           355                 360
<210> 3
   <211> 1085
   <212> DNA
   <213> Homo sapiens
<400> 3
   cacagcgccc gcatgtacaa catgatggag acggagctga agccgccggg cccgcagcaa      60
   acttcggggg gcggcggcgg caactccacc gcggcggcgg ccggcggcaa ccagaaaaac     120
   agcccggacc gcgtcaagcg gcccatgaat gccttcatgg tgtggtcccg cgggcagcgg     180
   cgcaagatgg cccaggagaa ccccaagatg cacaactcgg agatcagcaa gcgcctgggc     240
   gccgagtgga aacttttgtc ggagacggag aagcggccgt tcatcgacga ggctaagcgg     300
   ctgcgagcgc tgcacatgaa ggagcacccg gattataaat accggccccg gcggaaaacc     360
   aagacgctca tgaagaagga taagtacacg ctgcccggcg ggctgctggc ccccggcggc     420
   aatagcatgg cgagcggggt cggggtgggc gccggcctgg gcgcgggcgt gaaccagcgc     480
   atggacagtt acgcgcacat gaacggctgg agcaacggca gctacagcat gatgcaggac     540
   cagctgggct acccgcagca cccgggcctc aatgcgcacg gcgcagcgca gatgcagccc     600
   atgcaccgct acgacgtgag cgccctgcag tacaactcca tgaccagctc gcagacctac     660
   atgaacggct cgcccaccta cagcatgtcc tactcgcagc agggcacccc tggcatggct     720
   cttggctcca tgggttcggt ggtcaagtcc gaggccagct ccagcccccc tgtggttacc     780
   tcttcctccc actccagggc gccctgccag gccggggacc tccgggacat gatcagcatg     840
   tatctccccg gcgccgaggt gccggaaccc gccgccccca gcagacttca catgtcccag     900
   cactaccaga gcggcccggt gcccggcacg gccattaacg gcacactgcc cctctcacac     960
   atgtgagggc cggacagcga actggagggg ggagaaattt tcaaagaaaa acgagggaaa    1020
   tgggaggggt gcaaaagagg agagtaagaa acagcatgga gaaaacccgg tacgctcaaa    1080
   aaaaa                                                                1085
<210> 4
   <211> 317
   <212> PRT
   <213> Homo sapiens
<400> 4
<210> 5
   <211> 2098
   <212> DNA
   <213> Homo sapiens
<400> 5
   attataaatc tagagactcc aggattttaa cgttctgctg gactgagctg gttgcctcat 60
   gttattatgc aggcaactca ctttatccca atttcttgat acttttcctt ctggaggtcc 120
   tatttctcta acatcttcca gaaaagtctt aaagctgcct taaccttttt tccagtccac 180
   ctcttaaatt ttttcctcct cttcctctat actaacatga gtgtggatcc agcttgtccc 240
   caaagcttgc cttgctttga agcatccgac tgtaaagaat cttcacctat gcctgtgatt 300
   tgtgggcctg aagaaaacta tccatccttg caaatgtctt ctgctgagat gcctcacacg 360
   gagactgtct ctcctcttcc ttcctccatg gatctgctta ttcaggacag ccctgattct 420
   tccaccagtc ccaaaggcaa acaacccact tctgcagaga agagtgtcgc aaaaaaggaa 480
   gacaaggtcc cggtcaagaa acagaagacc agaactgtgt tctcttccac ccagctgtgt 540
   gtactcaatg atagatttca gagacagaaa tacctcagcc tccagcagat gcaagaactc 600
   tccaacatcc tgaacctcag ctacaaacag gtgaagacct ggttccagaa ccagagaatg 660
   aaatctaaga ggtggcagaa aaacaactgg ccgaagaata gcaatggtgt gacgcagaag 720
   gcctcagcac ctacctaccc cagcctttac tcttcctacc accagggatg cctggtgaac 780
   ccgactggga accttccaat gtggagcaac cagacctgga acaattcaac ctggagcaac 840
   cagacccaga acatccagtc ctggagcaac cactcctgga acactcagac ctggtgcacc 900
   caatcctgga acaatcaggc ctggaacagt cccttctata actgtggaga ggaatctctg 960
   cagtcctgca tgcagttcca gccaaattct cctgccagtg acttggaggc tgccttggaa 1020
   gctgctgggg aaggccttaa tgtaatacag cagaccacta ggtattttag tactccacaa 1080
   accatggatt tattcctaaa ctactccatg aacatgcaac ctgaagacgt gtgaagatga 1140
   gtgaaactga tattactcaa tttcagtctg gacactggct gaatccttcc tctcccctcc 1200
   tcccatccct cataggattt ttcttgtttg gaaaccacgt gttctggttt ccatgatgcc 1260
   catccagtca atctcatgga gggtggagta tggttggagc ctaatcagcg aggtttcttt 1320
   tttttttttt ttcctattgg atcttcctgg agaaaatact tttttttttt ttttttttga 1380
   aacggagtct tgctctgtcg cccaggctgg agtgcagtgg cgcggtcttg gctcactgca 1440
   agctccgtct cccgggttca cgccattctc ctgcctcagc ctcccgagca gctgggacta 1500
   caggcgcccg ccacctcgcc cggctaatat tttgtatttt tagtagagac ggggtttcac 1560
   tgtgttagcc aggatggtct cgatctcctg accttgtgat ccacccgcct cggcctccct 1620
   aacagctggg atttacaggc gtgagccacc gcgccctgcc tagaaaagac attttaataa 1680
   ccttggctgc cgtctctggc tatagataag tagatctaat actagtttgg atatctttag 1740
   ggtttagaat ctaacctcaa gaataagaaa tacaagtaca aattggtgat gaagatgtat 1800
   tcgtattgtt tgggattggg aggctttgct tattttttaa aaactattga ggtaaagggt 1860
   taagctgtaa catacttaat tgatttctta ccgtttttgg ctctgttttg ctatatcccc 1920
   taatttgttg gttgtgctaa tctttgtaga aagaggtctc gtatttgctg catcgtaatg 1980
   acatgagtac tgctttagtt ggtttaagtt caaatgaatg aaacaactat ttttccttta 2040
   gttgatttta ccctgatttc accgagtgtt tcaatgagta aatatacagc ttaaacat 2098
<210> 6
   <211> 305
   <212> PRT
   <213> Homo sapiens
<400> 6
   Met Ser Val Asp Pro Ala Cys Pro Gln Ser Leu Pro Cys Phe Glu Ala
   1               5                   10                  15
   Ser Asp Cys Lys Glu Ser Ser Pro Met Pro Val Ile Cys Gly Pro Glu
               20                  25                  30
   Glu Asn Tyr Pro Ser Leu Gln Met Ser Ser Ala Glu Met Pro His Thr
           35                  40                  45
   Glu Thr Val Ser Pro Leu Pro Ser Ser Met Asp Leu Leu Ile Gln Asp
       50                  55                  60
   Ser Pro Asp Ser Ser Thr Ser Pro Lys Gly Lys Gln Pro Thr Ser Ala
   65                  70                  75                  80
   Glu Lys Ser Val Ala Lys Lys Glu Asp Lys Val Pro Val Lys Lys Gln
                   85                  90                  95
   Lys Thr Arg Thr Val Phe Ser Ser Thr Gln Leu Cys Val Leu Asn Asp
               100                 105                 110
   Arg Phe Gln Arg Gln Lys Tyr Leu Ser Leu Gln Gln Met Gln Glu Leu
           115                 120                 125
   Ser Asn Ile Leu Asn Leu Ser Tyr Lys Gln Val Lys Thr Trp Phe Gln
       130                 135                 140
   Asn Gln Arg Met Lys Ser Lys Arg Trp Gln Lys Asn Asn Trp Pro Lys
   145                 150                 155                 160
   Asn Ser Asn Gly Val Thr Gln Lys Ala Ser Ala Pro Thr Tyr Pro Ser
                   165                 170                 175
   Leu Tyr Ser Ser Tyr His Gln Gly Cys Leu Val Asn Pro Thr Gly Asn
               180                 185                 190
   Leu Pro Met Trp Ser Asn Gln Thr Trp Asn Asn Ser Thr Trp Ser Asn
           195                 200                 205
   Gln Thr Gln Asn Ile Gln Ser Trp Ser Asn His Ser Trp Asn Thr Gln
       210                 215                 220
   Thr Trp Cys Thr Gln Ser Trp Asn Asn Gln Ala Trp Asn Ser Pro Phe
   225 230 235 240
   Tyr Asn Cys Gly Glu Glu Ser Leu Gln Ser Cys Met Gln Phe Gln Pro
   245 250 255
   Asn Ser Pro Ala Ser Asp Leu Glu Ala Ala Leu Glu Ala Ala Gly Glu
   260 265 270
   Gly Leu Asn Val Ile Gln Gln Thr Thr Arg Tyr Phe Ser Thr Pro Gln
   275 280 285
   Thr Met Asp Leu Phe Leu Asn Tyr Ser Met Asn Met Gln Pro Glu Asp
   290 295 300
   Val
   305
<210> 7
   <211> 780
   <212> DNA
   <213> Homo sapiens
<400> 7
   gtgcggggga agatgtagca gcttcttctc cgaaccaacc ctttgccttc ggacttctcc 60
   ggggccagca gccgcccgac caggggcccg gggccacggg ctcagccgac gaccatgggc 120
   tccgtgtcca accagcagtt tgcaggtggc tgcgccaagg cggcagaaga ggcgcccgag 180
   gaggcgccgg aggacgcggc ccgggcggcg gacgagcctc agctgctgca cggtgcgggc 240
   atctgtaagt ggttcaacgt gcgcatgggg ttcggcttcc tgtccatgac cgcccgcgcc 300
   ggggtcgcgc tcgacccccc agtggatgtc tttgtgcacc agagtaagct gcacatggaa 360
   gggttccgga gcttgaagga gggtgaggca gtggagttca cctttaagaa gtcagccaag 420
   ggtctggaat ccatccgtgt caccggacct ggtggagtat tctgtattgg gagtgagagg 480
   cggccaaaag gaaagagcat gcagaagcgc agatcaaaag gagacaggtg ctacaactgt 540
   ggaggtctag atcatcatgc caaggaatgc aagctgccac cccagcccaa gaagtgccac 600
   ttctgccaga gcatcagcca tatggtagcc tcatgtccgc tgaaggccca gcagggccct 660
   agtgcacagg gaaagccaac ctactttcga gaggaagaag aagaaatcca cagccctacc 720
   ctgctcccgg aggcacagaa ttgagccaca atgggtgggg gctattcttt tgctatcagg 780
<210> 8
   <211> 209
   <212> PRT
   <213> Homo sapiens
<400> 8
   Met Gly Ser Val Ser Asn Gln Gln Phe Ala Gly Gly Cys Ala Lys Ala
   1               5                   10                  15
   Ala Glu Glu Ala Pro Glu Glu Ala Pro Glu Asp Ala Ala Arg Ala Ala
               20                  25                  30
   Asp Glu Pro Gln Leu Leu His Gly Ala Gly Ile Cys Lys Trp Phe Asn
           35                  40                  45
   Val Arg Met Gly Phe Gly Phe Leu Ser Met Thr Ala Arg Ala Gly Val
       50                  55                  60
   Ala Leu Asp Pro Pro Val Asp Val Phe Val His Gln Ser Lys Leu His
   65                  70                  75                  80
   Met Glu Gly Phe Arg Ser Leu Lys Glu Gly Glu Ala Val Glu Phe Thr
                   85                  90                  95
   Phe Lys Lys Ser Ala Lys Gly Leu Glu Ser Ile Arg Val Thr Gly Pro
               100                 105                 110
   Gly Gly Val Phe Cys Ile Gly Ser Glu Arg Arg Pro Lys Gly Lys Ser
           115                 120                 125
   Met Gln Lys Arg Arg Ser Lys Gly Asp Arg Cys Tyr Asn Cys Gly Gly
       130                 135                 140
   Leu Asp His His Ala Lys Glu Cys Lys Leu Pro Pro Gln Pro Lys Lys
   145                 150                 155                 160
   Cys His Phe Cys Gln Ser Ile Ser His Met Val Ala Ser Cys Pro Leu
                   165                 170                 175
   Lys Ala Gln Gln Gly Pro Ser Ala Gln Gly Lys Pro Thr Tyr Phe Arg
               180                 185                 190
   Glu Glu Glu Glu Glu Ile His Ser Pro Thr Leu Leu Pro Glu Ala Gln
           195                 200                 205
   Asn
<210> 9
   <211> 2639
   <212> DNA
   <213> Homo sapiens
<400> 9
   tcgaggcgac cgcgacagtg gtgggggacg ctgctgagtg gaagagagcg cagcccggcc 60
   accggaccta cttactcgcc ttgctgattg tctatttttg cgtttacaac ttttctaaga 120
   acttttgtat acaaaggaac tttttaaaaa agacgcttcc aagttatatt taatccaaag 180
   aagaaggatc tcggccaatt tggggttttg ggttttggct tcgtttcttc tcttcgttga 240
   ctttggggtt caggtgcccc agctgcttcg ggctgccgag gaccttctgg gcccccacat 300
   taatgaggca gccacctggc gagtctgaca tggctgtcag cgacgcgctg ctcccatctt 360
   tctccacgtt cgcgtctggc ccggcgggaa gggagaagac actgcgtcaa gcaggtgccc 420
   cgaataaccg ctggcgggag gagctctccc acatgaagcg acttccccca gtgcttcccg 480
   gccgccccta tgacctggcg gcggcgaccg tggccacaga cctggagagc ggcggagccg 540
   gtgcggcttg cggcggtagc aacctggcgc ccctacctcg gagagagacc gaggagttca 600
   acgatctcct ggacctggac tttattctct ccaattcgct gacccatcct ccggagtcag 660
   tggccgccac cgtgtcctcg tcagcgtcag cctcctcttc gtcgtcgccg tcgagcagcg 720
   gccctgccag cgcgccctcc acctgcagct tcacctatcc gatccgggcc gggaacgacc 780
   cgggcgtggc gccgggcggc acgggcggag gcctcctcta tggcagggag tccgctcccc 840
   ctccgacggc tcccttcaac ctggcggaca tcaacgacgt gagcccctcg ggcggcttcg 900
   tggccgagct cctgcggcca gaattggacc cggtgtacat tccgccgcag cagccgcagc 960
   cgccaggtgg cgggctgatg ggcaagttcg tgctgaaggc gtcgctgagc gcccctggca 1020
   gcgagtacgg cagcccgtcg gtcatcagcg tcagcaaagg cagccctgac ggcagccacc 1080
   cggtggtggt ggcgccctac aacggcgggc cgccgcgcac gtgccccaag atcaagcagg 1140
   aggcggtctc ttcgtgcacc cacttgggcg ctggaccccc tctcagcaat ggccaccggc 1200
   cggctgcaca cgacttcccc ctggggcggc agctccccag caggactacc ccgaccctgg 1260
   gtcttgagga agtgctgagc agcagggact gtcaccctgc cctgccgctt cctcccggct 1320
   tccatcccca cccggggccc aattacccat ccttcctgcc cgatcagatg cagccgcaag 1380
   tcccgccgct ccattaccaa gagctcatgc cacccggttc ctgcatgcca gaggagccca 1440
   agccaaagag gggaagacga tcgtggcccc ggaaaaggac cgccacccac acttgtgatt 1500
   acgcgggctg cggcaaaacc tacacaaaga gttcccatct caaggcacac ctgcgaaccc 1560
   acacaggtga gaaaccttac cactgtgact gggacggctg tggatggaaa ttcgcccgct 1620
   cagatgaact gaccaggcac taccgtaaac acacggggca ccgcccgttc cagtgccaaa 1680
   aatgcgaccg agcattttcc aggtcggacc acctcgcctt acacatgaag aggcattttt 1740
   aaatcccaga cagtggatat gacccacact gccagaagag aattcagtat tttttacttt 1800
   tcacactgtc ttcccgatga gggaaggagc ccagccagaa agcactacaa tcatggtcaa 1860
   gttcccaact gagtcatctt gtgagtggat aatcaggaaa aatgaggaat ccaaaagaca 1920
   aaaatcaaag aacagatggg gtctgtgact ggatcttcta tcattccaat tctaaatccg 1980
   acttgaatat tcctggactt acaaaatgcc aagggggtga ctggaagttg tggatatcag 2040
   ggtataaatt atatccgtga gttgggggag ggaagaccag aattcccttg aattgtgtat 2100
   tgatgcaata taagcataaa agatcacctt gtattctctt taccttctaa aagccattat 2160
   tatgatgtta gaagaagagg aagaaattca ggtacagaaa acatgtttaa atagcctaaa 2220
   tgatggtgct tggtgagtct tggttctaaa ggtaccaaac aaggaagcca aagttttcaa 2280
   actgctgcat actttgacaa ggaaaatcta tatttgtctt ccgatcaaca tttatgacct 2340
   aagtcaggta atatacctgg tttacttctt tagcattttt atgcagacag tctgttatgc 2400
   actgtggttt cagatgtgca ataatttgta caatggttta ttcccaagta tgccttaagc 2460
   agaacaaatg tgtttttcta tatagttcct tgccttaata aatatgtaat ataaatttaa 2520
   gcaaacgtct attttgtata tttgtaaact acaaagtaaa atgaacattt tgtggagttt 2580
   gtattttgca tactcaaggt gagaattaag ttttaaataa acctataata ttttatctg 2639
<210> 10
   <211> 470
   <212> PRT
   <213> Homo sapiens
<400> 10
   Met Ala Val Ser Asp Ala Leu Leu Pro Ser Phe Ser Thr Phe Ala Ser
   1               5                   10                  15
   Gly Pro Ala Gly Arg Glu Lys Thr Leu Arg Gln Ala Gly Ala Pro Asn
               20                  25                  30
   Asn Arg Trp Arg Glu Glu Leu Ser His Met Lys Arg Leu Pro Pro Val
           35                  40                  45
   Leu Pro Gly Arg Pro Tyr Asp Leu Ala Ala Ala Thr Val Ala Thr Asp
       50                  55                  60
   Leu Glu Ser Gly Gly Ala Gly Ala Ala Cys Gly Gly Ser Asn Leu Ala
   65                  70                  75                  80
   Pro Leu Pro Arg Arg Glu Thr Glu Glu Phe Asn Asp Leu Leu Asp Leu
                   85                  90                  95
   Asp Phe Ile Leu Ser Asn Ser Leu Thr His Pro Pro Glu Ser Val Ala
               100                 105                 110
   Ala Thr Val Ser Ser Ser Ala Ser Ala Ser Ser Ser Ser Ser Pro Ser
           115                 120                 125
   Ser Ser Gly Pro Ala Ser Ala Pro Ser Thr Cys Ser Phe Thr Tyr Pro
       130                 135                 140
   Ile Arg Ala Gly Asn Asp Pro Gly Val Ala Pro Gly Gly Thr Gly Gly
   145                 150                 155                 160
   Gly Leu Leu Tyr Gly Arg Glu Ser Ala Pro Pro Pro Thr Ala Pro Phe
                   165                 170                 175
   Asn Leu Ala Asp Ile Asn Asp Val Ser Pro Ser Gly Gly Phe Val Ala
               180                 185                 190
   Glu Leu Leu Arg Pro Glu Leu Asp Pro Val Tyr Ile Pro Pro Gln Gln
           195                 200                 205
   Pro Gln Pro Pro Gly Gly Gly Leu Met Gly Lys Phe Val Leu Lys Ala
       210                 215                 220
   Ser Leu Ser Ala Pro Gly Ser Glu Tyr Gly Ser Pro Ser Val Ile Ser
   225                 230                 235                 240
   Val Ser Lys Gly Ser Pro Asp Gly Ser His Pro Val Val Val Ala Pro
                   245                 250                 255
   Tyr Asn Gly Gly Pro Pro Arg Thr Cys Pro Lys Ile Lys Gln Glu Ala
               260                 265                 270
   Val Ser Ser Cys Thr His Leu Gly Ala Gly Pro Pro Leu Ser Asn Gly
           275                 280                 285
   His Arg Pro Ala Ala His Asp Phe Pro Leu Gly Arg Gln Leu Pro Ser
       290                 295                 300
   Arg Thr Thr Pro Thr Leu Gly Leu Glu Glu Val Leu Ser Ser Arg Asp
   305                 310                 315                 320
   Cys His Pro Ala Leu Pro Leu Pro Pro Gly Phe His Pro His Pro Gly
                   325                 330                 335
   Pro Asn Tyr Pro Ser Phe Leu Pro Asp Gln Met Gln Pro Gln Val Pro
               340                 345                 350
   Pro Leu His Tyr Gln Glu Leu Met Pro Pro Gly Ser Cys Met Pro Glu
           355                 360                 365
   Glu Pro Lys Pro Lys Arg Gly Arg Arg Ser Trp Pro Arg Lys Arg Thr
       370                 375                 380
   Ala Thr His Thr Cys Asp Tyr Ala Gly Cys Gly Lys Thr Tyr Thr Lys
   385                 390                 395                 400
   Ser Ser His Leu Lys Ala His Leu Arg Thr His Thr Gly Glu Lys Pro
                   405                 410                 415
   Tyr His Cys Asp Trp Asp Gly Cys Gly Trp Lys Phe Ala Arg Ser Asp
               420                 425                 430
   Glu Leu Thr Arg His Tyr Arg Lys His Thr Gly His Arg Pro Phe Gln
           435                 440                 445
   Cys Gln Lys Cys Asp Arg Ala Phe Ser Arg Ser Asp His Leu Ala Leu
       450                 455                 460
   His Met Lys Arg His Phe
   465                 470
<210> 11
   <211> 2377
   <212> DNA
   <213> Homo sapiens
<400> 11
   acccccgagc tgtgctgctc gcggccgcca ccgccgggcc ccggccgtcc ctggctcccc      60
   tcctgcctcg agaagggcag ggcttctcag aggcttggcg ggaaaaagaa cggagggagg     120
   gatcgcgctg agtataaaag ccggttttcg gggctttatc taactcgctg tagtaattcc     180
   agcgagaggc agagggagcg agcgggcggc cggctagggt ggaagagccg ggcgagcaga     240
   gctgcgctgc gggcgtcctg ggaagggaga tccggagcga atagggggct tcgcctctgg     300
   cccagccctc ccgctgatcc cccagccagc ggtccgcaac ccttgccgca tccacgaaac     360
   tttgcccata gcagcgggcg ggcactttgc actggaactt acaacacccg agcaaggacg     420
   cgactctccc gacgcgggga ggctattctg cccatttggg gacacttccc cgccgctgcc     480
   aggacccgct tctctgaaag gctctccttg cagctgctta gacgctggat ttttttcggg     540
   tagtggaaaa ccagcagcct cccgcgacga tgcccctcaa cgttagcttc accaacagga     600
   actatgacct cgactacgac tcggtgcagc cgtatttcta ctgcgacgag gaggagaact     660
   tctaccagca gcagcagcag agcgagctgc agcccccggc gcccagcgag gatatctgga     720
   agaaattcga gctgctgccc accccgcccc tgtcccctag ccgccgctcc gggctctgct     780
   cgccctccta cgttgcggtc acacccttct cccttcgggg agacaacgac ggcggtggcg     840
   ggagcttctc cacggccgac cagctggaga tggtgaccga gctgctggga ggagacatgg     900
   tgaaccagag tttcatctgc gacccggacg acgagacctt catcaaaaac atcatcatcc      960
   aggactgtat gtggagcggc ttctcggccg ccgccaagct cgtctcagag aagctggcct     1020
   cctaccaggc tgcgcgcaaa gacagcggca gcccgaaccc cgcccgcggc cacagcgtct     1080
   gctccacctc cagcttgtac ctgcaggatc tgagcgccgc cgcctcagag tgcatcgacc     1140
   cctcggtggt cttcccctac cctctcaacg acagcagctc gcccaagtcc tgcgcctcgc     1200
   aagactccag cgccttctct ccgtcctcgg attctctgct ctcctcgacg gagtcctccc     1260
   cgcagggcag ccccgagccc ctggtgctcc atgaggagac accgcccacc accagcagcg     1320
   actctgagga ggaacaagaa gatgaggaag aaatcgatgt tgtttctgtg gaaaagaggc     1380
   aggctcctgg caaaaggtca gagtctggat caccttctgc tggaggccac agcaaacctc     1440
   ctcacagccc actggtcctc aagaggtgcc acgtctccac acatcagcac aactacgcag     1500
   cgcctccctc cactcggaag gactatcctg ctgccaagag ggtcaagttg gacagtgtca     1560
   gagtcctgag acagatcagc aacaaccgaa aatgcaccag ccccaggtcc tcggacaccg     1620
   aggagaatgt caagaggcga acacacaacg tcttggagcg ccagaggagg aacgagctaa     1680
   aacggagctt ttttgccctg cgtgaccaga tcccggagtt ggaaaacaat gaaaaggccc     1740
   ccaaggtagt tatccttaaa aaagccacag catacatcct gtccgtccaa gcagaggagc     1800
   aaaagctcat ttctgaagag gacttgttgc ggaaacgacg agaacagttg aaacacaaac     1860
   ttgaacagct acggaactct tgtgcgtaag gaaaagtaag gaaaacgatt ccttctaaca     1920
   gaaatgtcct gagcaatcac ctatgaactt gtttcaaatg catgatcaaa tgcaacctca     1980
   caaccttggc tgagtcttga gactgaaaga tttagccata atgtaaactg cctcaaattg     2040
   gactttgggc ataaaagaac ttttttatgc ttaccatctt ttttttttct ttaacagatt      2100
   tgtatttaag aattgttttt aaaaaatttt aagatttaca caatgtttct ctgtaaatat     2160
   tgccattaaa tgtaaataac tttaataaaa cgtttatagc agttacacag aatttcaatc     2220
   ctagtatata gtacctagta ttataggtac tataaaccct aatttttttt atttaagtac      2280
   attttgcttt ttaaagttga tttttttcta ttgtttttag aaaaaataaa ataactggca     2340
   aatatatcat tgagccaaaa aaaaaaaaaa aaaaaaa                              2377
<210> 12
   <211> 454
   <212> PRT
   <213> Homo sapiens
<400> 12
   Met Asp Phe Phe Arg Val Val Glu Asn Gln Gln Pro Pro Ala Thr Met
   1               5                   10                  15
   Pro Leu Asn Val Ser Phe Thr Asn Arg Asn Tyr Asp Leu Asp Tyr Asp
   20 25 30
   Ser Val Gln Pro Tyr Phe Tyr Cys Asp Glu Glu Glu Asn Phe Tyr Gln
   35 40 45
   Gln Gln Gln Gln Ser Glu Leu Gln Pro Pro Ala Pro Ser Glu Asp Ile
   50 55 60
   Trp Lys Lys Phe Glu Leu Leu Pro Thr Pro Pro Leu Ser Pro Ser Arg
   65 70 75 80
   Arg Ser Gly Leu Cys Ser Pro Ser Tyr Val Ala Val Thr Pro Phe Ser
   85 90 95
   Leu Arg Gly Asp Asn Asp Gly Gly Gly Gly Ser Phe Ser Thr Ala Asp
   100 105 110
   Gln Leu Glu Met Val Thr Glu Leu Leu Gly Gly Asp Met Val Asn Gln
   115 120 125
   Ser Phe Ile Cys Asp Pro Asp Asp Glu Thr Phe Ile Lys Asn Ile Ile
   130 135 140
   Ile Gln Asp Cys Met Trp Ser Gly Phe Ser Ala Ala Ala Lys Leu Val
   145 150 155 160
   Ser Glu Lys Leu Ala Ser Tyr Gln Ala Ala Arg Lys Asp Ser Gly Ser
   165 170 175
   Pro Asn Pro Ala Arg Gly His Ser Val Cys Ser Thr Ser Ser Leu Tyr
   180 185 190
   Leu Gln Asp Leu Ser Ala Ala Ala Ser Glu Cys Ile Asp Pro Ser Val
   195 200 205
   Val Phe Pro Tyr Pro Leu Asn Asp Ser Ser Ser Pro Lys Ser Cys Ala
   210 215 220
   Ser Gln Asp Ser Ser Ala Phe Ser Pro Ser Ser Asp Ser Leu Leu Ser
   225 230 235 240
   Ser Thr Glu Ser Ser Pro Gln Gly Ser Pro Glu Pro Leu Val Leu His
   245 250 255
   Glu Glu Thr Pro Pro Thr Thr Ser Ser Asp Ser Glu Glu Glu Gln Glu
   260 265 270
   Asp Glu Glu Glu Ile Asp Val Val Ser Val Glu Lys Arg Gln Ala Pro
   275 280 285
   Gly Lys Arg Ser Glu Ser Gly Ser Pro Ser Ala Gly Gly His Ser Lys
   290 295 300
   Pro Pro His Ser Pro Leu Val Leu Lys Arg Cys His Val Ser Thr His
   305 310 315 320
   Gln His Asn Tyr Ala Ala Pro Pro Ser Thr Arg Lys Asp Tyr Pro Ala
   325 330 335
   Ala Lys Arg Val Lys Leu Asp Ser Val Arg Val Leu Arg Gln Ile Ser
   340 345 350
   Asn Asn Arg Lys Cys Thr Ser Pro Arg Ser Ser Asp Thr Glu Glu Asn
   355 360 365
   Val Lys Arg Arg Thr His Asn Val Leu Glu Arg Gln Arg Arg Asn Glu
   370 375 380
   Leu Lys Arg Ser Phe Phe Ala Leu Arg Asp Gln Ile Pro Glu Leu Glu
   385 390 395 400
   Asn Asn Glu Lys Ala Pro Lys Val Val Ile Leu Lys Lys Ala Thr Ala
   405 410 415
   Tyr Ile Leu Ser Val Gln Ala Glu Glu Gln Lys Leu Ile Ser Glu Glu
   420 425 430
   Asp Leu Leu Arg Lys Arg Arg Glu Gln Leu Lys His Lys Leu Glu Gln
   435 440 445
   Leu Arg Asn Ser Cys Ala
   450
<210> 13
   <211> 551
   <212> PRT
   <213> Homo sapiens
<400> 13
<210> 14
   <211> 21
   <212> DNA
   <213> Artificial Sequence
<220>
   <223> Oligonucleotide
<400> 14
   acctggaaaa cctgttcctg c 21
<210> 15
   <211> 21
   <212> DNA
   <213> Artificial Sequence
<220>
   <223> Oligonucleotide
<400> 15
   agctcggatc ctcatcagtt g 21
<210> 16
   <211> 21
   <212> DNA
   <213> Artificial Sequence
<220>
   <223> Oligonucleotide
<400> 16
   aaccagcgga tggacagcta c 21
<210> 17
   <211> 21
   <212> DNA
   <213> Artificial Sequence
<220>
   <223> Oligonucleotide
<400> 17
   gcttttcacc acgctgccca t 21
<210> 18
   <211> 21
   <212> DNA
   <213> Artificial Sequence
<220>
   <223> Oligonucleotide
<400> 18
   aagacctaca ccaagagcag c 21
<210> 19
   <211> 21
   <212> DNA
   <213> Artificial Sequence
<220>
   <223> Oligonucleotide
<400> 19
   aggtggtcag atctgctgaa g 21
<210> 20
   <211> 21
   <212> DNA
   <213> Artificial Sequence
<220>
   <223> Oligonucleotide
<400> 20
   cccctgaacg acagctctag c 21
<210> 21
   <211> 21
   <212> DNA
   <213> Artificial Sequence
<220>
   <223> Oligonucleotide
<400> 21
   ttctccacgg acaccacgtc g 21
<210> 22
   <211> 21
   <212> DNA
   <213> Artificial Sequence
<220>
   <223> Oligonucleotide
<400> 22
   aaatacagcc cttgtgcctg g 21
<210> 23
   <211> 21
   <212> DNA
   <213> Artificial Sequence
<220>
   <223> Oligonucleotide
<400> 23
   ggtgagctgg catacgaatc a 21
<210> 24
   <211> 19
   <212> DNA
   <213> Artificial Sequence
<220>
   <223> Oligonucleotide
<400> 24
   aaggccaagg agtacagtc 19
<210> 25
   <211> 20
   <212> DNA
   <213> Artificial Sequence
<220>
   <223> Oligonucleotide
<400> 25
   atcttcagtt tcggaggtaa 20
<210> 26
   <211> 21
   <212> DNA
   <213> Artificial Sequence
<220>
   <223> Oligonucleotide
<400> 26
   tcccagacca aggtttcttt c 21
<210> 27
   <211> 21
   <212> DNA
   <213> Artificial Sequence
<220>
   <223> Oligonucleotide
<400> 27
   ttacctggct taggggtggt c 21
<210> 28
   <211> 26
   <212> DNA
   <213> Artificial Sequence
<220>
   <223> Oligonucleotide
<400> 28
   cctgctcaag ctgactcgac accgtg 26
<210> 29
   <211> 25
   <212> DNA
   <213> Artificial Sequence
<220>
   <223> Oligonucleotide
<400> 29
   ggaaaagctg gccctggggt ggagc 25
<210> 30
   <211> 21
   <212> DNA
   <213> Artificial Sequence
<220>
   <223> Oligonucleotide
<400> 30
   tgacactggc aaaacaatgc a 21
<210> 31
   <211> 21
   <212> DNA
   <213> Artificial Sequence
<220>
   <223> Oligonucleotide
<400> 31
   ggtccttttc accagcaagc t 21
<210> 32
   <211> 2930
   <212> DNA
   <213> Homo sapiens
<400> 32
   agcagacgag ggcttgtgcg agagggggcc gggcggctgc agggaaggcg gagtccaagg     60
   ggaaaacgaa actgagaacc agctctcccg aagccgcggg tctccggccg gcggcggcgg    120
   cggcggcggc ggcggcgcag tttgctcata ctttgtgact tgcggtcaca gtggcattca    180
   gctccacact tggtagaacc acaggcacga caagcataga aacatcctaa acaatcttca    240
   tcgaggcatc gaggtccatc ccaataaaaa tcaggagacc ctggctatca tagaccttag    300
   tcttcgctgg tatcactcgt ctgtctgaac cagcggttgc atttttttaa gccttctttt    360
   ttctctttta ccagtttctg gagcaaattc agtttgcctt cctggatttg taaattgtaa    420
   tgacctcaaa actttagcag ttcttccatc tgactcaggt ttgcttctct ggcggtcttc    480
   agaatcaaca tccacacttc cgtgattatc tgcgtgcatt ttggacaaag cttccaacca    540
   ggatacggga agaagaaatg gctggtgatc tttcagcagg tttcttcatg gaggaactta    600
   atacataccg tcagaagcag ggagtagtac ttaaatatca agaactgcct aattcaggac    660
   ctccacatga taggaggttt acatttcaag ttataataga tggaagagaa tttccagaag    720
   gtgaaggtag atcaaagaag gaagcaaaaa atgccgcagc caaattagct gttgagatac 780
   ttaataagga aaagaaggca gttagtcctt tattattgac aacaacgaat tcttcagaag 840
   gattatccat ggggaattac ataggcctta tcaatagaat tgcccagaag aaaagactaa 900
   ctgtaaatta tgaacagtgt gcatcggggg tgcatgggcc agaaggattt cattataaat 960
   gcaaaatggg acagaaagaa tatagtattg gtacaggttc tactaaacag gaagcaaaac 1020
   aattggccgc taaacttgca tatcttcaga tattatcaga agaaacctca gtgaaatctg 1080
   actacctgtc ctctggttct tttgctacta cgtgtgagtc ccaaagcaac tctttagtga 1140
   ccagcacact cgcttctgaa tcatcatctg aaggtgactt ctcagcagat acatcagaga 1200
   taaattctaa cagtgacagt ttaaacagtt cttcgttgct tatgaatggt ctcagaaata 1260
   atcaaaggaa ggcaaaaaga tctttggcac ccagatttga ccttcctgac atgaaagaaa 1320
   caaagtatac tgtggacaag aggtttggca tggattttaa agaaatagaa ttaattggct 1380
   caggtggatt tggccaagtt ttcaaagcaa aacacagaat tgacggaaag acttacgtta 1440
   ttaaacgtgt taaatataat aacgagaagg cggagcgtga agtaaaagca ttggcaaaac 1500
   ttgatcatgt aaatattgtt cactacaatg gctgttggga tggatttgat tatgatcctg 1560
   agaccagtga tgattctctt gagagcagtg attatgatcc tgagaacagc aaaaatagtt 1620
   caaggtcaaa gactaagtgc cttttcatcc aaatggaatt ctgtgataaa gggaccttgg 1680
   aacaatggat tgaaaaaaga agaggcgaga aactagacaa agttttggct ttggaactct 1740
   ttgaacaaat aacaaaaggg gtggattata tacattcaaa aaaattaatt catagagatc 1800
   ttaagccaag taatatattc ttagtagata caaaacaagt aaagattgga gactttggac 1860
   ttgtaacatc tctgaaaaat gatggaaagc gaacaaggag taagggaact ttgcgataca 1920
   tgagcccaga acagatttct tcgcaagact atggaaagga agtggacctc tacgctttgg 1980
   ggctaattct tgctgaactt cttcatgtat gtgacactgc ttttgaaaca tcaaagtttt 2040
   tcacagacct acgggatggc atcatctcag atatatttga taaaaaagaa aaaactcttc 2100
   tacagaaatt actctcaaag aaacctgagg atcgacctaa cacatctgaa atactaagga 2160
   ccttgactgt gtggaagaaa agcccagaga aaaatgaacg acacacatgt tagagccctt 2220
   ctgaaaaagt atcctgcttc tgatatgcag ttttccttaa attatctaaa atctgctagg 2280
   gaatatcaat agatatttac cttttatttt aatgtttcct ttaatttttt actattttta 2340
   ctaatctttc tgcagaaaca gaaaggtttt cttctttttg cttcaaaaac attcttacat 2400
   tttacttttt cctggctcat ctctttattc tttttttttt tttaaagaca gagtctcgct 2460
   ctgttgccca ggctggagtg caatgacaca gtcttggctc actgcaactt ctgcctcttg 2520
   ggttcaagtg attctcctgc ctcagcctcc tgagtagctg gattacaggc atgtgccacc 2580
   cacccaacta atttttgtgt ttttaataaa gacagggttt caccatgttg gccaggctgg 2640
   tctcaaactc ctgacctcaa gtaatccacc tgcctcggcc tcccaaagtg ctgggattac     2700
   agggatgagc caccgcgccc agcctcatct ctttgttcta aagatggaaa aaccaccccc     2760
   aaattttctt tttatactat taatgaatca atcaattcat atctatttat taaatttcta     2820
   ccgcttttag gccaaaaaaa tgtaagatcg ttctctgcct cacatagctt acaagccagc     2880
   tggagaaata tggtactcat taaaaaaaaa aaaaaaagtg atgtacaacc                2930